# EUROPEAN PATENT SPECIFICATION

(11) **EP 3 101 087 B1**
(45) Date of publication and mention of the grant of the patent: **17.01.2018**
(21) Application number: 15170553.0
(22) Date of filing: 03.06.2015
(51) Int. Cl.: C09K 11/06, C07D 471/02, H01L 33/50, C07D 471/06

(54) **MULTIPLE CHROMOPHORES WITH A PERYLENEDIIMIDE SKELETON**
MEHRFACHCHROMOPHORE MIT EINEM PERYLENEDIIMIDGERÜST
CHROMOPHORES MULTIPLES AVEC UN SQUELETTE DE PÉRYLÈNE DIIMIDE

(43) Date of publication of application: 07.12.2016
(73) Proprietor: BASF SE, 67056 Ludwigshafen am Rhein (DE)
(72) Inventor: Koenemann, Martin, 68199 Mannheim (DE); Wagenblast, Gerhard, 67157 Wachenheim (DE); Ivanovici, Sorin, 69126 Heidelberg (DE); Carvalho, Mary-Ambre, 42110 Civens (FR)

(56) References cited:
- EP-A1- 0 033 079
- DE-A1-102005 037 115
- DE-B1- 1 569 841
- GUIJU QI ET AL: "Efficient collection of excitation energy from a linear-shaped weakly interacted perylenetetracarboxylic diimides array", PHYSICAL CHEMISTRY CHEMICAL PHYSICS, vol. 15, no. 40, 1 January 2013 (2013-01-01), page 17342, XP055210020, ISSN: 1463-9076, DOI: 10.1039/c3cp52941j
- MICHAEL J. AHRENS ET AL: "Self-Assembly of Supramolecular Light-Harvesting Arrays from Covalent Multi-Chromophore Perylene-3,4:9,10-bis(dicarboximide) Building Blocks", JOURNAL OF THE AMERICAN CHEMICAL SOCIETY, vol. 126, no. 26, 1 July 2004 (2004-07-01) , pages 8284-8294, XP055029040, ISSN: 0002-7863, DOI: 10.1021/ja039820c

## Description

The present invention relates to multiple chromophores with a perylenediimide skeleton and to their use. In particular, the present invention relates to the use of said compound(s) in color converters for improving the energy conversion efficacy of white-light emitting diodes. The present invention also relates to the use of these color converters and to lighting devices comprising at least one LED or OLED and at least one color converter.

LEDs (light-emitting diodes, LEDs) and OLEDs (organic light-emitting diodes, OLEDs) are an important class of devices that convert electric energy into light. LEDs are attractive candidates to replace conventional light sources such as incandescent lamps and fluorescent lamps. Because of their low energy consumption, LEDs are increasingly being used as a light source for general lighting, for example in offices and residences, or for architectural lighting, in information signs, small appliances, and in the automobile and aircraft industries. Light emission is based on the recombination of electron-hole pairs (excitons) in the junction region of a pn junction poled in forward direction in a semiconductor. The size of the band gap of this semiconductor determines the approximate wavelength of the light emitted. In order to generate a particular color, LEDs with different band gaps can be combined to form a multi-LED.

Alternatively, a radiation conversion luminophore (also referred to as phosphor, or fluorescent colorant or fluorescent dye; in the context of the present invention, these terms are used interchangeable to describe a material that converts light of a first wavelength into light of a second wavelength) can also be combined with a LED. In this context, the radiation emitted by the LED is partly absorbed by the radiation conversion luminophore, which is thus induced to photoluminesce. The resulting light color of the LED results from the proportion of non-absorbed and transmitted LED light and the emission spectrum and intensity of the radiation converting luminophore. According to one approach called "phosphor on a chip", a polymeric material comprising a radiation converting luminophore is directly applied onto the LED light source (LED chip). The polymeric material is applied onto the LED chip, for instance, in droplet form or in hemispherical form, as a result of which particular optical effects contribute to the emission of the light. In phosphor on a chip LEDs, the radiation converting luminophores used are generally inorganic materials. Organic luminophore materials are not suitable, because the polymeric material and the radiation conversion luminophore are subject to relatively high thermal stress and radiation stress.

In another approach called "remote phosphor", the color converter (also referred to as "converter" or "light converter"), which generally comprises a polymer layer and one or more radiation converting luminophore(s), is spatially separated from the LED source.

The spatial distance between the primary light source, the LED, and the color converter reduces the stress resulting from heat and radiation to such an extent that organic fluorescent dyes can also be used as radiation conversion luminophores. Furthermore, LEDs according to the "remote phosphor" concept are more energy-efficient than those according to the "phosphor on a chip" concept. The use of organic fluorescent dyes in these converters offers various advantages, e.g., the composition/hue of the light can be precisely adjusted by applying optimized amounts of fluorescent dyes. Therefore, the broad emission spectra of fluorescent dyes allow to get white light with a high color rendering index.

White light-emitting LEDs are used in a wide range of applications as a light source or as a backlight in full-color displays including in flat panel display applications due to their long lifetime, high reliability and low power consumption. Two methods are commonly used to create white light with LEDs. The basis for the emission of white light is always the superimposition (mixing) of various colors. The first approach is through the combination of so called multi-LEDs, usually red, green, and blue ones. Because of the different brightnesses and operating conditions for the various light-emitting diodes, the multi-LED is technically complex and therefore expensive Moreover, component miniaturization of the multi-LED is severely limited.

Among the blue, green and red LEDs, the blue ones typically demonstrate the best thermal stability and efficiency. Thus, the second approach is through the combination of a blue LED with a converting material (phosphor) through luminescence conversion. The light source appears white to an human observer, if a portion of the blue light generated from a blue LED is converted to yellow light. In recent years, several classes of luminescent materials such as inorganic colorants and organic dyes have been described for use in color converters wherein the color converters contain a colorant/dye that is dispersed/dissolved into a polymer layer and is able to absorb the primary radiation of the LED or OLED and to generate a longer-wave secondary radiation. A conventional converter material is an inorganic converter material such as cerium-doped yttrium aluminum garnet (also referred to as Ce:YAG). A standard Ce:YAG-based white LED emits cold-white light, where the red component in the spectrum is absent. Generation of warm-white light is not possible with that configuration (without adding a red light emitting phosphor). Therefore, the light of a standard Ce:YAG-based white LED is often seen as unattractive.

In place of conventional inorganic phosphors, organic phosphors, namely organic fluorescent dyes, can also be used. Organic fluorescent dyes are advantageous in that they allow to create a warm-white light LED light since the fluorescent dye allows to tailor the hue of the light. In addition, organic fluorescent dyes exhibit much higher absorptivity compared to inorganic phosphors and are often available at low cost. No expensive materials such as rare-earth metals are needed. Although a great variety of organic fluorescent dyes have been available, they often exhibit low fluorescence quantum yields in a polymer matrix, unsufficient photostabilities, unsufficient thermal stability and/or are sensitive to moisture and oxygen. Further disadvantages are that many dyes are obtainable only by complicated, multistage syntheses using protecting group chemistry. Therefore, there is an ongoing need for organic fluorescent dyes having high quantum yields in the polymer matrix and excellent stabilities under the practical application conditions. In addition, the synthesis of the dyes should only require some steps, and preferably, without introduction and removal of a protective group.

WO 2007/014902 describes a multiple chromophore of the formula (A) 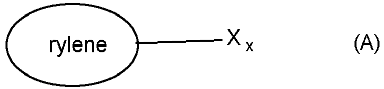 where rylene is perylene, terrylene or quaterrylene and where rylene is functionalized by at least one imide group, ester group or amide group and which may additionally be substituted by aryloxy, arylthio, hetaryloxy and/or hetarylthio,
X is a rylenedicarboximide radical which absorbs at a different wavelength from the rylene radical, and is joined to the rylene chromophore in the peri-position via the moiety -Y'-A-Y- which is bonded to X via Y and may likewise be substituted by aryloxy, arylthio, hetaryloxy and/or hetarylthio;
A is a bridging member having at least one aromatic or heteroaromatic radical, the Y or Y and Y' groups being bonded to the aromatic or heteroaromatic radical;
Y is -O- or -S-, and
Y' is 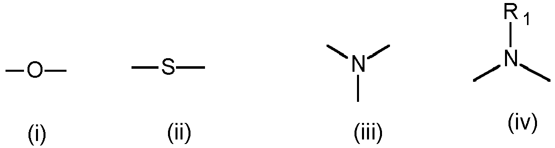 where the moieties (i) and (ii) may be part of the ester groups of the rylene radical and the moieties (iii) are part of the imide groups of the rylene radical and the moieties (iv) are part of the amide groups of the rylene radical; R₁ is hydrogen or C₁-C₁₈-alkyl, and x is from 1 to 7. The multiple chromophores are used for coloring organic and inorganic materials, for producing aqueous polymer dispersions which absorb and/or emit electromagnetic radiation, for obtaining markings and inscriptions invisible to the human eye, as filters or emitters in display applications, as emitters in chemiluminescence applications and as active components in photovoltaics. The use of the multiple chromophores as fluorescent dye in color converters comprising at least one polymer as a matrix material is not described.

In recent years, several classes of luminescent materials such as inorganic colorants, and organic dyes have been described for use in color converters wherein the color converter comprises a colorant/dye that is dispersed/dissolved into a polymer layer and is able to absorb the primary radiation of the LED or OLED and to generate a longer-wave secondary radiation.

Color converters can comprise a single organic fluorescent dye or a combination of several organic fluorescent dyes. For example, it has been known to use the light emitted by one fluorescent dye to stimulate another fluorescent dye or other material to emit light at a longer wavelength. For example, EP 1 868 419 describes a concept for such a color converter comprising a first perylene fluorescent dye, a second fluorescent dye and optionally, a third fluorescent dye. The first perylene fluorescent dye emits red fluorescence (wavengths of 580 to 700 nm) and the second fluorescent dye absorbs light in a ultraviolet to blue region (wavelengths of 300 nm to 500 nm) to emit light having a longer wavelength than the absorbed light (wavelength of 430 nm to 575 nm, for example), wherein the first perylene fluorescent dye absorbs light containing the fluorescence emitted by the second fluorescent dye. The optional third fluorescent dye can absorb light from the second fluorescent dye and transmit light of longer wavelength to the first perylene fluorescent dye. The first perylene fluorescent dye is a compound of formula (B) 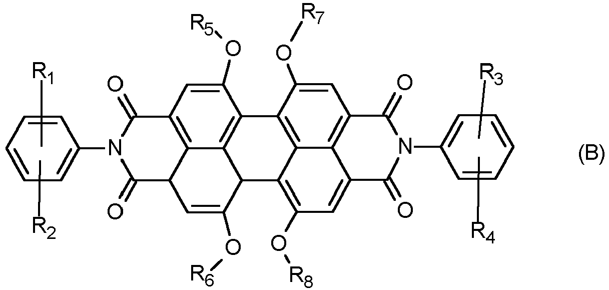 wherein R₁ to R₄ are each hydrogen, a linear alkyl group, a branched alkyl group or a cycloalkyl group, and may be substituted; R₅ to R₈ are a phenyl group, a heteroaromatic group, a linear alkyl group or a branched alkyl group, and may be substituted, and R₁ to R₈ may be the same or different. Specifically, the second fluorescent dye is a perylene fluorescent dye, coumarin dye, phthalocyanine dye, stilbene dye, cyanine dye, polyphenylene dye or rhodamine dye, preferably a core-unsubstituted perylene bisimide compound carrying on the imide nitrogen atoms a substituent selected from hydrogen, alkyl, and cycloalkyl. The third fluorescent dye is also a core-unsubstituted perylene bisimide compound, carrying on the imide nitrogen atoms a phenyl group optionally substituted by alkyl or cycloalkyl.

A disadvantage of known color converters is that only a portion of the photons emitted from the LED or OLED is converted into light of a longer wavelength and thus, the overall conversion efficiency is not always satisfactoy. Some of the known color converters comprising a combination of different organic fluorescent dyes suffer from different aging of the organic fluorescent dyes which cause prematurely undesirable color variation of the resulting total spectrum emitted. For white LEDs, the color converter often comprises a blue absorbing and green emitting or yellow emitting dye and a red emitting dye with low absorption of the blue LED light. Thus, a major problem is that the red emission (as a part of the outcoming white light) is only excited by the blue LED by direct excitation to a low extent. Instead the energy is transferred indirectly by absorption of the yellow dye which partly transfers its energy to the red emitting dye. This two-step process for excitation of excited red dyes is less efficient. There are additional losses for desactivation. Furthermore, conventional white LEDs often have reduced conversion efficiency due to multi-phosphors self-absorption losses, and reduced photometric conversion efficiency because of low human eye sensitivity for deep-red light within the broad spectrum of red phosphor emission. Thus, the fluorescence quantum yield needs to be optimized in addition to the excitation and the conversion efficiencies, in order to obtain efficient color converters.

Furthermore, the standard color converter-based white LED is often unsuitable for many applications in terms of its high color temperature.

Thus, there is a need to provide a color converter leading to a desired color temperature, preferably warm-white light, with high conversion efficiency and high photometric efficiency.

It was therefore an object of the invention to provide compounds having good optical properties, in particular good luminescent properties and which can be prepared in a manner advantageous in process technology terms, especially also on the industrial scale, via few synthetic stages. In particular, the compounds should have an excitation maximum which allows excitation with a LED, preferably a blue LED and emission in the wavelength range from 520 to 700 nm. In particular, the compounds should exhibit high extinction coefficient for wavelengths of the blue LED light. In addition, the compounds should have a high fluorescence quantum yield and high stability under the practical application conditions. They should preferably exhibit fluorescence quantum yields over 85%, preferably over 90%, in solid polymer matrices, especially polymer matrices selected from polycarbonate (PC), polyethylene terephtalate (PET) and polystyrene (PS), while having high thermal and photochemical stability. Moreover, the compound should be stable under the practical application conditions.

The compounds are further intended for use in color converters for converting light emitted from a light source into light of a second longer wavelength, in particular for converting light emitted from a blue LED or an OLED. Thus, the compounds should be stable under the application conditions during the lifetime of the complete light source. For use in color converters, the compounds embedded in a polymer matrix should have an absorption at the emission wavelength of the blue light source with high extinction coefficient. In addition, they should have high fluoresence quantum yield as well as high photostability under practical application conditions. The compounds are further intended for use in color converters for converting light emitted from a LED, especially a blue LED, into light of a second longer-wave wavelength in combination with further luminescent materials to provide white LEDs with high photometric conversion efficacy. A further object of the present invention is to provide a white-light emitting LED with natural-white color temperature or warm-white color temperature with high conversion efficacy through combining a blue LED with a green and/or yellow dye and red luminescent dyes embedded in a polymer matrix. A further object of the present invention is to provide a red-light emitting LED.

These and further objects are achieved by the 3,4;9,10-perylenetetracarboxylic diimide of the formula (I) described below. Similar compounds are known from DE1569841 B1.

### SUMMARY OF THE INVENTION

According to a first aspect of the present invention there is provided a 3,4;9,10-perylenetetracarboxylic diimide of the formula (I) 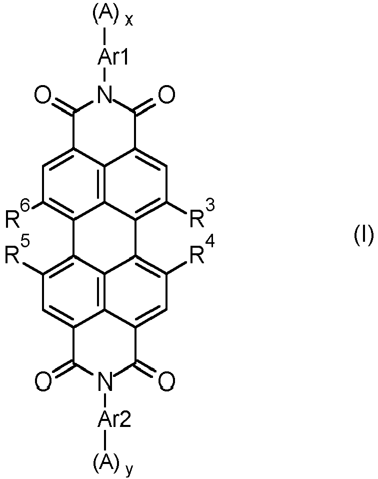 wherein the compound of the formula (I) comprises at least one group A
wherein
each group A is independently selected from a group of the following formula 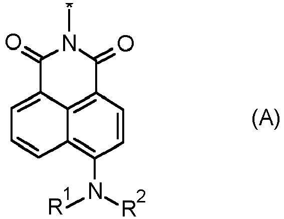 wherein
- *: denotes the bonding site to the remainder of the molecule;
- R¹ and R²,: independently of each other, are selected from hydrogen, C₁-C₃₀-alkyl, C₂-C₃₀-alkenyl, C₂-C₃₀-alkynyl, C₃-C₁₂-cycloalkyl and C₆-C₂₄-aryl wherein the two last-mentioned radicals are unsubstituted or carry one, two or three radicals selected from C₁-C₁₀-alkyl;
- x: is 0 or 1;
- y: is 0 or 1;
- Ar1: is C₆-C₂₄-aryl which does not bear a substituent R^{Ar1} or bears 1, 2 or 3 identical or different substituent(s) R^{Ar1}, where each R^{Ar1}, independently of each other, is selected from C₁-C₃₀-alkyl, C₁-C₃₀-haloalkyl, C₁-C₃₀-alkoxy, C₁-C₃₀-alkylsulfanyl, C₂-C₃₀-alkenyl and C₂-C₃₀-alkynyl;
- Ar2 is: C₆-C₂₄-aryl which does not bear a substituent R^{Ar2} or bears 1, 2 or 3 identical or different substituent(s) R^{Ar2}, where each R^{Ar2}, independently of each other, is selected from C₁-C₃₀-alkyl, C₁-C₃₀-haloalkyl, C₁-C₃₀-alkoxy, C₁-C₃₀-alkylsulfanyl, C₂-C₃₀-alkenyl and C₂-C₃₀-alkynyl;
- R³, R⁴, R⁵ and R⁶,: independently of each other, are selected from hydrogen and a group of formula (A)_{z}-Ar3-X-#,
wherein
- #: denotes the bonding site to the remainder of the molecule;
- z: is 0 or 1;
- X: is O or S;
- Ar3: C₆-C₂₄-aryl which does not bear a substituent R^{Ar3} or bears 1, 2 or 3 identical or different substituent(s) R^{Ar3}, wherein each R^{Ar3}, independently of each other, is selected from C₁-C₃₀-alkyl, C₁-C₃₀-haloalkyl, C₁-C₃₀-alkoxy, C₁-C₃₀-alkylsulfanyl, C₂-C₃₀-alkenyl, C₂-C₃₀-alkynyl, C₃-C₁₂-cycloalkyl and C₆-C₂₄-aryl.

The present invention also refers to mixtures of the compounds of formula (I).

The present invention also provides the use of a compound of the formula (I) or a mixture thereof as defined above and in the following, in color converters for converting light emitted from a light source, in particular a light source selected from LEDs and OLEDs, into light of a second, longer wavelength, for coloring coatings, printing inks and plastics, producing aqueous polymer dispersions which absorb and/or emit electromagnetic radiation, for data storage, for optical labels, for security labels in documents and for brand protection or as a fluorescent label for biomolecules.

The present invention also provides a color converter comprising at least one polymer as a matrix and at least one compound of the formula (I) or a mixture thereof as defined above and in the following as a fluorescent dye, wherein the at least one polymer is selected from polystyrene, polycarbonate, polymethylmethacrylate, polyvinylpyrrolidone, polymethacrylate, polyvinyl acetate, polyvinyl chloride, polybutene, silicone, polyacrylate, epoxy resin, polyvinyl alcohol, poly(ethylene vinylalcohol)-coplymer (EVA, EVOH), polyacrylonitrile, polyvinylidene chloride (PVDC), polystyreneacrylonitrile (SAN), polybutylene terephthalate (PBT), polyethylene terephthalate (PET), polyvinyl butyrate (PVB), polyvinyl chloride (PVC), polyamides, polyoxymethylenes, polyimides, polyetherimides and mixtures thereof.

The present invention also provides the use of a color converter as defined above and in the following for conversion of light generated by LEDs or OLEDs.

The present invention also provides a lighting device comprising at least one LED and at least one color converter as defined above and in the following.

The present invention also provides a device producing electric power upon illumination comprising a photovoltaic cell and the color converter as defined above and in the following, where at least a part of the light not absorbed by the photovoltaic cell is absorbed by the color converter.

The inventive perylenetetracarboxylic diimide of formula (I) is photostable, in particular stable in the presence of blue light. The compound of formula (I) has beneficial excitation and emission properties. The compound of formula (I) has an excitation maximum which allows excitation with a blue LED or OLED and has emission in the green, orange to red wavelength range from 520 to 700 nm with high fluorescence quantum yield. The compounds of formula (I) usually have a high extinction coefficient for blue LED light. The compound of formula (I) is insensitive to moisture and oxygen and can advantageously be used in a color converter for conversion of light generated by LEDs or OLEDs, in particular for generating warm-white light. In particular, the compound of formula (I) can be easily prepared via few synthetic stages. The combination of the inventive compound of formula I with a further fluorescent dye that can be excited by a blue LED results in pleasant light with high overall energy efficiency. In particular, a color converter which comprises the inventive compound of formula I in combination with a green and/or yellow fluorescent dye, together with blue light of a LED is able to provide white-light having any color temperature in the range between 2000 K and 7500 K with high overall energy efficiency.

Thus, a major advantage is that the inventive red-emitting dyes in combination with green-, greenish yellow and/or yellow emitting dyes dissolved or dispersed in a convenient matrix polymer allow to produce color-converting elements which exhibit higher conversion efficiencies compared to the mixture of non-inventive red-emitting dyes in combination with other dyes.

### BRIEF DESCRIPTION OF THE DRAWINGS

Figure 1 shows the absorption spectrum (broken line) and emission spectrum (solid line; excitation at 440nm) of the compound C1 in toluene (c = 10⁻⁴ mol/L). 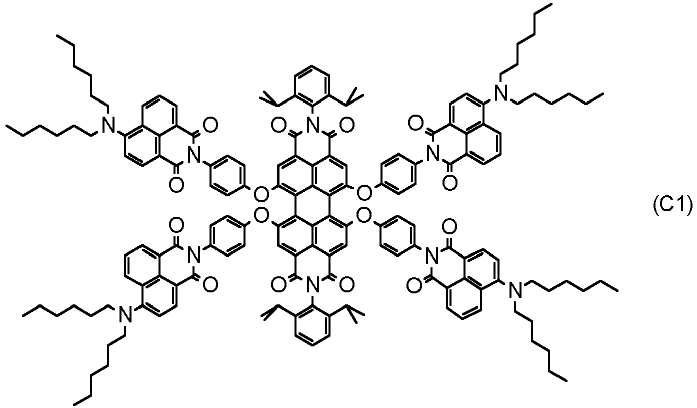
Figure 2 shows the absorbance spectrum (broken line) and the emission spectrum (solid line, excitation at 408 nm) of the compound C2 of example 2 in toluene (c = 10⁻⁴ mol/L).

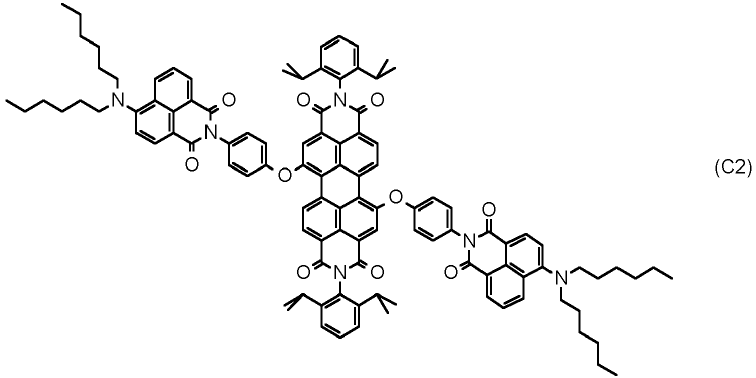

### EMBODIMENTS OF THE INVENTION

Specifically, the invention comprises the following preferred embodiments:
1. 3,4;9,10-Perylenetetracarboxylic diimide of the formula I 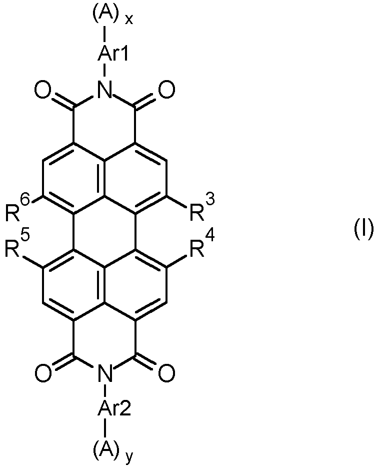 wherein the compound of the formula (I) comprises at least one group A
   wherein
   - each group A: is independently selected from a group of the following formula 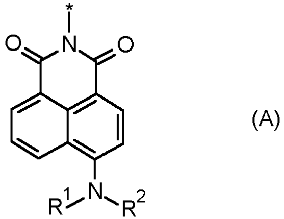 wherein
   * denotes the bonding site to the remainder of the molecule;
   R¹ and R², independently of each other, are selected from hydrogen, C₁-C₃₀-alkyl, C₂-C₃₀-alkenyl, C₂-C₃₀-alkynyl, C₃-C₁₂-cycloalkyl and C₆-C₂₄-aryl wherein the two last-mentioned radicals are unsubstituted or carry one, two or three radicals selected from C₁-C₁₀-alkyl;
   - x: is 0 or 1;
   - y: is 0 or 1;
   - Ar1: is C₆-C₂₄-aryl which does not bear a substituent R^{Ar1} or bears 1, 2 or 3 identical or different substituent(s) R^{Ar1}, where each R^{Ar1}, independently of each other, is selected from C₁-C₃₀-alkyl, C₁-C₃₀-haloalkyl, C₁-C₃₀-alkoxy, C₁-C₃₀-alkylsulfanyl, C₂-C₃₀-alkenyl and C₂-C₃₀-alkynyl;
   - Ar2: is C₆-C₂₄-aryl which does not bear a substituent R^{Ar2} or bears 1, 2 or 3 identical or different substituent(s) R^{Ar2}, where each R^{Ar2}, independently of each other, is selected from C₁-C₃₀-alkyl, C₁-C₃₀-haloalkyl, C₁-C₃₀-alkoxy, C₁-C₃₀-alkylsulfanyl, C₂-C₃₀-alkenyl and C₂-C₃₀-alkynyl;
   - R³, R⁴, R⁵ and R⁶,: independently of each other, are selected from hydrogen and a group of formula (A)_{z}Ar3-X-#,
   wherein
   # denotes the bonding site to the remainder of the molecule;
   z is 0 or 1;
   X is O or S;
   Ar3 C₆-C₂₄-aryl which does not bear a substituent R^{Ar3} or bears 1, 2 or 3 identical or different substituent(s) R^{Ar3}, wherein each R^{Ar3}, independently of each other, is selected from C₁-C₃₀-alkyl, C₁-C₃₀-haloalkyl, C₁-C₃₀-alkoxy, C₁-C₃₀-alkylsulfanyl, C₂-C₃₀-alkenyl, C₂-C₃₀-alkynyl, C₃-C₁₂-cycloalkyl and C₆-C₂₄-aryl.
2. 3,4;9,10-Perylenetetracarboxylic diimide of the formula I according to embodiment 1, where R¹ and R², independently of each other, are selected from C₁-C₂₀-alkyl.
3. 3,4;9,10-Perylenetetracarboxylic diimide of the formula I according to embodiment 1 or 2, where
   Ar1 is selected from phenyl which carries 1 or 2 C₁-C₆-alkyl substituents R^{Ar1} and phenyl which carries one group A; and
   Ar2 is selected from phenyl which carries 1 or 2 C₁-C₆-alkyl substituents R^{Ar2} and phenyl which carries one group A.
4. 3,4;9,10-Perylenetetracarboxylic diimide of the formula I according to any of the preceding claims, where R³, R⁴, R⁵ and R⁶, independently of each other, are selected from hydrogen, phenoxy, which does neither bear a substituent R^{Ar3} nor a group A and phenoxy which carries one group A.
5. 3,4;9,10-Perylenetetracarboxylic diimide of the formula I according to any of the preceding claims, wherein two of the radicals R³, R⁴, R⁵ and R⁶ are hydrogen and the other radicals R³, R⁴, R⁵ and R⁶ are selected from phenoxy, which does neither carry a radical R^{Ar3} nor a group A and phenoxy which carries one group A.
6. 3,4;9,10-Perylenetetracarboxylic diimide of the formula I according to any of embodiments 1 to 4, wherein the radicals R³, R⁴, R⁵ and R⁶ have the same meaning.
7. 3,4;9,10-Perylenetetracarboxylic diimide of the formula I according to any of the preceding embodiments selected from 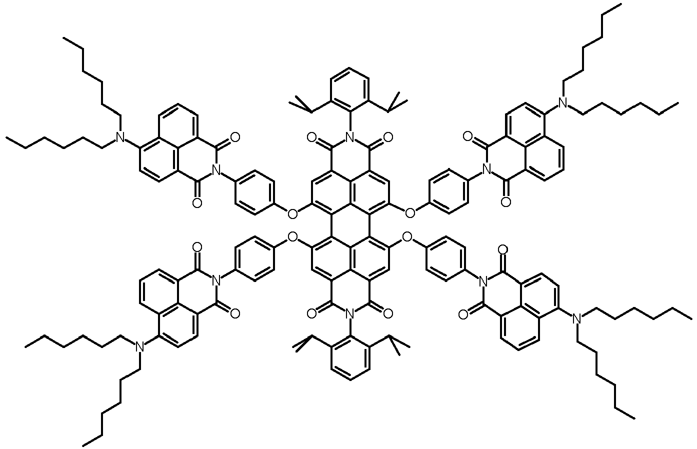 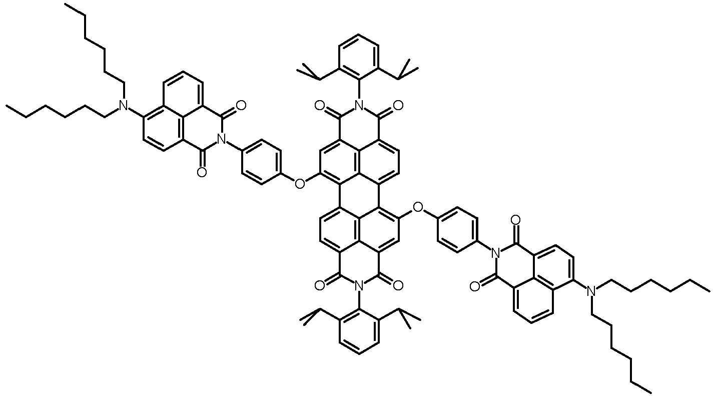 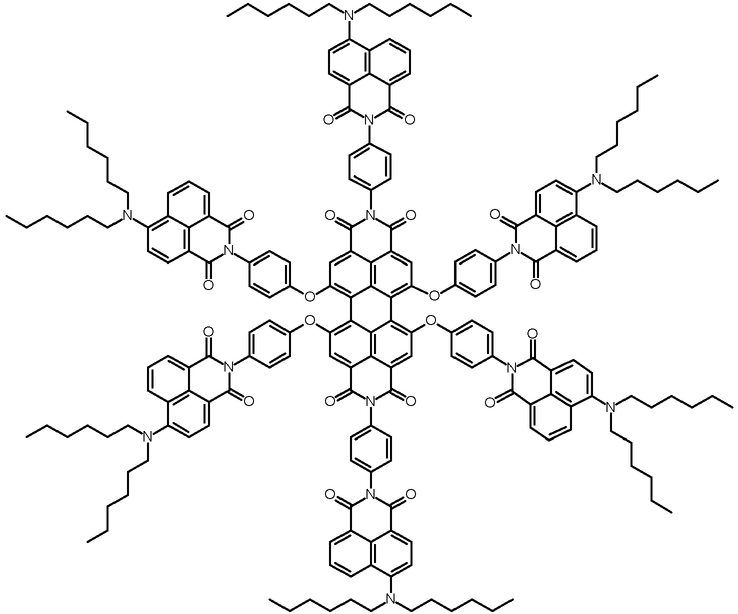 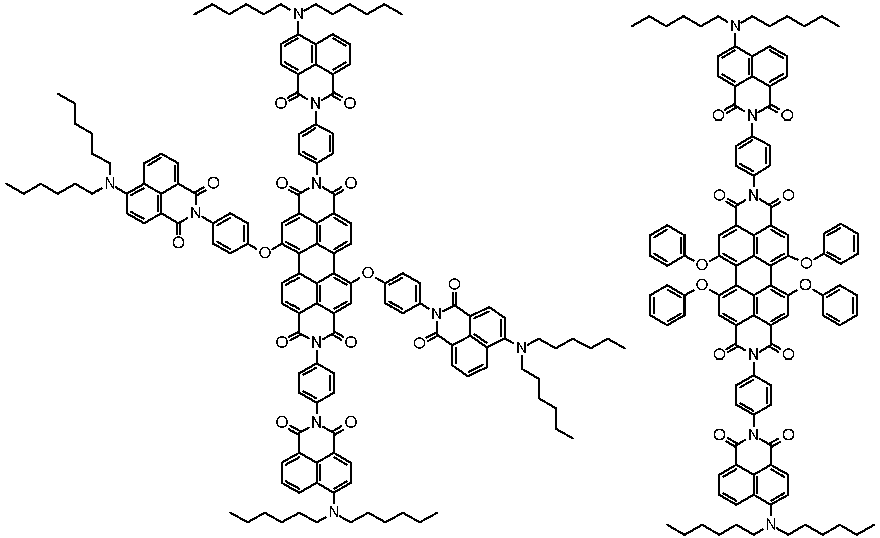 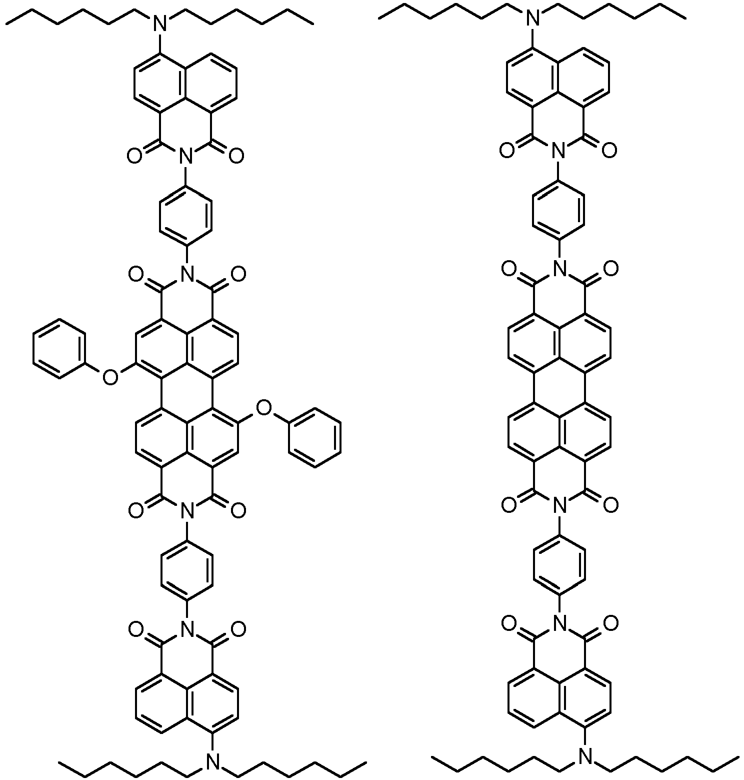
8. A color converter comprising at least one polymer as a matrix and at least one compound of the formula I or a mixture thereof as defined in any of embodiments 1 to 7 as a fluorescent dye, wherein the at least one polymer is selected from polystyrene, polycarbonate, polymethylmethacrylate, polyvinylpyrrolidone, polymethacrylate, polyvinyl acetate, polyvinyl chloride, polybutene, silicone, polyacrylate, epoxy resin, polyvinyl alcohol, poly(ethylene vinylalcohol)-coplymer (EVA, EVOH), polyacrylonitrile, polyvinylidene chloride (PVDC), polystyreneacrylonitrile (SAN), polybutylene terephthalate (PBT), polyethylene terephthalate (PET), polyvinyl butyrate (PVB), polyvinyl chloride (PVC), polyamides, polyoxymethylenes, polyimides, polyetherimides and mixtures thereof,
9. The color converter according to embodiment 8, wherein the at least one polymer consists essentially of polystyrene, polycarbonate, or polyethylene terephthalate.
10. The color converter according to embodiment 8or 9, wherein the color converter additionally comprises at least one inorganic white pigment as a scattering body.
11. The color converter according to any of embodiments 8 to 10, comprising at least a further fluorescent material selected from inorganic fluorescent material and further organic fluorescent dyes.
12. The use of a color converter as defined in any of embodiments 8 to 12, for conversion of light generated by LEDs or OLEDs.
13. The use of a color converter as defined in embodiment 13 for conversion of light generated by a blue LED or by a white LED.
14. The use of a color converter as defined in any of embodiments 8 to 12 in displays.
15. A lighting device comprising at least one LED and at least one color converter according to any of embodiments 7 to 11, the LED and color converter being preferably in a remote phosphor arrangement.
16. A device producing electric power upon illumination comprising a photovoltaic cell and the color converter as defined in any of embodiments 8 to 12, where at least a part of the light not absorbed by the photovoltaic cell is absorbed by the color converter.
17. The use of a compound of the formula I or a mixture thereof as defined in any of embodiments 1 to 7, in color converters for converting light emitted from a light source, in particular a light source selected from LEDs and OLEDs, into light of a second, longer wavelength, for coloring coatings, printing inks and plastics, producing aqueous polymer dispersions which absorb and/or emit electromagnetic radiation, for data storage, for optical labels, for security labels in documents and for brand protection or as a fluorescent label for biomolecules.

### DETAILED DESCRIPTION OF INVENTION

The definitions of the variables specified in the above formulae use collective terms which are generally representative of the respective substituents. The definition Cₙ-Cₘ gives the number of carbon atoms possible in each case in the respective substituent or substituent moiety.

In the context of the present invention, a "blue LED" is understood to mean a LED which emits light in the blue range of the electromagnetic spectrum, i.e. in the wavelength range from 400 to 500 nm, preferably 420 to 480 nm and especially 440 to 470 nm. Suitable semiconductor materials are silicon carbide, zinc selenide and nitrides such as aluminum nitride (AIN), gallium nitride (GaN), indium nitride (InN) and indium gallium nitride (InGaN). In the context of the present invention, a "green LED" is understood to mean an LED which emits light in the wavelength range from 501 to 560 nm, preferably 501 to 540 nm and especially 520 to 540 nm. Suitable semiconductor materials are for example based on GalnNAs.

In the context of the present invention, a "white LED" is understood to mean an LED which produces white light. Examples of a white LED are multi-LEDs or a blue LED in combination with at least one radiation converting luminophore.

In the context of the present invention, a "red LED" is understood to mean a LED which emits light with wavelengths from 590 nm to 700 nm.

In the context of the present invention, "color converter" is understood to mean all physical devices capable of absorbing light of particular wavelengths and converting it to light of second wavelengths. Color converters are, for example, part of lighting devices, especially those lighting devices which utilize UV light or LEDs or OLEDs as a light source, or of fluorescence conversion solar cells. Thus, the blue light may be (at least) partly converted into visible light of higher wavelengths than the excitation wavelengths.

A quantum dot is a nanocrystal made of semiconductor materials that is small enough to exhibit quantum mechanical properties. The quantum dot has a defined emission wavelength which is not subject to aging and is also photochemically stable. The color output of the dots can be tuned by controlling the size of the crystals. With a smaller size in quantum dots, the quantum dots emit light of a shorter wavelength.

The luminous efficacy of a source is a measure for the efficiency with which the source provides visible light and is measured in lumens per watt. Not all wavelengths of light are equally visible, or equally effective at stimulating human vision, due to the spectral sensitivity of the human eye. The overall luminous efficacy of a source is the product of how well it converts energy to electromagnetic radiation, and how well the emitted radiation is detected by the human eye.

The word "essentially" in the context of the present invention encompasses the words "completely", "wholly" and "all". The word encompasses a proportion of 90% or more, such as 95% or more, especially 99% or 100%.

The definitions of the variables specified in the above formulae use collective terms which are generally representative of the respective substituents. The definition Cₙ-Cₘ gives the number of carbon atoms possible in each case in the respective substituent or substituent moiety.

The expression "halogen" denotes in each case fluorine, bromine, chlorine or iodine, particularly chlorine, bromide or iodine.

The term "aliphatic radical" refers to an acyclic saturated or unsaturated, straight-chain or branched hydrocarbon radical. Usually the aliphatic radical has 1 to 100 carbon atoms. Examples for an aliphatic radical are alkyl, alkenyl and alkynyl.

The term "cycloaliphatic radical" refers to a cyclic, non-aromatic saturated or unsaturated hydrocarbon radical having usually 3 to 20 ring carbon atoms. Examples are cycloalkanes, cycloalkenes, and cycloalkynes. The cycloaliphatic radical may also heteroatoms or heteroatom groups.

The term "alkyl" as used herein and in the alkyl moieties of alkoxy, alkylthio, alkylsulfinyl, alkylsulfonyl, alkylcarbonyl, alkoxycarbonyl and the like refers to saturated straight-chain or branched hydrocarbon radicals having 1 to 100 ("C₁-C₁₀₀-alkyl"), 1 to 30 ("C₁-C₃₀-alkyl"),1 to 18 ("C₁-C₁₈-alkyl"), 1 to 12 ("C₁-C₁₂-alkyl"), 1 to 8 ("C₁-C₈-alkyl") or 1 to 6 ("C₁-C₆-alkyl") carbon atoms. Alkyl is preferably C₁-C₂₄-alkyl, more preferably C₁-C₂₀-alkyl. Examples of alkyl groups are especially methyl, ethyl, n-propyl, isopropyl, n-butyl, isobutyl, sec-butyl, tert-butyl, n-pentyl, 1-methylbutyl, 1-ethylpropyl, neo-pentyl, n-hexyl, 1-methylpentyl, 2-methylpentyl, 1-ethylbutyl, 2-ethylbutyl, n-heptyl, 1-methylhexyl, 2-methylhexyl, 1-ethylpentyl, 1-propylbutyl, 2-ethylpentyl, n-octyl, 1-methylheptyl, 2-methylheptyl, 1-ethylhexyl, 2-ethylhexyl, 1-propylpentyl, 2-propylpentyl, n-nonyl, 1-methyloctyl, 2-methyloctyl, 1-ethylheptyl, 2-ethylheptyl, 1-propylhexyl, 2-propylhexyl, 1-butylpentyl, n-decyl, 2-methyldecyl, 1-methylnonyl, 2-methylnonyl, 1-ethyloctyl, 2-ethyloctyl, 1-propylheptyl, 2-propylheptyl, 1-butylhexyl, 2-butylhexyl, n-undecyl, 2-ethylnonyl, 1-propyloctyl, 2-propyloctyl, 1-butylheptyl, 2-butylheptyl, 1-pentylhexyl, n-dodecyl, 2-ethyldecyl, 2-propylnonyl, 1-butyloctyl, 2-butyloctyl, 1-pentylheptyl, 2-pentylheptyl, 2-propyldecyl, n-tridecyl, 1-pentyloctyl, 2-pentyloctyl, 1-hexylheptyl, 2-butylnonyl, n-tetradecyl, 1-hexyloctyl, 2-hexyloctyl, 2-pentylnonyl, 2-hexylnonyl, 2-pentyldecyl, 2-butyldecyl,n-hexadecyl, 1-heptyloctyl, 2-heptylnonyl, 2-hexyldecyl, 2-heptyldecyl, n-octadecyl, 2-octyldecyl, n-eicosyl, 2-nonylundecyl, 2-octylundecyl, 2-heptylundecyl, 2-hexylundecyl, 2-pentylundecyl, 2-butylundecyl, 2-propylundecyl, 2-ethylundecyl, 2-methylundecyl, 2-decyldodecyl, 2-nonyldodecyl, 2-octyldodecyl, 2-heptyldodecyl, 2-hexyldodecyl, 2-pentyldodecyl, 2-butyldodecyl, 2-propyldodecyl, 2-ethyldodecyl, 2-methyldodecyl, 2-undecyltridecyl, 2-decyltridecyl, 2-nonyltridecyl, 2-octyltridecyl, 2-heptyltridecyl, 2-hexyltridecyl, 2-pentyltridecyl, 2-butyltridecyl, 2-propyltridecyl, 2-ethyltridecyl, 2-methyltridecyl, 2-undecyltetradecyl, 2-decyltetradecyl, 2-nonyltetradecyl, 2-octyltetradecyl, 2-hetyltetradecyl, 2-hexyltetradecyl, 2-pentyltetradecyl, 2-butyltetradecyl, 2-propyltetradecyl, 2-ethyltetradecyl, 2-methyltetradecyl, 2-tetradecylhexadecyl, 2-tridecylhexadecyl, 2-dodecylhexadecyl, 2-undecylhexadecyl, 2-decylhexadecyl, 2-nonylhexadecyl, 2-octylhexadecyl, 2-heptylhexadecyl, 2-hexylhexadecyl, 2-pentylhexadecyl, 2-butylhexadecyl, 2-propylhexadecyl, 2-ethylhexadecyl, 2-methylhexadecyl, 2-dodecyloctadecyl, 2-undecyloctadecyl, 2-decyloctadecyl, 2-nonyloctadecyl, 2-octyloctadecyl, 2-heptyloctadecyl, 2-hexyloctadecyl, 2-pentyloctadecyl, 2-butyloctadecyl, 2-propyloctadecyl, 2-ethyloctadecyl, 2-methyloctadecyl, 2-decyleicosanyl, 2-nonyleicosanyl, 2-octyleicosanyl, 2-heptyleicosanyl, 2-hexyleicosanyl, 2-pentyleicosanyl, 2-butyleicosanyl, 2-propyleicosanyl, 2-ethyleicosanyl, 2-methyleicosanyl, 2-octadecyldocosanyl, 2-heptadecyldocosanyl, 2-hexadecyldocosanyl, 2-pentadecyldocosanyl, 2-tetradecyldocosanyl, 2-tridecyldocosanyl, 2-undecyldocosanyl, 2-decyldocosanyl, 2-nonyldocosanyl, 2-octyldocosanyl, 2-heptyldocosanyl, 2-hexyldocosanyl, 2-pentyldocosanyl, 2-butyldocosanyl, 2-propyldocosanyl, 2-ethyldocosanyl, 2-methyldocosanyl, 2-docosanyltetracosanyl, 2-hexadecyltetracosanyl, 2-pentadecyltetracosanyl, 2-pentadecyltetracosanyl, 2-tetradecyltetracosanyl, 2-tridecyltetracosanyl, 2-dodecyltetracosanyl, 2-undecyltetracosanyl, 2-decyltetracosanyl, 2-nonyltetracosanyl, 2-octyltetracosanyl, 2-heptyltetracosanyl, 2-hexyltetracosanyl, 2-pentyltetracosanyl, 2-butyltetracosanyl, 2-propyltetracosanyl, 2-ethyltetracosanyl, 2-methyltetracosanyl, 2-dodecyloctacosanyl, 2-undecyloctacosanyl, 2-decyloctacosanyl, 2-nonyloctacosanyl, 2-octyloctacosanyl, 2-heptyloctacosanyl, 2-hexyloctacosanyl, 2-pentyloctacosanyl, 2-butyloctacosanyl, 2-propyloctacosanyl, 2-ethyloctacosanyl and 2-methyloctacosanyl.

Haloalkyl and all haloalkyl moieties in haloalkoxy: straight-chain or branched alkyl groups having usually 1 to 24, frequently 1 to 20 carbon atoms (as specified above), where some or all of the hydrogen atoms in these groups are replaced by halogen atoms as specified above.

The expression alkyl also comprises alkyl radicals whose carbon chains may be interrupted by one or more nonadjacent groups which are selected from -O-, -Sand -NR^{cc}. R^{cc} is hydrogen, C₁-C₂₀-alkyl, C₃-C₂₄-cycloalkyl, heterocycloalkyl, C₆-C₂₄-aryl or heteroaryl. The above remarks regarding alkyl also apply to the alkyl moiety in alkoxy, alkylthio (= alkylsulfanyl), monoalkylamino, dialkylamino and the alkylene moiety in aryl-C₁-C₁₀-alkylene.

Examples of alkyl groups whose carbon chains are interrupted by one or more, e.g. 1, 2, 3, 4, 5, 6., 7, 8 or more than 8, nonadjacent groups are especially 2-methoxyethyl, 2-ethoxyethyl, 2-propoxyethyl, 2-isopropoxyethyl, 2-butoxyethyl, 2- and 3-methoxypropyl, 2- and 3-ethoxypropyl, 2- and 3-propoxypropyl, 2- and 3-butoxypropyl, 2- and 4-methoxybutyl, 2- and 4-ethoxybutyl, 2- and 4-propoxybutyl, 3,6-dioxaheptyl, 3,6-dioxaoctyl, 4,8-dioxanonyl, 3,7-dioxaoctyl, 3,7-dioxanonyl, 4,7-dioxaoctyl, 4,7-dioxanonyl, 2- and 4-butoxybutyl, 4,8-dioxadecyl, 3,6,9-trioxadecyl, 3,6,9-trioxaundecyl, 3,6,9-trioxadodecyl, 3,6,9,12-tetraoxatridecyl and 3,6,9,12-tetra-oxatetradecyl; 2-methylthioethyl, 2-ethylthioethyl, 2-propylthioethyl, 2-isopropylthio-ethyl, 2-butylthioethyl, 2- and 3-methylthiopropyl, 2- and 3-ethylthiopropyl, 2- and 3-propylthiopropyl, 2- and 3-butylthiopropyl, 2- and 4-methylthiobutyl, 2- and 4-ethyl-thiobutyl, 2- and 4-propylthiobutyl, 3,6-dithiaheptyl, 3,6-dithiaoctyl, 4,8-dithianonyl, 3,7-dithiaoctyl, 3,7-dithianonyl, 2- and 4-butylthiobutyl, 4,8-dithiadecyl, 3,6,9-tri-thiadecyl, 3,6,9-trithiaundecyl, 3,6,9-trithiadodecyl, 3,6,9,12-tetrathiatridecyl and 3,6,9,12-tetrathiatetradecyl; 2-monomethyl- and 2-monoethylaminoethyl, 2-dimethylaminoethyl, 2- and 3-dimethylaminopropyl, 3-monoisopropylaminopropyl, 2-and 4-monopropylaminobutyl, 2- and 4-dimethylaminobutyl, 6-methyl-3,6-diazaheptyl, 3,6-dimethyl-3,6-diazaheptyl, 3,6-diazaoctyl, 3,6-dimethyl-3,6-diazaoctyl, 9-methyl-3,6,9-triazadecyl, 3,6,9-trimethyl-3,6,9-triazadecyl, 3,6,9-triazaundecyl, 3,6,9-trimethyl-3,6,9-triazaundecyl, 12-methyl-3,6,9,12-tetraazatridecyl and 3,6,9,12-tetramethyl-3,6,9,12-tetraazatridecyl; (1-ethylethylidene)aminoethylene, (1-ethylethylidene)-aminopropylene, (1-ethylethylidene)aminobutylene, (1-ethylethylidene)aminodecylene and (1-ethylethylidene)aminododecylene.

Alkylene represents a linear or branched saturated hydrocarbon chain having from 1 to 10 and especially from 1 to 6 carbon atoms, such as linear C₁-C₆-alkylene, e.g. methylene (-CH₂-), ethane-1,2-diyl, propane-1,3-diyl, butane-1,4-diyl, pentane-1,5-diyl or hexane-1,6-diyl and branched C₂-C₆-alkylene, such as ethane-1,1-diyl, propane-1,2-diyl, butane-1,2-diyl or butane-1,3-diyl.

The term "alkenyl" as used herein refers to straight-chain or branched hydrocarbon groups having 2 to 100 ("C₂-C₁₀₀-alkenyl"), 2 to 30 ("C₂-C₃₀-alkenyl"), 2 to 18 ("C₂-C₁₈-alkenyl"), 2 to 10 ("C₂-C₁₀-alkenyl"), 2 to 8 ("C₂-C₈-alkenyl"), or 2 to 6 ("C₂-C₆-alkenyl") carbon atoms and one or more, e.g. 2 or 3, double bonds in any position.

The term "alkynyl" as used herein (also referred to as alkyl whose carbon chain may comprise one or more triple bonds) refers to straight-chain or branched hydrocarbon groups having 2 to 100 ("C₂-C₁₀₀-alkynyl"), 2 to 30 ("C₂-C₃₀-alkynyl"), 2 to 18 ("C₂-C₁₈-alknyl"), 2 to 10 ("C₂-C₁₀-alkynyl"), 2 to 8 ("C₂-C₈-alkynyl"), or 2 to 6 ("C₂-C₆-alkynyl") carbon atoms and one or more, e.g. 2 or 3, triple bonds in any position.

The term "cycloalkyl" as used herein refers to mono- or bi- or polycyclic saturated hydrocarbon radicals having 3 to 24 (C₃-C₂₄-cycloalkyl), 3 to 20 ("C₃-C₂₀-cycloalkyl"), 3 to 12 ("C₃-C₁₂-cycloalkyl") toms, preferably 3 to 8 ("C₃-C₈-cycloalkyl") or 3 to 6 carbon atoms ("C₃-C₆-cycloalkyl"). Examples of monocyclic radicals having 3 to 6 carbon atoms comprise cyclopropyl, cyclobutyl, cyclopentyl and cyclohexyl. Examples of monocyclic radicals having 3 to 8 carbon atoms comprise cyclopropyl, cyclobutyl, cyclopentyl, cyclohexyl, cycloheptyl and cyclooctyl. Examples of bicyclic radicals having 7 to 12 carbon atoms comprise bicyclo[2.2.1]heptyl, bicyclo[3.1.1]heptyl, bicyclo[2.2.2]octyl, bicyclo[3.3.0]octyl, bicyclo[3.2.1]octyl, bicyclo[3.3.1]nonyl, bicyclo[4.2.1]nonyl, bicyclo[4.3.1]decyl, bicyclo[3.3.2]decyl, bicyclo[4.4.0]decyl, bicyclo[4.2.2]decyl, bicyclo[4.3.2] undecyl, bicyclo[3.3.3]undecyl, bicyclo[4.3.3]dodecyl,and perhydronaphthyl. Examples of polycyclic rings are perhydroanthracyl, perhydrofluorenyl, perhydrochrysenyl, perhydropicenyl, and adamantly.

The term heterocycloalkyl refers to nonaromatic, partially unsaturated or fully saturated, heterocyclic rings having generally 5 to 8 ring members, preferably 5 or 6 ring members, comprising besides carbon atoms as ring members, one, two, three or four heteroatoms or heteroatom-containing groups selected from O, N, NR^{cc}, S, SO and S(O)₂ as ring members, wherein R^{cc} is hydrogen, C₁-C₂₀-alkyl, C₃-C₂₄-cycloalkyl, heterocycloalkyl, C₆-C₂₄-aryl or heteroaryl. Examples of heterocycloalkyl groups are especially pyrrolidinyl, piperidinyl, imidazolidinyl, pyrazolidinyl, oxazolidinyl, morpholinyl, thiazolidinyl, isothiazolidinyl, isoxazolidinyl, piperazinyl, tetrahydrothiophenyl, dihydrothien-2-yl, tetrahydrofuranyl, dihydrofuran-2-yl, tetrahydropyranyl, 2-oxazolinyl, 3-oxazolinyl, 4-oxazolinyl and dioxanyl.

The expression C₆-C₂₄-aryl refers to an aromatic radical having 6 to 24 carbon atoms, preferably 6 to 20 carbon atoms, especially 6 to 14 carbon atoms as ring members. Aryl is preferably phenyl, naphthyl, indenyl, fluorenyl, anthracenyl, phenanthrenyl, naphthacenyl, chrysenyl, pyrenyl, coronenyl, perylenyl, etc., and more preferably phenyl or naphthyl.

Aryl which bears one or more C₁-C₂₄-alkyl radicals is, for example, 2-, 3- and 4-methylphenyl, 2,4-, 2,5-, 3,5- and 2,6-dimethylphenyl, 2,4,6-trimethylphenyl, 2-, 3-and 4-ethylphenyl, 2,4-, 2,5-, 3,5- and 2,6-diethylphenyl, 2,4,6-triethylphenyl, 2-, 3- and 4-propylphenyl, 2,4-, 2,5-, 3,5- and 2,6-dipropylphenyl, 2,4,6-tripropylphenyl, 2-, 3- and 4-isopropylphenyl, 2,4-, 2,5-, 3,5- and 2,6-diisopropylphenyl, 2,4,6-triisopropylphenyl, 2-, 3- and 4-butylphenyl, 2,4-, 2,5-, 3,5- and 2,6-dibutylphenyl, 2,4,6-tributylphenyl, 2-, 3- and 4-isobutylphenyl, 2,4-, 2,5-, 3,5- and 2,6-diisobutylphenyl, 2,4,6-triisobutylphenyl, 2-, 3- and 4-sec-butylphenyl, 2,4-, 2,5-, 3,5- and 2,6-di-sec-butylphenyl, 2,4,6-tri-sec-butylphenyl, 2-, 3- and 4-tert-butylphenyl, 2,4-, 2,5-, 3,5- and 2,6-di-tert-butylphenyl and 2,4,6-tri-tert-butylphenyl.

The term C₆-C₂₄-aryl-C₁-C₁₀-alkylene refers to an aromatic radical having 6 to 24 carbon atoms as defined above, which is bound to the remainder of the molecule via a C₁-C₁₀-alkylene group, as defined above, in particular via a methylene, 1,1-ethylene or 1,2-ethylene group. Examples for C₆-C₂₄-aryl-C₁-C₁₀-alkylene are benzyl, 1-phenylethyl and 2-phenylethyl, phenylpropyl, naphthylmethyl, naphthylethyl etc.

The term "heteroaryl" (also referred to as hetaryl) comprises heteroaromatic, mono- or polycyclic groups. In addition to the ring carbon atoms, these have 1, 2, 3, 4 or more than 4 heteroatoms as ring members. The heteroatoms are preferably selected from oxygen, nitrogen, selenium and sulfur. The heteroaryl groups have preferably 5 to 18, e.g. 5, 6, 8, 9, 10, 11, 12, 13 or 14, ring atoms.

Monocyclic heteroaryl groups are preferably 5- or 6-membered heteroaryl groups, such as 2 furyl (furan-2-yl), 3-furyl (furan-3-yl), 2-thienyl (thiophen-2-yl), 3 thienyl (thiophen-3 yl), selenophen-2-yl, selenophen-3-yl, 1H-pyrrol-2-yl, 1H-pyrrol-3-yl, pyrrol-1-yl, imidazol-2-yl, imidazol-1-yl, imidazol-4-yl, pyrazol-1-yl, pyrazol-3-yl, pyrazol-4-yl, pyrazol-5-yl, 3-isoxazolyl, 4-isoxazolyl, 5-isoxazolyl, 3 isothiazolyl, 4-isothiazolyl, 5-isothiazolyl, 2-oxazolyl, 4-oxazolyl, 5-oxazolyl, 2-thiazolyl, 4-thiazolyl, 5-thiazolyl, 1,2,4-oxadiazol-3-yl, 1,2,4-oxadiazol-5-yl, 1,3,4 oxadiazol 2 yl, 1,2,4 thiadiazol-3-yl, 1,2,4-thiadiazol-5-yl, 1,3,4-thiadiazol-2-yl, 4H-[1,2,4]-triazol-3-yl, 1,3,4-triazol-2-yl, 1,2,3-triazol-1-yl, 1,2,4-triazol-1-yl, pyridin-2-yl, pyridin-3-yl, pyridin-4-yl, 3 pyridazinyl, 4-pyridazinyl, 2-pyrimidinyl, 4-pyrimidinyl, 5-pyrimidinyl, 2 pyrazinyl, 1,3,5-triazin-2-yl and 1,2,4-triazin-3-yl.

Polycyclic heteroaryl groups have 2, 3, 4 or more than 4 fused rings. The fused-on rings may be aromatic, saturated or partly unsaturated. Examples of polycyclic heteroaryl groups are quinolinyl, isoquinolinyl, indolyl, isoindolyl, indolizinyl, benzofuranyl, isobenzofuranyl, benzothiophenyl, benzoxazolyl, benzisoxazolyl, benzthiazolyl, benzoxadiazolyl, benzothiadiazolyl, benzoxazinyl, benzopyrazolyl, benzimidazolyl, benzotriazolyl, benzotriazinyl, benzoselenophenyl, thienothiophenyl, thienopyrimidyl, thiazolothiazolyl, dibenzopyrrolyl (carbazolyl), dibenzofuranyl, dibenzothiophenyl, naphtho[2,3-b]thiophenyl, naphtha[2,3-b]furyl, dihydroindolyl, dihydroindolizinyl, dihydroisoindolyl, dihydroquinolinyl and dihydroisoquinolinyl.

The term "aryloxy" as used herein refers to an aromatic radical having 6 to 24 carbon atoms, preferably 6 to 20 carbon atoms, especially 6 to 14 carbon atoms as ring members which is attached via an oxygen atom to the remainder of the molecule. Preference is given to phenoxy and naphthyloxy.

The term "arylthio" as used herein refers to an aromatic radical having 6 to 24 carbon atoms, preferably 6 to 20 carbon atoms, especially 6 to 14 carbon atoms as ring members which is attached via a sulfur atom to the remainder of the molecule. Preference is given to phenylthio and naphthylthio.

Preferred embodiments and further aspects of the present invention are outlined in the following paragraphs:
The remarks made below concerning preferred embodiments of the variables of the compounds of formula (I), especially with respect to the variables x, y, z and their substituents Ar1, Ar2, Ar3, R³, R⁴, R⁵, R⁶, A, is valid for the compounds according to the invention per se as well as for the use thereof.

The compounds of formula (I) according to the invention are substituted by at least one group A. Preference is given to compounds of formula (I), which carry 2, 4 or 6 groups A.

In the context of group A, the R¹ and R² radicals may have identical or different definitions. In a preferred embodiment, the R¹ and R² radicals have identical definitions.

Preferred are compounds of the formula (I), where R¹ and R², independently of each other, are selected from C₁-C₂₀-alkyl. More preferably, R¹ and R² are independently of each other linear or branched C₅-C₂₀-alkyl, with preference given to linear C₅-C₂₀-alkyl such as n-pentyl, n-hexyl, n-heptyl, n-octyl, n-nonyl, n-decyl, n-undecyl, n-dodecyl, n-tridecyl, n-tetradecyl, n-pentadecyl, n-hexadecyl, n-heptadecyl, n-octadecyl or n-eicosyl.

Preferred are compounds of formula (I), where x is 1, i.e. one group A is attached to Ar1, where A is as defined above. Reference is made to the afore-mentioned preferred meanings of A. In this context, Ar1 is preferably phenyl or naphthyl, in particular phenyl. Especially more preferred are compounds of formula (I), wherein Ar1 is phenyl which carries a group A in the para-position.

Preferred are also compounds of formula (I), where y is 1, i.e., one group A is attached to Ar2, where A is as defined above. Reference is made to the afore-mentioned preferred meanings of A. In this context, Ar2 is preferably phenyl or naphthyl, in particular phenyl. Especially more preferred are compounds of formula (I), wherein Ar2 is phenyl which carries a group A in the para-position.

Preferred are also compounds of formula (I), where Ar1 is C₆-C₂₄-aryl which bears 1, 2 or 3 identical or different substituent(s) R^{Ar1}. More preferably, Ar1 is phenyl or naphthyl, where the aromatic ring of phenyl and naphthyl carries 1, 2 or 3 identical or different radicals R^{Ar1}. Irrespective of its occurrence, R^{Ar1} is preferably C₁-C₁₀-alkyl such methyl, ethyl, n-propyl, isopropyl, n-butyl, isobutyl, sec-butyl, tert-butyl, n-pentyl, 1-methylbutyl, 1-ethylpropyl, neo-pentyl, n-hexyl, 1-methylpentyl, 2-methylpentyl, 1-ethylbutyl, 2-ethylbutyl, n-heptyl, 1-methylhexyl, 2-methylhexyl, 1-ethylpentyl, 1-propylbutyl, 2-ethylpentyl, n-octyl, 1-methylheptyl, 2-methylheptyl, 1-ethylhexyl, 2-ethylhexyl, 1-propylpentyl, 2-propylpentyl, n-nonyl, 1-methyloctyl, 2-methyloctyl, 1-ethylheptyl, 2-ethylheptyl, 1-propylhexyl, 2-propylhexyl, 1-butylpentyl, n-decyl.

Particularly preferred are compounds of formula (I), wherein Ar1 is selected from phenyl which carries one or two C₁-C₆-alkyl substituent(s) R^{Ar1}. Even more preferred are compounds of formula (I), wherein Ar1 is phenyl which carries two C₁-C₆-alkyl substituents R^{Ar1}. The phenyl radical is preferably substituted by only two substituents R^{Ar1} that are in the ortho- and ortho'-position (2- and 6-position) or in the ortho and para-position (2- and 4-position). Especially more preferred are compounds (I), where Ar1 is 2,6-(di-C₁-C₄-alkyl)phenyl or 2,4-(di-C₁-C₄-alkyl)phenyl, even more especially Ar1 is 2,6-(di-C₁-C₄-alkyl)phenyl.

Preferred are also compounds of formula (I), where Ar2 is C₆-C₂₄-aryl which carries 1, 2 or 3 identical or different substituent(s) R^{Ar2}. More preferably, Ar2 is phenyl or naphthyl, where the aromatic ring of phenyl and naphthyl carries 1, 2 or 3 identical or different radical(s) R^{Ar2}. Irrespective of its occurrence, R^{Ar2} is preferably C₁-C₁₀-alkyl such as methyl, ethyl, n-propyl, isopropyl, n-butyl, isobutyl, sec-butyl, tert-butyl, n-pentyl, 1-methylbutyl, 1-ethylpropyl, neo-pentyl, n-hexyl, 1-methylpentyl, 2-methylpentyl, 1-ethylbutyl, 2-ethylbutyl, n-heptyl, 1-methylhexyl, 2-methylhexyl, 1-ethylpentyl, 1-propylbutyl, 2-ethylpentyl, n-octyl, 1-methylheptyl, 2-methylheptyl, 1-ethylhexyl, 2-ethylhexyl, 1-propylpentyl, 2-propylpentyl, n-nonyl, 1-methyloctyl, 2-methyloctyl, 1-ethylheptyl, 2-ethylheptyl, 1-propylhexyl, 2-propylhexyl, 1-butylpentyl, n-decyl.

Particularly preferred are compounds of formula (I), wherein R^{Ar2} is selected from phenyl which carries one or two C₁-C₆-alkyl substituent(s). Even more preferred are compounds of formula (I), wherein R^{Ar} is phenyl which carries two C₁-C₆-alkyl substituents. The phenyl radical is preferably substituted by only two substituents R^{Ar2} that are in the ortho- and ortho'-position (2- and 6-position) or in the ortho and para-position (2- and 4-position). Especially more preferred are compounds (I), where R^{Ar2} is 2,6-(di-C₁-C₄-alkyl)phenyl or 2,4-(di-C₁-C₄-alkyl)phenyl, even more especially 2,6-(di-C₁-C₄-alkyl)phenyl.

In the compounds of the formula (I), the R^{Ar1} and R^{Ar2} radicals may have identical or different definitions. In a preferred embodiment, the R^{Ar1} and R^{Ar2} radicals have identical definitions.

Compounds of formula (I) are preferred, wherein
Ar1 is selected from phenyl which carries 1 or 2 C₁-C₆-alkyl substituents R^{Ar1} and phenyl which carries one group A; and
Ar2 is selected from phenyl which carries 1 or 2 C₁-C₆-alkyl substituents R^{Ar2} and phenyl which carries one group A.

Preferred are compounds of formula (I), where z is 1, i.e. one group A is attached to Ar3, where A is as defined above. Reference is made to the afore-mentioned preferred meanings of A. Ar3 is preferably phenyl or naphthyl, in particular phenyl. Especially more preferred are compounds of formula (I), wherein Ar3 is phenyl which carries a group A in the para-position. According to this embodiment, Ar3 preferably does not bear a substituent R^{Ar3}.

Preferred are also compounds of formula (I), where z is 0, i.e. Ar3 does not bear a group A.

According to a further embodiment, Ar3 bears 1, 2 or 3 identical or different substituents R^{Ar3} and (A)_{z} is absent, i.e z is 0.

Preferably, the radicals R³, R⁴, R⁵ and R⁶, independently of each other, are selected from hydrogen, phenoxy, which is unsubstituted and phenoxy which carries one group A.

Even more preferably, two of the radicals R³, R⁴, R⁵ and R⁶ are hydrogen and the other radicals R³, R⁴, R⁵ and R⁶ are selected from phenoxy, which is unsubstituted and phenoxy which carries one group A. In this context, the compounds of formula (I) are in particular disubstituted in the 1,7- or 1,6-position of the perylene skeleton.

Especially, R³ and R⁵ are each hydrogen and R⁴ and R⁶ are selected from phenoxy which neither bears a radical R^{Ar3} nor a group A (i.e. phenoxy is unsubstituted) and phenoxy which carries one group A. More especially, R³ and R⁵ are each hydrogen and R⁴ and R⁶ have the same meaning and are phenoxy which is unsubstituted or phenoxy which is substituted with one group A in the para-position.

Likewise, even more preferably, the radicals R³, R⁴, R⁵ and R⁶ have the same meaning. Especially, R³, R⁴, R⁵ and R⁶ are all hydrogen. Likewise, more especially, R³, R⁴, R⁵ and R⁶ are all phenoxy. Likewise, more especially, R³, R⁴, R⁵ and R⁶ are all phenoxy which is substituted with one group A in the para-position.

Some particularly preferred compounds (I) are the compounds (C1), (C2), (C3), (C4), (C5), (C6) and (C7) specified below:

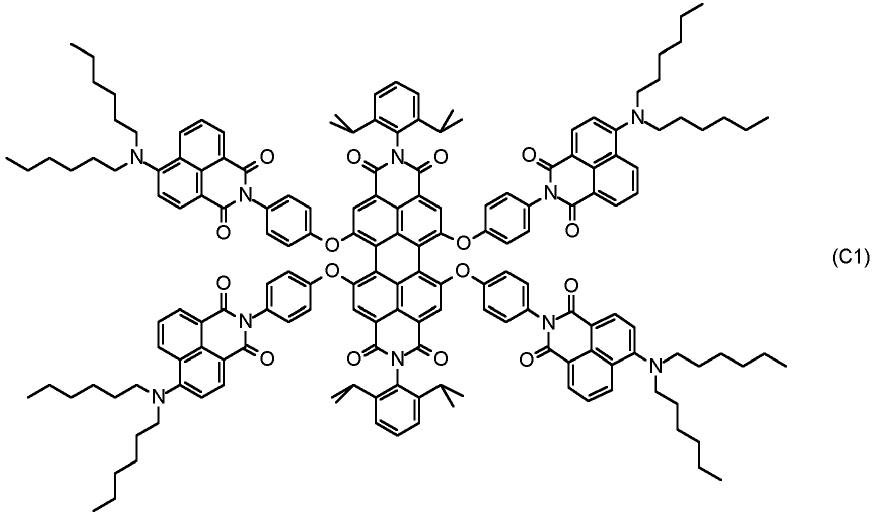

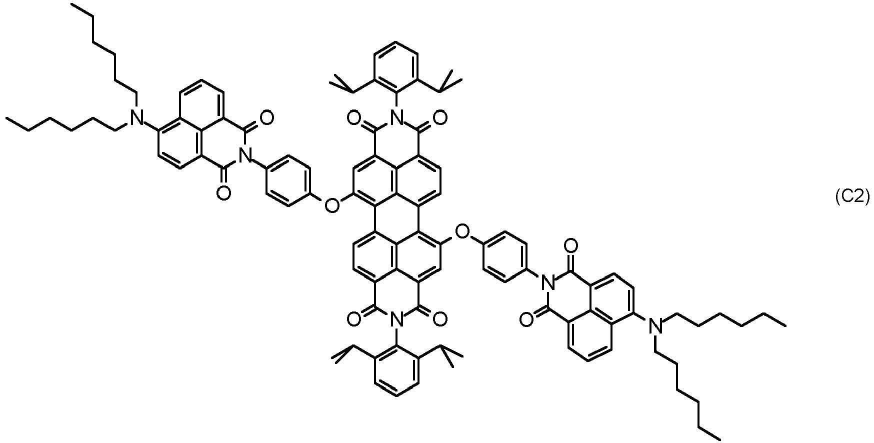

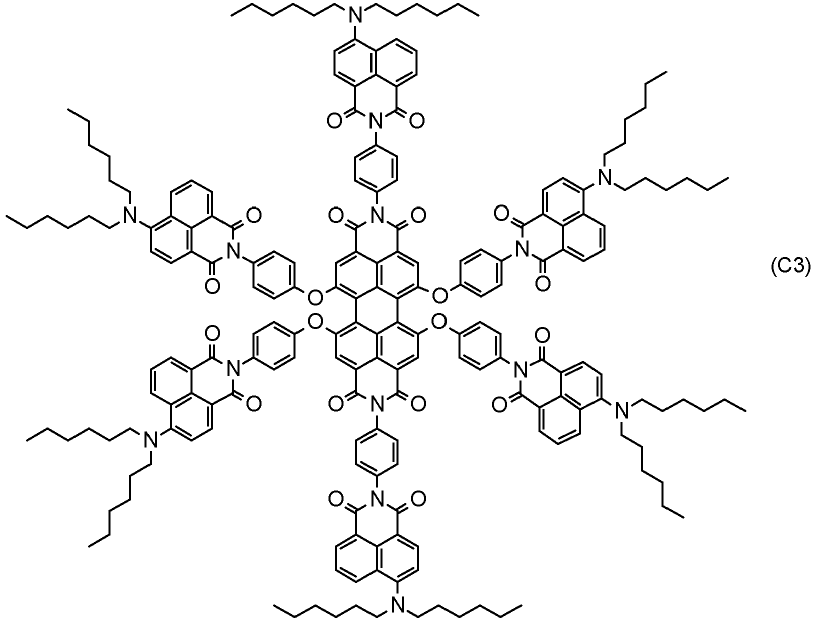

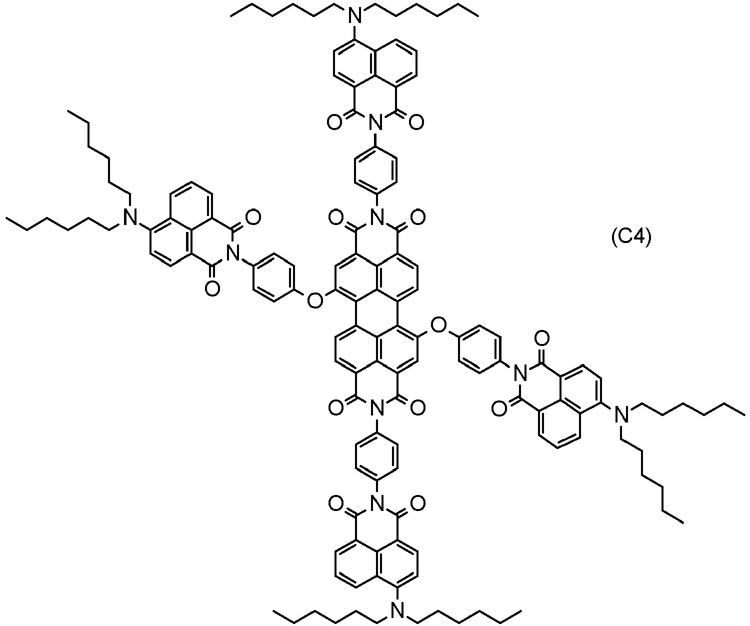

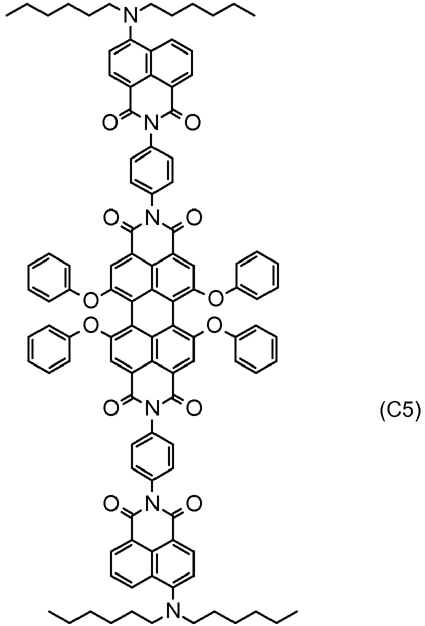

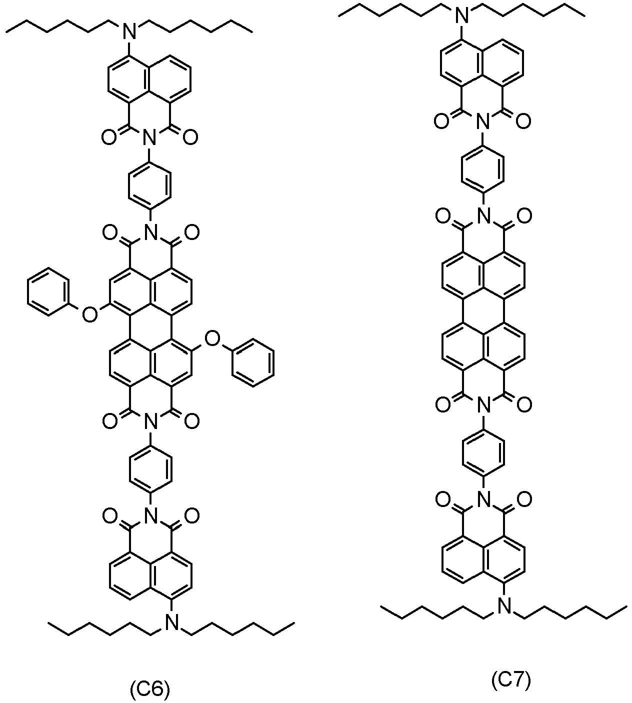

The compounds of formula (I) are especially suitable as color downconverter dyes for color converters as they absorb photons generated by a blue LED or by a white LED and then emits light photons in the wavelength range from 520 to 700 nm with very high fluorescence quantum yield. The fluorescence quantum yield is usually greater than 85%, in particular greater than 90%. Without wishing to be bound to any theory, it is believed that a donor group, namely group A, is excited and transfers the energy to an acceptor group, namely the perylene skeleton, through a non-radiative process (Förster Resonance Energy Transfer), and which in turn relaxes to the ground state via fluorescence, thus providing high overall energy conversion efficiency.

In addition, the compounds of formula (I) are very stable, especially under the practical application conditions in a color converter. Moreover, the compound of formula (I) has high extinction coefficient.

Compounds of formula (I) can be prepared on the industrial scale via few synthetic stages.

The group(s) A which characterize(s) the inventive compound of formula (I) may be introduced by reacting the appropriate halogenated perylene diimide of the formula (2) with a compound of the formula (3) in the sense of a halogen exchange to give a compound of formula (I), where at least one of the radicals R³, R⁴, R⁵ or R⁶ carries a group (A)_{z}-Ar3-X-, and the other radicals R³, R⁴, R⁵ or R⁶ are hydrogen.

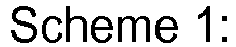

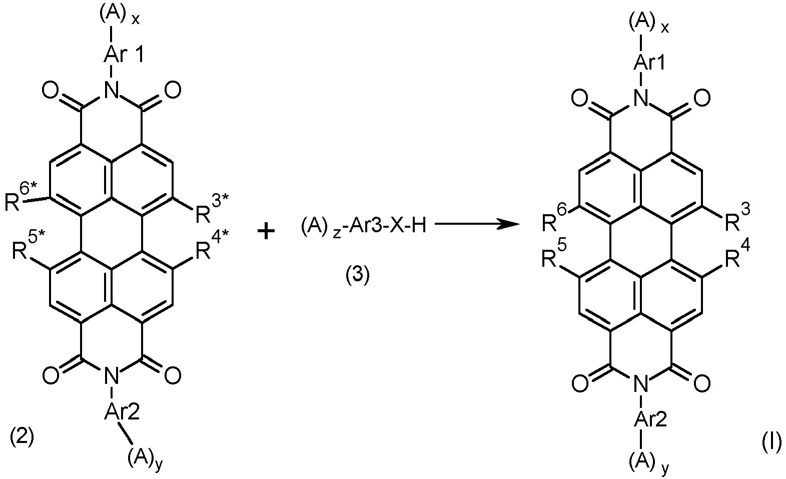

In Scheme 1, (A)ₓ, (A)_{y}, (A)_{z}, Ar1, Ar2, Ar3 and X are as defined above, R^{3*}, R^{4*}, R^{5*} and R^{6*} are hydrogen or halogen (preferably chlorine or bromine).

This reaction is usually carried out the presence of a base. Suitable bases are organic bases and inorganic bases. Preferred bases are in particular inorganic alkali metal or alkaline earth metal bases, the alkali metal bases being particularly suitable. Examples of inorganic bases are the carbonates and hydrogencarbonates, hydroxides, hydrides and amides of alkali metals and alkaline earth metals. Preferred bases are the carbonates and hydrogencarbonates, particular preference being given to the carbonates. Preferred alkali metals are lithium, sodium, potassium and cesium; particularly suitable alkaline earth metals are magnesium and calcium. It will be appreciated that it is also possible to use base mixtures. Very particularly preferred bases are lithium carbonate, sodium carbonate, potassium carbonate and cesium carbonate.

This reaction is usually carried out the presence of a polar aprotic solvent. Suitable solvents are aliphatic carboxamides, preferably N,N-di-C₁-C₄-alkyl-C₁-C₄-carboxamides) and lactams such as dimethylformamide, diethylformamide, dimethylacetamide, dimethylbutyramide and N-methyl-2-pyrrolidone (NMP).

The reaction temperature is usually within the range of 0 °C to the boiling point of the solvent, preferably 20 to 200°C.

Compounds of the formula (2), wherein (A)ₓ and (A)_{y} are absent, i.e. x and y are each 0, and Ar1 and Ar2 aryl which carries 1, 2 or 3 identical or differents substituents selected from C₁-C₃₀-alkyl, C₁-C₃₀-haloalkyl, C₁-C₃₀-alkoxy, C₁-C₃₀-alkylsulfanyl, C₂-C₃₀-alkenyl, C₂-C₃₀-alkynyl are known from WO 2007/006717, DE 19547210 and DE 19547209 or can be prepared in analogy to the methods described therein.

Compounds of formula (3), where z is 1, in the following referred to as compounds of formula (3a) can be prepared as shown in Scheme 2:

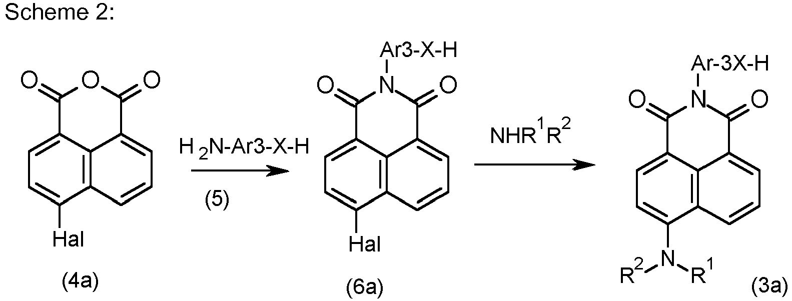

In Scheme 2, Hal is chlorine or bromine, and R¹, R², Ar3, and X are as defined above.

First, naphthalic anhydride of formula (4a) is treated with an amine of formula (5) to obtain a compound of formula (6a). Suitable reaction conditions are known from WO2007/074137. Then the compound of formula (6a) is treated with an amine of the formula NHR¹R² in the sense of a nucleophic substitution reaction to yield the compound (3a).

Compounds of formula (I), where (A)ₓ and (A)_{y} are present, i.e. x and y are each 1, can be prepared in analogy to known method, e.g. those described in WO 2007/006717 starting from an unsubstituted or an appropriate substituted perylene,3,4; 9,10-tetracarboxylic dianhydride and an amine H₂N-Ar1-A, which has the following formula (7), 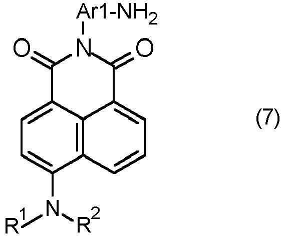 wherein Ar1, R¹ and R² are as defined above,
and, if in formula (I), Ar1-(A) is different from Ar2-(A), with a amine H₂N-Ar2-A, which has the following formula (8) 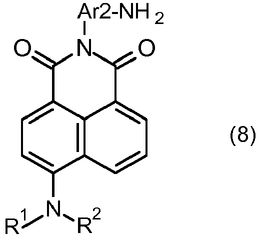 where Ar2, R¹ and R² are as defined above.

Compounds of the formula (7), and likewise compounds of the formula (8), can be prepared as described in Scheme 2 but using 1,4-phenylenediamine instead of the amine of formula (5).

The inventive compound of formula (I) may be incorporated without any problem into organic and inorganic materials and is therefore suitable for a whole series of end uses, some of which will be listed by way of example below. It may be used generally for coloring coatings, printing inks and plastics, producing aqueous polymer dispersions which absorb and/or emit electromagnetic radiation, for data storage, for optical labels, for security labels in documents and for brand protection or as a fluorescent label for biomolecules or as a fluorescent label for biomolecules. The perylene compound (I) is notable for its fluorescence which lies within the visible region of the electromagnetic spectrum. The inventive perylene compound (I) may be used to produce aqueous polymer dispersions which absorb and/or emit electromagnetic radiation. Fluorescent polymer dispersions can be obtained with perylene compound (I). The perylene compound (I) is (are) especially suitable for data storage, for optical labels, for security labels in documents and for brand protection or as a fluorescent label for biomolecules.

The inventive perylene compound (I) is notable for use in color converters for converting light emitted from a light source, in particular a light source selected from LEDs and OLEDs, into light of a second longer wavelength. The compounds of formula (I) are red-fluorescing fluorescent dyes.

Accordingly, the present invention further provides a color converter comprising at least one polymer as a matrix material and at least one compound of formula (I). Amongst the compound of formula (I), special preference is given to the compounds (C1), (C2), (C3), (C4), (C5), (C6) and (C7).

In a special embodiment, the color converter comprises only one compound of formula (I).

Suitable polymers are in principle all polymers capable of dissolving or homogeneously dispersing the at least one compound of formula (I) in a sufficient amount.

Suitable polymers may be inorganic polymers or organic polymers.

In a preferred embodiment, the at least one polymer is selected from polystyrene, polycarbonate, polymethylmethacrylate, polyvinylpyrrolidone, polymethacrylate, polyvinyl acetate, polyvinyl chloride, polybutene, silicone, polyacrylate, epoxy resin, polyvinyl alcohol, poly(ethylene vinylalcohol)-coplymer (EVA, EVOH), polyacrylonitrile, polyvinylidene chloride (PVDC), polystyreneacrylonitrile (SAN), polybutylene terephthalate (PBT), polyethylene terephthalate (PET), polyvinyl butyrate (PVB), polyvinyl chloride (PVC), polyamides, polyoxymethylenes, polyimides, polyetherimides and mixtures thereof.

More preferably, the at least one polymer consist essentially of polystyrene, polycarbonate, polymethylmethacrylate, polyvinylpyrrolidone, polymethacrylate, polyvinyl acetate, polyvinyl chloride, polybutene, silicone, polyacrylate, epoxy resin, polyvinyl alcohol, poly(ethylene vinylalcohol)-coplymer (EVA, EVOH), polyacrylonitrile, polyvinylidene chloride (PVDC), polystyreneacrylonitrile (SAN), polybutylene terephthalate (PBT), polyethylene terephthalate (PET), polyvinyl butyrate (PVB), polyvinyl chloride (PVC), polyamides, polyoxymethylenes, polyimides, polyetherimides or mixtures thereof.

In particular, the at least one polymer consists essentially of polystyrene (PS), polycarbonate (PC), polymethylmethacrylate (PMMA), polyethylene terephthalate (PET) or mixtures thereof.

Most preferably, the at least one polymer consists essentially of polystyrene, polyethylene terephthalate or polycarbonate, especially of polycarbonate or polyethylene terephthalate.

Polystyrene is understood here to mean, inter alia, all homo- or copolymers which result from polymerization of styrene and/or derivatives of styrene. Derivatives of styrene are, for example, alkylstyrenes such as alpha-methylstyrene, ortho-, meta-, para-methylstyrene, para-butylstyrene, especially para-tert-butylstyrene, alkoxystyrene such as para-methoxystyrene, para-butoxystyrene, para-tert-butoxystyrene.

In general, suitable polystyrenes have a mean molar mass Mₙ of 10 000 to 1 000 000 g/mol (determined by GPC), preferably 20 000 to 750 000 g/mol, more preferably 30 000 to 500 000 g/mol.

In a preferred embodiment, the matrix of the color converter consists essentially or completely of a homopolymer of styrene or styrene derivatives.

In further preferred embodiments of the invention, the matrix consists essentially or completely of a styrene copolymer, which are likewise regarded as polystyrene in the context of this application. Styrene copolymers may comprise, as further constituents, for example, butadiene, acrylonitrile, maleic anhydride, vinylcarbazole or esters of acrylic, methacrylic or itaconic acid as monomers. Suitable styrene copolymers generally comprise at least 20% by weight of styrene, preferably at least 40% and more preferably at least 60% by weight of styrene. In another embodiment, they comprise at least 90% by weight of styrene.

Preferred styrene copolymers are styrene-acrylonitrile copolymers (SAN) and acrylonitrile-butadiene-styrene copolymers (ABS), styrene-1,1'-diphenylethene copolymers, acrylic ester-styrene-acrylonitrile copolymers (ASA), methyl methacrylate-acrylonitrile-butadiene-styrene copolymers (MABS).

A further preferred polymer is alpha-methylstyrene-acrylonitrile copolymer (AMSAN).

The styrene homo- or copolymers can be prepared, for example, by free-radical polymerization, cationic polymerization, anionic polymerization or under the influence of organometallic catalysts (for example Ziegler-Natta catalysis). This can lead to isotactic, syndiotactic or atactic polystyrene or copolymers. They are preferably prepared by free-radical polymerization. The polymerization can be performed as a suspension polymerization, emulsion polymerization, solution polymerization or bulk polymerization.

The preparation of suitable polystyrenes is described, for example, in Oscar Nuyken, Polystyrenes and Other Aromatic Polyvinyl Compounds, in Kricheldorf, Nuyken, Swift, New York 2005, p. 73-150 and references cited therein; and in Elias, Macromolecules, Weinheim 2007, p. 269-275.

Polyethylene terephthalate is obtainable by condensation of ethylene glycol with terephthalic acid.

Polycarbonates are polyesters of carbonic acid with aromatic or aliphatic dihydroxyl compounds. Preferred dihydroxyl compounds are, for example, methylenediphenylenedihydroxyl compounds, for example bisphenol A.

One means of preparing polycarbonates is the reaction of suitable dihydroxyl compounds with phosgene in an interfacial polymerization. Another means is the reaction with diesters of carbonic acid such as diphenyl carbonate in a condensation polymerization.

The preparation of suitable polycarbonates is described, for example, in Elias, Macromolecules, Weinheim 2007, p. 343-347.

In a preferred embodiment, polymers which have been polymerized with exclusion of oxygen are used. Preferably, the monomers during the polymerization comprised a total of not more than 1000 ppm of oxygen, more preferably not more than 100 ppm and especially preferably not more than 10 ppm.

Suitable polymers may comprise, as further constituents, additives such as flame retardants, antioxidants, light stabilizers, UV absorbers, free-radical scavengers, antistats. Stabilizers of this kind are known to those skilled in the art.

Suitable antioxidants or free-radical scavengers are, for example, phenols, especially sterically hindered phenols such as butylhydroxyanisole (BHA) or butylhydroxytoluene (BHT), or sterically hindered amines (HALS). Stabilizers of this kind are sold, for example, by BASF under the Irganox® trade name. In some cases, antioxidants and free-radical scavengers can be supplemented by secondary stabilizers such as phosphites or phosphonites, as sold, for example, by BASF under the Irgafos® trade name.

Suitable UV absorbers are, for example, benzotriazoles such as 2-(2-hydroxyphenyl)-2H-benzotriazole (BTZ), triazines such as (2-hydroxyphenyl)-s-triazine (HPT), hydroxybenzophenones (BP) or oxalanilides. UV absorbers of this kind are sold, for example, by BASF under the Uvinul® trade name.

In a preferred embodiment, TiO₂ is used as the sole UV absorber.

In a preferred embodiment of the invention, suitable polymers do not comprise any antioxidants or free-radical scavengers.

In a further embodiment of the invention, suitable polymers are transparent polymers.

In another embodiment, suitable polymers are opaque polymers.

The polymers mentioned above serve as matrix material for the organic fluorescent dye of formula (I) according to the invention and mixtures thereof.

The inventive fluorescent dye, i.e. the compound of the formula (I) according to the invention, may either be dissolved in the polymer or may be in the form of a homogeneously distributed mixture. The fluorescent dye is preferably dissolved in the polymer.

In a preferred embodiment, the color converter according to the invention comprises no further fluorescent material. Such a color converter according to the invention together with blue light of a LED is able to provide red light with high energy efficiency.

In a further preferred embodiment, color converters comprise, in addition to the at least one red-fluorescent dye according to the invention, a further fluorescent material selected from inorganic fluorescent material and further organic fluorescent dyes.

Suitable further organic fluorescent dyes may be any organic fluorescent dye that absorbs light in the wavelength range from 400 to 500 nm and emits light having a longer wavelength than that of the absorbed light, especially in the wavelength range from 450 to 600 nm.

Different organic fluorescent dyes may be combined to optimize energy efficiency and - in particular - photometric efficacy. Suitable further organic fluorescent dyes are green-fluorescent dyes, yellow-green fluorescent dyes, yellow-fluorescent dyes, and orange-fluorescent dyes. Preferably, fluorescent dyes are combined with one another such that color converters with high energy and photometric efficacy are obtained.

In a preferred embodiment, color converters comprise, as well as the at least one fluorescent dye of the invention or a mixture thereof, further organic fluorescent dyes.

In particular, further organic fluorescent dyes are selected from
(i) a cyanated naphthalene benzimidazole compound of the formula II 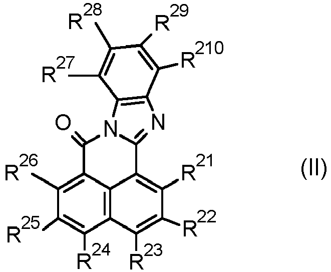 wherein R²
   ¹, R²², R²³, R²⁴, R²⁵, R²⁶, R²⁷, R²⁸, R²⁹ and R²¹⁰ are each independently hydrogen, cyano or aryl which is unsubstituted or has one or more identical or different substituents R^{2Ar},
   where
   each R^{2Ar} is independently selected from cyano, hydroxyl, mercapto, halogen, C₁-C₂₀-alkoxy, C₁-C₂₀-alkylthio, nitro, -NR^{2Ar2}R^{2Ar3}, -NR^{2Ar2}COR^{2Ar3}, -CONR^{2Ar2}R^{2Ar3} -SO₂NR^{2Ar2}R^{2Ar3} -COOR^{2Ar2}, -SO₃R^{2Ar2},
   C₁-C₃₀-alkyl, C₂-C₃₀-alkenyl, C₂-C₃₀-alkynyl, where the three latter radicals are unsubstituted or bear one or more R^{2a} groups,
   C₃-C₈-cycloalkyl, 3- to 8-membered heterocyclyl, where the two latter radicals are unsubstituted or bear one or more R^{2b} groups,
   aryl, U-aryl, heteroaryl and U-heteroaryl, where the four latter radicals are unsubstituted or bear one or more R^{2b} groups,
   where
   each R^{2a} is independently selected from cyano, hydroxyl, oxo, mercapto, halogen, C₁-C₂₀-alkoxy, C₁-C₂₀-alkylthio, nitro, -NR^{2Ar2}R^{2Ar3}, -NR^{2Ar2}COR^{2Ar3}, -CONR^{2Ar2}R^{2Ar3}, -SO₂NR^{2Ar2}R^{2Ar3}, -COOR^{2Ar2}, -SO₃R^{2Ar2}, C₃-C₈-cycloalkyl, 3- to 8-membered heterocyclyl, aryl and heteroaryl, where the cycloalkyl, heterocyclyl, aryl and heteroaryl radicals are unsubstituted or bear one or more R^{2b} groups;
   each R^{2b} is independently selected from cyano, hydroxyl, oxo, mercapto, halogen, C₁-C₂₀-alkoxy, C₁-C₂₀-alkylthio, nitro, -NR^{2Ar2}R^{2Ar3}, -NR^{2Ar2}COR^{2Ar3}, -CONR^{2Ar2}R^{2Ar3}, -SO₂NR^{2Ar2}R^{2Ar3}, -COOR^{2Ar2}, -SO₃R^{2Ar2}, C₁-C₁₈-alkyl, C₂-C₁₈-alkenyl, C₂-C₁₈-alkynyl, C₃-C₈-cycloalkyl, 3- to 8-membered heterocyclyl, aryl and heteroaryl, where the four latter radicals are unsubstituted or bear one or more R^{2b1} groups,
   each R^{2b1} is independently selected from cyano, hydroxyl, mercapto, oxo, nitro, halogen, -NR^{2Ar2}R^{2Ar3}, -NR^{2Ar2}COR^{Ar3}, -CONR^{2Ar2}R^{2Ar3}, -SO₂NR^{2Ar2}R^{2Ar3}, -COOR^{2Ar2}, -SO₃R^{2Ar2}, -SO₃R^{2Ar2}, C₁-C₁₈-alkyl, C₂-C₁₈-alkenyl, C₂-C₁₈-alkynyl, C₁-C₁₂-alkoxy, C₁-C₁₂-alkylthio,
   U is an -O-, -S-, -NR^{2Ar1}-, -CO-, -SO- or -SO₂- moiety;
   R^{2Ar1}, R^{2Ar2}, R^{2Ar3} are each independently hydrogen, C₁-C₁₈-alkyl, 3- to 8-membered cycloalkyl, 3- to 8-membered heterocyclyl, aryl or heteroaryl, where alkyl is unsubstituted or bears one or more R^{2a} groups, where 3- to 8-membered cycloalkyl, 3- to 8-membered heterocyclyl, aryl and heteroaryl are unsubstituted or bear one or more R^{2b} groups;
   with the proviso that the compound of the formula II comprises at least one cyano group
   or mixtures thereof;
(ii) a cyanated perylene compound of the formula (III) 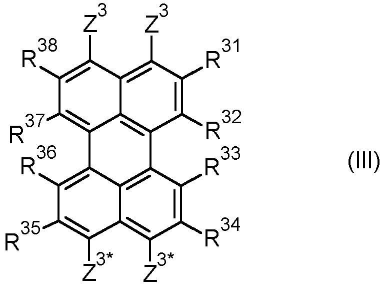 in which
   one of the Z³ substituents is cyano and the other Z³ substituent is CO₂R³⁹, CONR³¹⁰R³¹¹, C₁-C₁₈-alkyl, C₂-C₁₈-alkenyl, C₂-C₁₈-alkynyl, C₃-C₁₂-cycloalkyl or C₆-C₁₄-aryl, where
      C₁-C₁₈-alkyl, C₂-C₁₈-alkenyl, C₂-C₁₈-alkynyl are unsubstituted or bear one or more identical or different Z^{3a} substituents,
      C₃-C₁₂-cycloalkyl is unsubstituted or bears one or more identical or different Z^{3b} substituents, and
      C₆-C₁₄-aryl is unsubstituted or bears one or more identical or different Z^{3Ar} substituents;
   one of the Z^{3*} substituents is cyano and the other Z^{3*} substituent is CO₂R³⁹, CONR³¹⁰R³¹¹, C₁-C₁₈-alkyl, C₂-C₁₈-alkenyl, C₂-C₁₈-alkynyl, C₃-C₁₂-cycloalkyl or C₆-C₁₄-aryl, where
      C₁-C₁₈-alkyl, C₂-C₁₈-alkenyl, C₂-C₁₈-alkynyl are unsubstituted or bear one or more identical or different Z^{3a} substituents,
      C₃-C₁₂-cycloalkyl is unsubstituted or bears one or more identical or different Z^{3b} substituents, and
      C₆-C₁₄-aryl is unsubstituted or bears one or more identical or different Z^{3Ar} substituents;
   R³¹ R³², R³³, R³⁴ R³⁵ R³⁶, R³⁷ and R³⁸ are each independently selected from hydrogen, cyano, bromine and chlorine,
      with the proviso that 1, 2, 3, 4, 5, 6, 7 or 8 of the R³¹, R³², R³³, R³⁴, R³⁵, R³⁶, R³⁷ or R³⁸ substituents are cyano;
   where
   R³⁹ is hydrogen, C₁-C₁₀-alkyl, C₂-C₁₀-alkenyl, C₂-C₁₀-alkynyl, C₃-C₁₂-cycloalkyl or C₆-C₁₄-aryl, where
      C₁-C₁₀-alkyl, C₂-C₁₀-alkenyl, C₂-C₁₀-alkynyl are unsubstituted or bear one or more identical or different R^{3a} substituents,
      C₃-C₁₂-cycloalkyl is unsubstituted or bears one or more identical or different R^{3b} substituents and
      C₆-C₁₄-aryl is unsubstituted or bears one or more identical or different R^{3Ar} substituents;
   R³¹⁰ and R³¹¹ are each independently hydrogen, C₁-C₁₀-alkyl, C₂-C₁₀-alkenyl, C₂-C₁₀-alkynyl, C₃-C₁₂-cycloalkyl or C₆-C₁₄-aryl, where
      C₁-C₁₀-alkyl, C₂-C₁₀-alkenyl, C₂-C₁₀-alkynyl are unsubstituted or bear one or more identical or different R^{3a} substituents,
      C₃-C₁₂-cycloalkyl is unsubstituted or bears one or more identical or different R^{3b} substituents and
      C₆-C₁₄-aryl is unsubstituted or bears one or more identical or different R^{3Ar} substituents;
   each Z^{3a} is independently halogen, hydroxyl, NR^{310a}R^{311a}, C₁-C₁₀-alkoxy, C₁-C₁₀-haloalkoxy, C₁-C₁₀-alkylthio, C₃-C₁₂-cycloalkyl, C₆-C₁₄-aryl, C(=O)R^{39a}; C(=O)OR^{39a} or C(O)NR^{310a}R^{311a}, where
      C₃-C₁₂-cycloalkyl is unsubstituted or bears one or more identical or different R^{3b} substituents and
      C₆-C₁₄-aryl is unsubstituted or bears one or more identical or different R^{3Ar} substituents;
   each Z^{3b} and each Z^{3Ar} is independently halogen, hydroxyl, NR^{310a}R^{311a}, C₁-C₁₀-alkyl, C₁-C₁₀-alkoxy, C₁-C₁₀-haloalkoxy, C₁-C₁₀-alkylthio, C(=O)R^{39a}; C(=O)OR^{39a} or C(O)NR^{310a}R^{311a};
   each R^{3a} is independently halogen, hydroxyl, C₁-C₁₀-alkoxy, C₃-C₁₂-cycloalkyl or C₆-C₁₄-aryl;
   each R^{3b} is independently halogen, hydroxyl, C₁-C₁₀-alkyl, C₁-C₁₀-alkoxy, C₁-C₁₀-haloalkoxy, C₁-C₁₀-alkylthio, C₂-C₁₀-alkenyl, C₂-C₁₀-alkynyl, C₃-C₁₂-cycloalkyl or C₆-C₁₄-aryl;
   each R^{3Ar} is independently halogen, hydroxyl, C₁-C₁₀-alkyl, C₁-C₁₀-alkoxy, C₁-C₁₀-haloalkoxy, C₁-C₁₀-alkylthio, C₂-C₁₀-alkenyl, C₂-C₁₀-alkynyl, C₃-C₁₂-cycloalkyl or C₆-C₁₄-aryl;
   R^{39a} is hydrogen, C₁-C₁₀-alkyl, C₂-C₁₀-alkenyl, C₂-C₁₀-alkynyl, C₃-C₁₂-cycloalkyl or C₆-C₁₄-aryl; and
   R^{310a}, R^{311a} are each independently hydrogen, C₁-C₁₀-alkyl, C₂-C₁₀-alkenyl, C₂-C₁₀-alkynyl, C₃-C₁₂-cycloalkyl or C₆-C₁₄-aryl,
   and mixtures thereof.
(iii) a cyanated compound of the formula (IV) 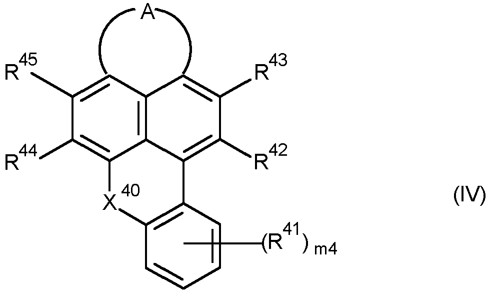 wherein
   - m4: is 0, 1, 2, 3 or 4;
   - each R⁴¹: independently from each other is selected from bromine, chlorine, cyano, -NR^{4a}R^{4b}, C₁-C₂₄-alkyl, C₁-C₂₄-haloalkyl, C₁-C₂₄-alkoxy, C₁-C₂₄-haloalkoxy, C₃-C₂₄-cycloalkyl, heterocycloalkyl, heteroaryl, C₆-C₂₄-aryl, C₆-C₂₄-aryloxy, C₆-C₂₄-aryl-C₁-C₁₀-alkylene, where the rings of cycloalkyl, heterocycloalkyl, heteroaryl, aryl, aryloxy in the six last-mentioned radicals are unsubstituted or substituted with 1, 2, 3, 4 or 5 identical or different radicals R^{41a} and where C₁-C₂₄-alkyl, C₁-C₂₄-haloalkyl, C₁-C₂₄-alkoxy, and the alkylene moiety of C₆-C₂₄-aryl-C₁-C₁₀-alkylene may be interrupted by one or more groups selected from O, S and NR^{4c};
   at least one of the radicals R⁴², R⁴³, R⁴⁴ and R⁴⁵ is CN, and the remaining radicals, independently from each other, are selected from hydrogen, chlorine and bromine;
   - X⁴⁰: is O, S, SO or SO₂;
   - A: is a diradical selected from diradicals of the general formulae (A.1), (A.2), (A.3), and (A.4) 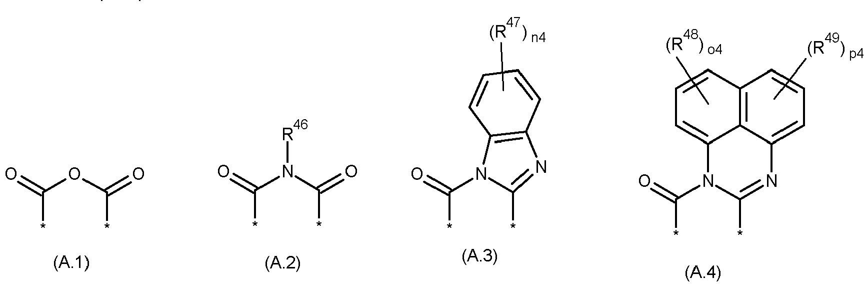
   wherein
   - *: in each case denotes the point of attachments to the remainder of the molecule;
   - n4: is 0, 1, 2, 3, 4 or 5;
   - o4: is 0, 1, 2, 3 or 4;
   - p4: is 0, 1, 2, 3 or 4;
   - R⁴⁶: is hydrogen, C₁-C₂₄-alkyl, C₁-C₂₄-haloalkyl, C₃-C₂₄-cycloalkyl, C₆-C₂₄-aryl or C₆-C₂₄-aryl-C₁-C₁₀-alkylene, where the rings of cycloalkyl, aryl, and aryl-alkylene in the three last-mentioned radicals are unsubstituted or substituted with 1, 2, 3, 4 or 5 identical or different radicals R^{46a}, and where C₁-C₂₄-alkyl, C₁-C₂₄-haloalkyl and the alkylene moiety of C₆-C₂₄-aryl-C₁-C₁₀-alkylene may be interrupted by one or more heteroatoms or heteroatomic groups selected from O, S and NR^{4c};
   - each R⁴⁷: independently from each other is selected from bromine, chlorine, cyano, -NR^{4a}R^{4b}, C₁-C₂₄-alkyl, C₁-C₂₄-haloalkyl, C₁-C₂₄-alkoxy, C₁-C₂₄-haloalkoxy, C₃-C₂₄-cycloalkyl, heterocycloalkyl, heteroaryl, C₆-C₂₄-aryl, C₆-C₂₄-aryloxy, C₆-C₂₄-aryl-C₁-C₁₀-alkylene, where the rings of cycloalkyl, heterocycloalkyl, heteroaryl, aryl and aryl-alkylene in the six last-mentioned radicals are unsubstituted or substituted with 1, 2, 3, 4 or 5 identical or different radicals R^{47a} and where C₁-C₂₄-alkyl, C₁-C₂₄-haloalkyl, C₁-C₂₄-alkoxy, C₁-C₂₄-haloalkoxy, and the alkylene moiety of C₆-C₂₄-aryl-C₁-C₁₀-alkylene may be interrupted by one or more groups selected from O, S and NR^{4c};
   - each R⁴⁸: independently from each other is selected from bromine, chlorine, cyano, NR^{4a}R^{4b}, C₁-C₂₄-alkyl, C₁-C₂₄-haloalkyl, C₁-C₂₄-alkoxy, C₁-C₂₄-haloalkoxy, C₃-C₂₄-cycloalkyl, heterocycloalkyl, heteroaryl, C₆-C₂₄-aryl, C₆-C₂₄-aryloxy, C₆-C₂₄-aryl-C₁-C₁₀-alkylene, where the rings of cycloalkyl, heterocycloalkyl, heteroaryl, aryl and aryl-alkylene in the six last-mentioned radicals are unsubstituted or substituted with 1, 2, 3, 4 or 5 identical or different radicals R^{48a} and where C₁-C₂₄-alkyl, C₁-C₂₄-haloalkyl, C₁-C₂₄-alkoxy, C₁-C₂₄-haloalkoxy, and the alkylene moiety of C₆-C₂₄-aryl-C₁-C₁₀-alkylene may be interrupted by one or more groups selected from O, S and NR^{4c};
   - each R⁴⁹: independently from each other is selected from bromine, chlorine, cyano, NR^{4a}R^{4b}, C₁-C₂₄-alkyl, C₁-C₂₄-haloalkyl, C₁-C₂₄-alkoxy, C₁-C₂₄-haloalkoxy, C₃-C₂₄-cycloalkyl, heterocycloalkyl, heteroaryl, C₆-C₂₄-aryl, C₆-C₂₄-aryloxy, C₆-C₂₄-aryl-C₁-C₁₀-alkylene, where the rings of cycloalkyl, heterocycloalkyl, heteroaryl, aryl and aryl- alkylene in the six last-mentioned radicals are unsubstituted or substituted with 1, 2, 3, 4 or 5 identical or different radicals R^{49a} and where C₁-C₂₄-alkyl, C₁-C₂₄-haloalkyl, C₁-C₂₄-alkoxy, C₁-C₂₄-haloalkoxy, and the alkylene moiety of C₆-C₂₄-aryl-C₁-C₁₀-alkylene may be interrupted by one or more groups selected from O, S and NR^{4c};
   - R^{41a} R^{46a}, R^{47a}, R^{48a}, R^{49a}: are independently of one another selected from C₁-C₂₄-alkyl, C₁-C₂₄-fluoroalkyl, C₁-C₂₄-alkoxy, fluorine, chlorine and bromine;
   - R^{4a}, R^{4b}, R^{4c}: are independently of one another are selected from hydrogen, C₁-C₂₀-alkyl, C₃-C₂₄-cycloalkyl, heterocycloalkyl, heteroaryl and C₆-C₂₄-aryl;
   and mixtures thereof;
(iv) a benzoxanthene compound of the formula (V) 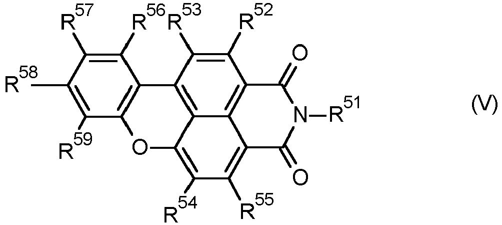 wherein
   - R⁵¹: is phenyl which is unsubstituted or carries 1, 2, 3, 4, or 5 substituents selected from halogen, R⁵¹¹, OR⁵⁵², NHR⁵⁵² and NR⁵⁵²R⁵⁵⁷;
   - R⁵², R⁵³ R⁵⁴ R⁵⁵ R⁵⁶ R⁵⁷ R⁵⁸ and R⁵⁹: are independently of each other selected from hydrogen, halogen, R⁵⁵³, OR⁵⁵³, NHR⁵⁵³ and NR⁵⁵³R⁵⁵⁴,
   wherein
   - R⁵¹¹: is selected from C₂-C₁₈-alkyl, C₆-C₂₄-aryl and heteroaryl;
   - R⁵⁵² and R⁵⁵⁷: are independently of each other selected from C₁-C₁₈-alkyl, C₆-C₂₄-aryl and heteroaryl; and
   - R⁵⁵³ and R⁵⁵⁴: are independently of each other selected from C₁-C₁₈-alkyl, C₆-C₂₄-aryl and heteroaryl;
   and mixtures thereof;
(v) a fluorescent compound comprising at least one structural unit of formula (VI) 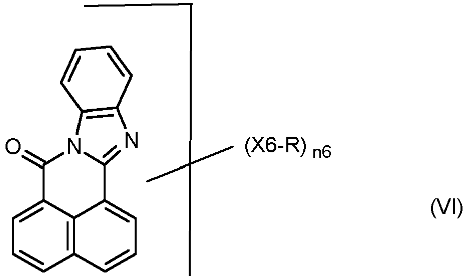 where one or more CH groups of the six-membered ring of the benzimidazole structure shown may be replaced by nitrogen and where the symbols are each defined as follows:
   - n6: is a number from 0 to (10-p6) for each structural unit of the formula (VI); where p6 is the number of CH units which have been replaced by nitrogen in the sixmembered ring of the benzimidazole structure shown
   - X6: is a chemical bond, O, S, SO, SO₂, NR⁶¹; and
   - R: is an aliphatic radical, cycloaliphatic radical, aryl, hetaroaryl, each of which may bear any desired substituents,
   an aromatic or heteroaromatic ring or ring system, each of which is fused to other aromatic rings of the structural unit of the formula (VI) is F, Cl, Br, CN, H when X6 is not a chemical bond;
   where two R radicals may be joined to give one cyclic radical and
   where X6 and R, when n6 > one, may be the same or different;
   - R⁶¹: is each independently hydrogen, C₁-C₁₈-alkyl or cycloalkyl, the carbon chain of which may comprise one or more -O-, -S-, -CO-, -SO- and/or -SO₂- moieties and which may be mono- or polysubstituted; aryl or heteroaryl which may be mono- or polysubstituted;
   and mixtures thereof;

Surprisingly, when the color converter comprises a combination of a compound of formula (I) or a mixture thereof and at least one further organic fluorescent dye selected from a compound of formulae (II), (III), (IV), (V) and (VI) and mixtures thereof, a color converter is provided with high overall conversion efficiency. In addition, the color converter according to the invention together with blue light of a LED is able to provide white-light having any color temperature in the range between 2000 K and 7500 K. In addition, the light of the LED source is converted to longer wavelength with a high overall energy efficiency.

Fluorescent dyes of the formula (II) are known from WO2015/019270. Among the compounds of formula (II), particular preference is given to those of the general formula II-A 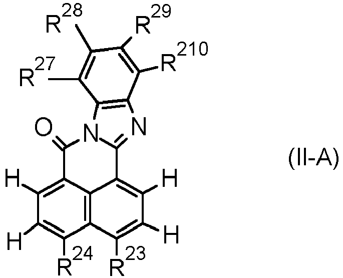 and mixtures thereof
in which
R²³ and R²⁴ are each independently cyano, phenyl, 4-cyanophenyl or phenyl which carries 1, 2 or 3 substituents selected from C₁-C₁₀-alkyl, especially cyano, phenyl or 4-cyanophenyl; and
R²⁷, R²⁸, R²⁹ and R²¹⁰ are each independently hydrogen, cyano, phenyl, 4-cyanophenyl or phenyl which carries 1, 2 or 3 substituents selected from C₁-C₁₀-alkyl, especially hydrogen, cyano, phenyl or 4-cyanophenyl.

Compounds in turn preferred among the compounds of the formula II-A are those
in which
- R23: is cyano;
- R²⁴: is phenyl, 4-cyanophenyl or phenyl which carries 1, 2 or 3 substituents selected from C₁-C₁₀-alkyl; and
two of the radicals R²⁷, R²⁸, R²⁹ and R²¹⁰ are each independently, phenyl, 4-cyanophenyl or phenyl which carries 1, 2 or 3 substituents selected from C₁-C₁₀alkyl and the other radicals R²⁷, R²⁸, R²⁹ and R²¹⁰ are hydrogen.

Likewise, compounds in turn preferred among the compounds of the formula II-A are also those which correspond to the formulae II-Ab and II-Ab' 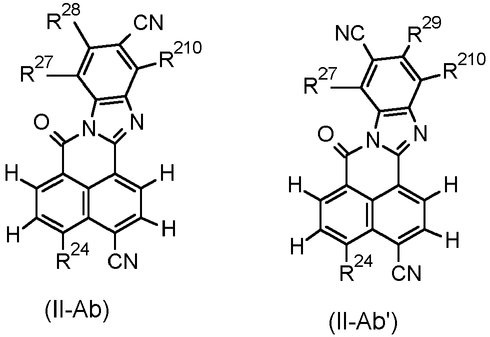 in which
- R²⁴: is phenyl, 4-cyanophenyl or phenyl which carries 1, 2 or 3 substituents selected from C₁-C₁₀-alkyl; and
- zero,: one or two of the radicals R²⁷, R²¹⁰, if present, R²⁸ and R²⁹ are each independently, phenyl, 4-cyanophenyl or phenyl which carries 1, 2 or 3 substituents selected from C₁-C₁₀-alkyl and the other radicals R²⁷, R²¹⁰, R²⁸, R²⁹, if present, are hydrogen.

Compounds in turn preferred among the compounds of the formula II-A are also those which correspond to the formula II-Ac 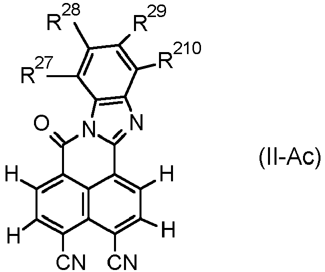 in which
one or two of the radicals R²⁷, R²⁸, R²⁹ and R²¹⁰ are each independently, phenyl, 4-cyanophenyl or phenyl which carries 1, 2 or 3 substituents selected from C₁-C₁₀-alkyl and the other radicals R²⁷, R²⁸, R²⁹ and R²¹⁰ are hydrogen.

Compounds in turn preferred among the compounds of the formula II-A are also those which correspond to the formula II-Ad and II-Ad' 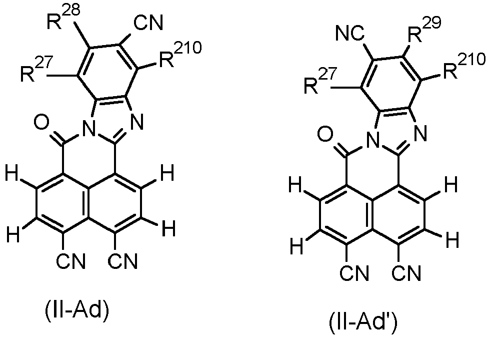 in which
one or two of the radicals R²⁷, R²⁸, R²⁹ and R²¹⁰ are each independently, phenyl, 4-cyanophenyl or phenyl which carries 1, 2 or 3 substituents selected from C₁-C₁₀-alkyl and the other radicals R²⁷, R²⁸, R²⁹ and R²¹⁰ are hydrogen.

Especially preferably, the at least one cyanated naphthalenebenzimidazole compound of the formula I is selected from the compounds of the formulae (II-1), (II-2), (II-3), (II-4), (II-5), (II-6), (II-7), (II-8), (II-9), (II-10), (II-11), (II-12), (II-13), (II-14), (II-15), (II-16), (II-17), (II-18), (II-19), (II-20), (II-21), (II-22), (II-23), (II-24), (II-25), (II-26), (II-27), (II-28), (II-29), (II-30), (II-31), (II-32), (II-33), (II-34), (II-35), (II-36), (II-37), (II-38), (II-39), (II-40), (II-41), (II-42), (II-43), (II-44), (II-45), (II-46), (II-47), (II-48), (II-49), and (II-50) and mixtures thereof

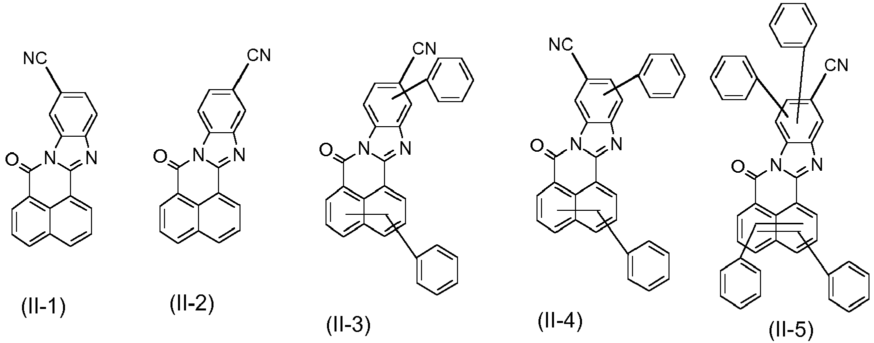

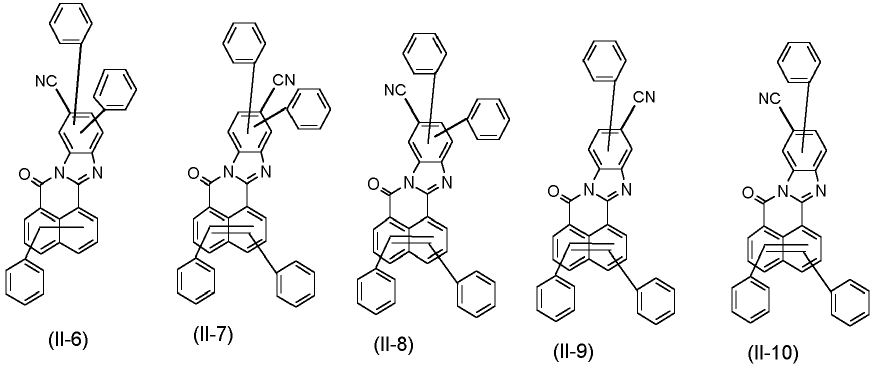

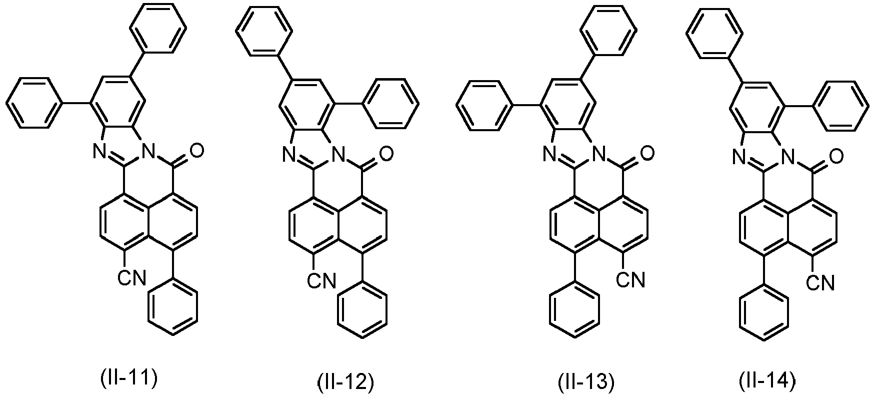

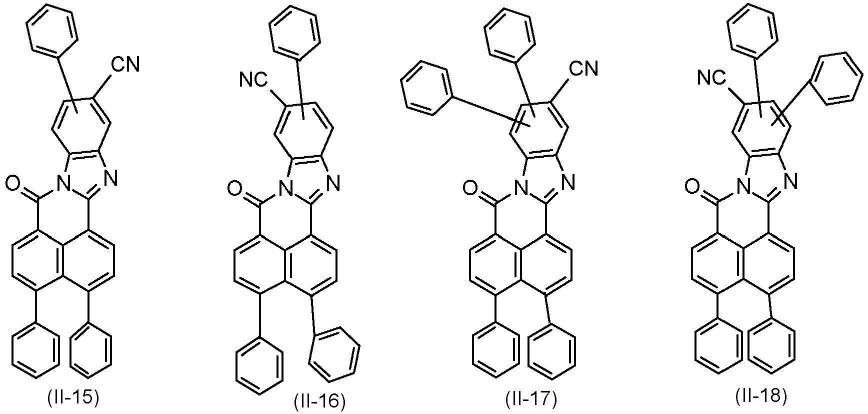

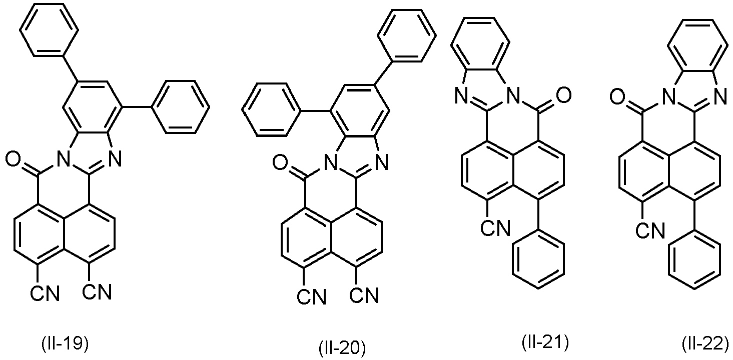

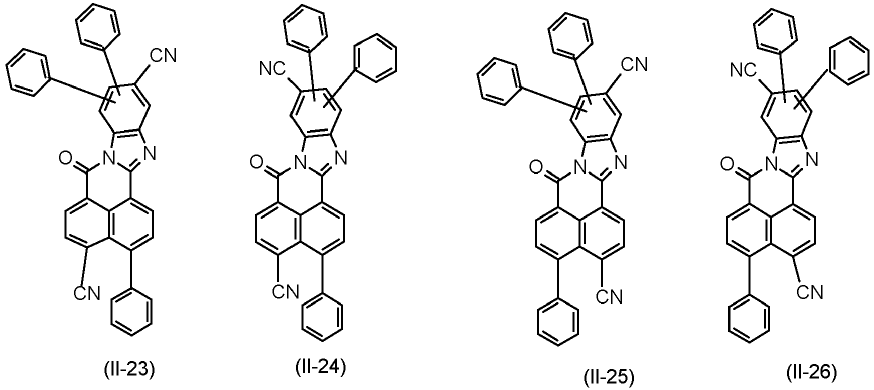

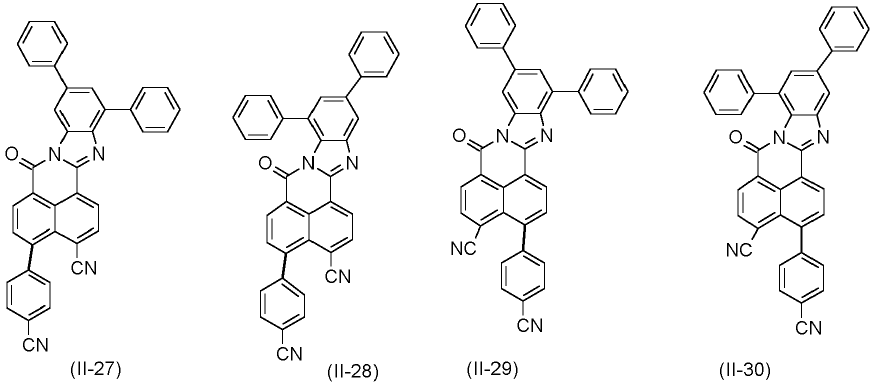

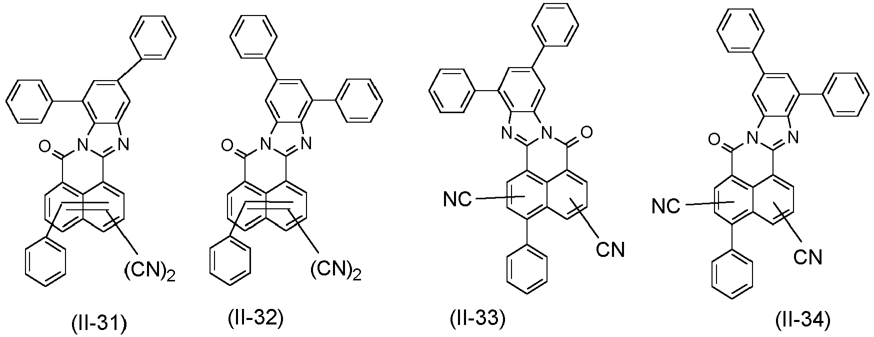

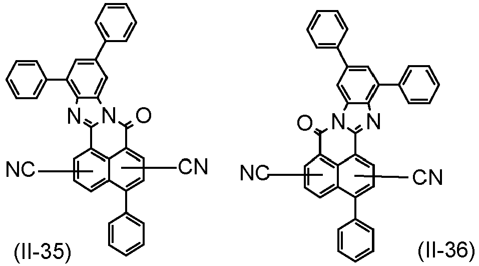

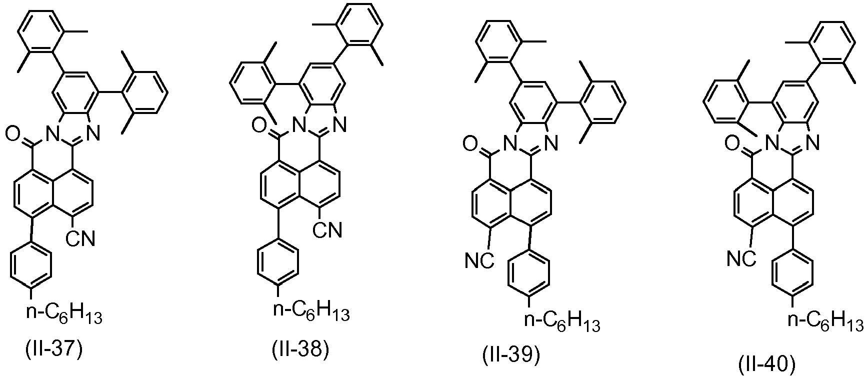

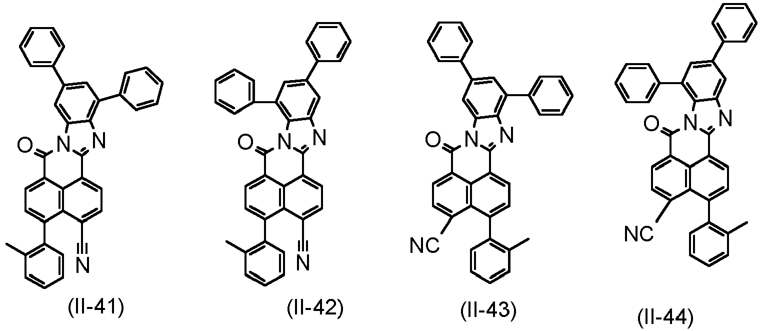

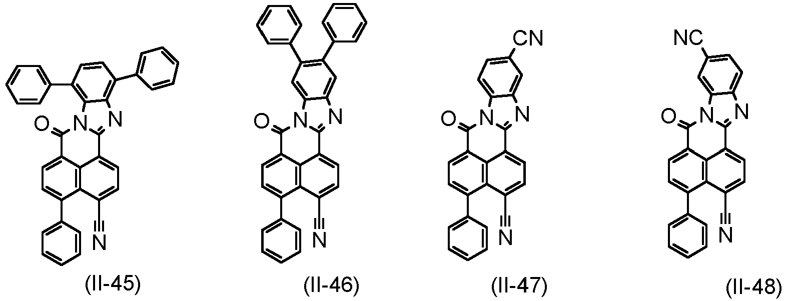

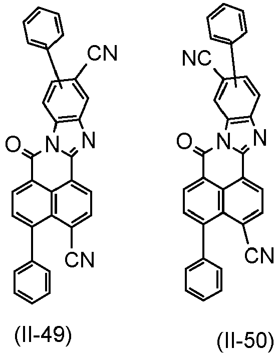

Compounds of the formula (II) are usually green, yellow-green or yellow fluorescent dyes.

Compounds of formula (III) are known from PCT/EP2015/060137. Compounds of formula (III) encompass the following compounds of the formulae III-a and 111-b as well as compounds of formulae III-c and IIII-d: 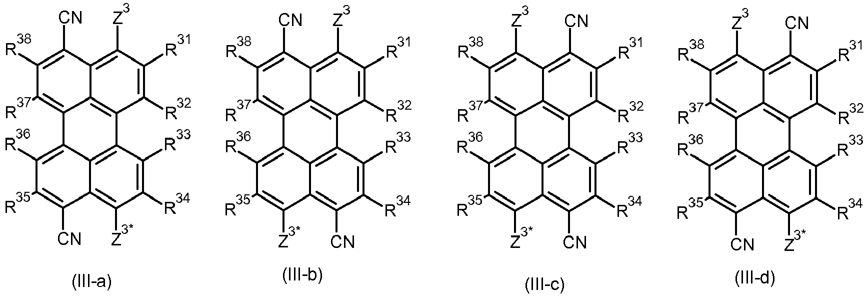 in which
R³¹, R³², R³³ R³⁴, R³⁵, R³⁶, R³⁷, R³⁸, Z³ and Z^{3*} are each as defined above, individually and mixtures thereof.

More particularly, in compounds of the formulae III-a, III-b, III-c and III-d, the R³¹, R³², R³³, R³⁴, R³⁵, R³⁶, R³⁷, R³⁸ substituents that are not cyano or hydrogen are all chlorine or all bromine.

In the perylene compounds of the formula III and mixtures thereof, 1, 2, 3, 4, 5, 6, 7 or 8 of the R³¹, R³², R³³, R³⁴, R³⁵, R³⁶, R³⁷ and R³⁸ substituents are cyano. The other R³¹, R³², R³³ R³⁴, R³⁵, R³⁶, R³⁷ and R³⁸ substituents are each independently hydrogen, bromine or chlorine. More particularly, the other R³¹, R³², R³³, R³⁴, R³⁵, R³⁶, R³⁷ and R³⁸ substituents are each independently hydrogen or bromine. In a further embodiment, the other R³¹, R³², R³³, R³⁴, R³⁵, R³⁶, R³⁷ and R³⁸ substituents are each independently hydrogen or chlorine.

Preferably, 1, 2, 3 or 4 of the R³¹, R³², R³³, R³⁴, R³⁵, R³⁶, R³⁷ and R³⁸ substituents are cyano. The other R³¹, R³², R³³, R³⁴, R³⁵, R³⁶, R³⁷, and R³⁸ substituents are each independently hydrogen, bromine or chlorine. Specifically, 1, 2, 3 or 4 of the R³², R³³, R³⁶, and R³⁷, substituents are cyano. In a specific embodiment, none of the R³¹, R³², R³, R³⁴, R³⁵, R⁶, R³⁷, or R³⁸ substituents is bromine or chlorine.

In a further preferred embodiment, one of the R³¹, R³², R³³, R³⁴, R³⁵, R³⁶, R³⁷ and R³⁸ substituents is cyano, and the other R³¹, R³², R³³, R³⁴, R³⁵, R³⁶, R³⁷ and R³⁸ substituents are hydrogen. In particular, one of the R³², R³³, R³⁶, and R³⁷, substituents is cyano, and the other R³¹, R³², R³³, R³⁴, R³⁵, R³⁶, R³⁷ and R³⁸ substituents are hydrogen.

In a further preferred embodiment, two of the R³¹, R³², R³³, R³⁴, R³⁵, R³⁶, R³⁷ and R³⁸ substituents are cyano, and the other R³¹, R³², R³³, R³⁴, R³⁵, R³⁶, R³⁷ and R³⁸ substituents are hydrogen. In particular, two of the R³², R³³, R³⁶ and R³⁷ substituents are cyano, and the other R³¹, R³², R³³, R³⁴, R³⁵, R³⁶, R³⁷ and R³⁸ substituents are hydrogen.

In the perylene compounds of the formula III and mixtures thereof, one of the Z³ substituents is cyano and one of the Z^{3*} substituents is cyano. The other Z³ substituent and the other Z^{3*} substituent are each as defined above and are preferably selected independently from C₁-C₁₀-alkyl, CO₂R³⁹, phenyl-C₁-C₁₀-alkyl and phenyl, where phenyl and the phenyl moiety of phenyl-C₁-C₁₀-alkyl are unsubstituted or bear one or more, for example 1, 2 or 3, substituents selected from C₁-C₆-alkyl, where R³⁹ is as defined above. Preferably, R³⁹ is linear or branched C₁-C₆-alkyl, specifically methyl, ethyl, n-propyl, isopropyl, n-butyl or isobutyl.

Even more preferably, one of the Z³ substituents is C₁-C₆-alkyl, C₁-C₆-alkoxycarbonyl or phenyl which is unsubstituted or bears 1, 2 or 3 C₁-C₄-alkyl groups. Specifically, one of the Z³ substituents is C₁-C₆-alkyl such as methyl, ethyl, n-propyl, isopropyl, n-butyl, isobutyl, C₁-C₆-alkoxycarbonyl such as methoxycarbonyl, ethoxycarbonyl, n-propoxycarbonyl, isopropoxycarbonyl, n-butoxycarbonyl, isobutoxycarbonyl, phenyl, or phenyl bearing 1, 2 or 3 C₁-C₄-alkyl groups, such as 2-methylphenyl or 2,6-dimethylphenyl.

Even more preferably, one of the Z^{3*} substituents is C₁-C₆-alkyl, C₁-C₆-alkoxycarbonyl or phenyl which is unsubstituted or bears 1, 2 or 3 C₁-C₄-alkyl groups. Specifically, one of the Z^{3*} substituents is C₁-C₆-alkyl such as methyl, ethyl, n-propyl, isopropyl, n-butyl, isobutyl, C₁-C₆-alkoxycarbonyl such as methoxycarbonyl, ethoxycarbonyl, n-propoxycarbonyl, isopropoxycarbonyl, n-butoxycarbonyl, isobutoxycarbonyl, phenyl, 2-methylphenyl or 2,6-dimethylphenyl.

Especially, the perylene compound of the formula IIII is selected from compounds of the formulae (III-1), (III-2), (III-3), (III-4), (III-5), (III-6), (III-7), (III-8), (III-9), (III-10), (III-11), (III-12), (III-13), (III-14), (III-15), (III-16), (III-17), (III-18), (III-19), (III-20) 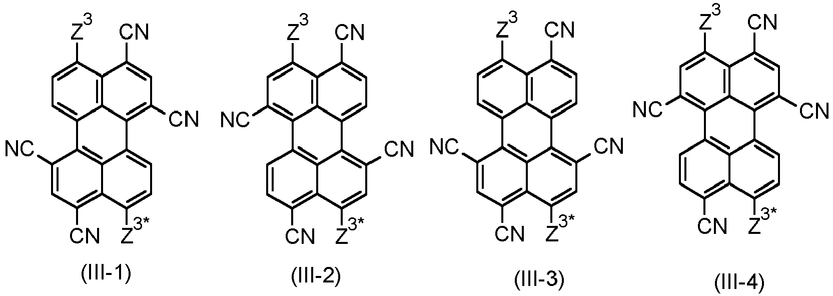 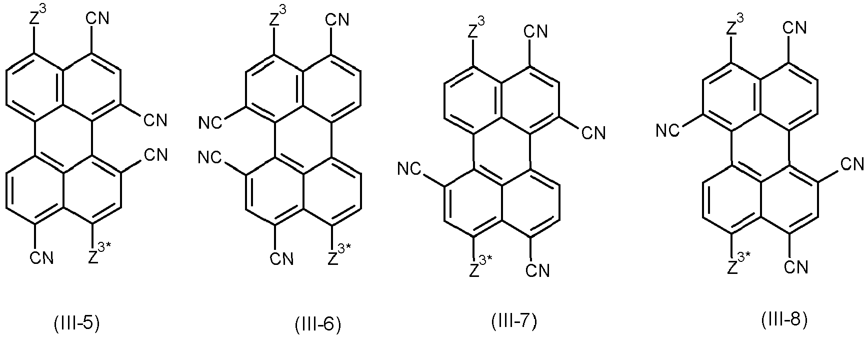 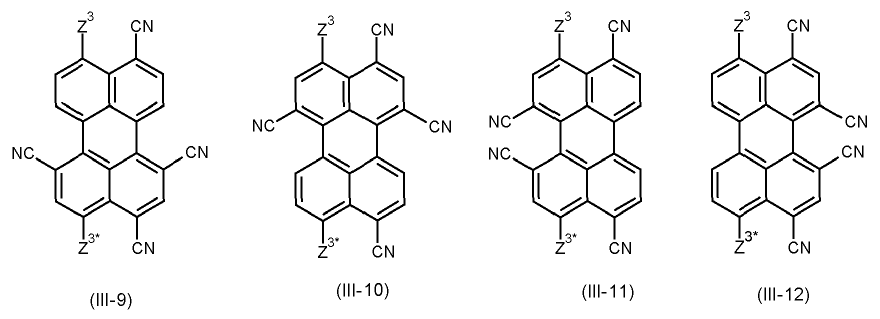 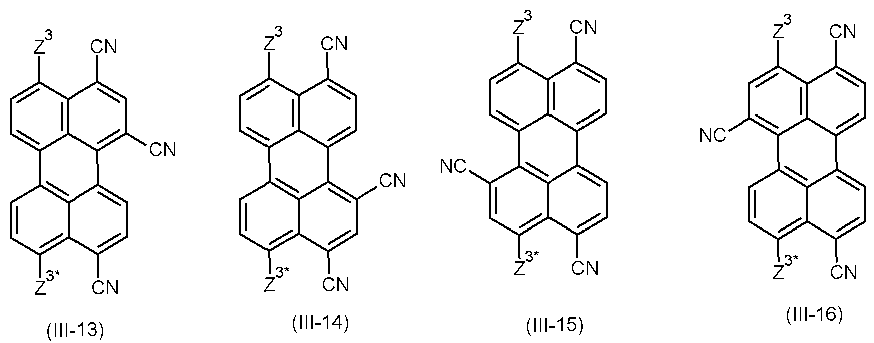 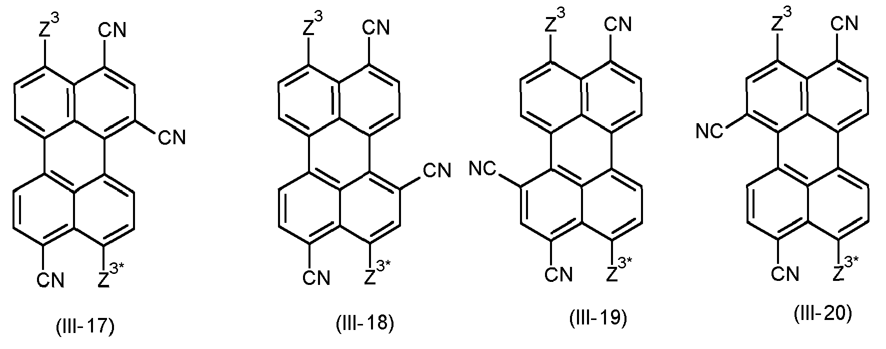 in which
- Z³: is selected from C₁-C₆-alkyl, C₁-C₆-alkoxycarbonyl, phenyl, and phenyl bearing 1, 2 or 3 C₁-C₄-alkyl groups; and
- Z^{3*}: is selected from C₁-C₆-alkyl, C₁-C₆-alkoxycarbonyl, phenyl, and phenyl bearing 1, 2 or 3 C₁-C₄-alkyl groups;
and mixtures thereof.

Among these, specific preference is given to perylene compounds of the formulae (III-1), (III-2), (III-3), (III-4), (III-5), (III-6), (III-7), (III-8), (III-9), (III-10), (III-11), (III-12), (III-13), (III-14), (III-15), (III-16), (III-17), (III-18), (III-19), (III-20) in which Z and Z* have the same definition.

A compound of the formula (III) corresponding to a compound of formula (III-A) 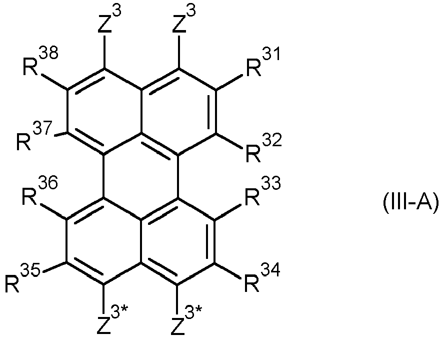 wherein
one of the Z³ substituents is cyano and the other Z³ substituent is C₁-C₁₈-alkyl, C₂-C₁₈-alkenyl, C₂-C₁₈-alkynyl, C₃-C₁₂-cycloalkyl or C₆-C₁₄-aryl, where
C₁-C₁₈-alkyl, C₂-C₁₈-alkenyl, C₂-C₁₈-alkynyl are unsubstituted or bear one or more identical or different Z^{3a} substituents, where Z^{3a} is as defined above;
C₃-C₁₂-cycloalkyl is unsubstituted or bears one or more identical or different Z^{3b} substituents, where Z^{3b} is as defined above; and
C₆-C₁₄-aryl is unsubstituted or bears one or more identical or different Z^{3Ar} substituents, where Z^{3Ar} is as defined above;
one of the Z^{3*} substituents is cyano and the other Z^{3*} substituent is C₁-C₁₈-alkyl, C₂-C₁₈-alkenyl, C₂-C₁₈-alkynyl, C₃-C₁₂-cycloalkyl or C₆-C₁₄-aryl, where
C₁-C₁₈-alkyl, C₂-C₁₈-alkenyl, C₂-C₁₈-alkynyl are unsubstituted or bear one or more identical or different Z^{3a} substituents, where Z^{3a} is as defined above;
C₃-C₁₂-cycloalkyl is unsubstituted or bears one or more identical or different Z^{3b} substituents, where Z^{3b} is as defined above; and
C₆-C₁₄-aryl is unsubstituted or bears one or more identical or different Z^{3Ar} substituents, where Z^{3Ar} is as defined above;
R³¹ R³⁴, R³⁵ and R³⁸ are each hydrogen;
two of the R³², R³³, R³⁶, or R³⁷ substituents are hydrogen; and the other R³², R³³, R³⁶ or R³⁷ substituents are cyano;
or mixtures thereof,
is obtainable by a process in which
a) perylene of formula (III-II) 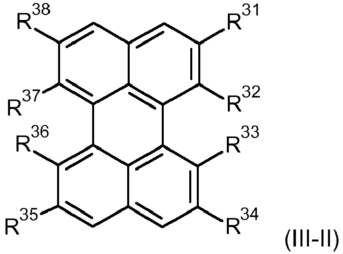 in which
   R³¹, R³², R³³, R³⁴, R³⁵, R³⁶, R³⁷ and R³⁸ are each hydrogen
   is halogenated to obtain a mixture of 3,9-dihaloperylene of the formula (III-IIIa) and 3,10-dihaloperylene of the formula (III-IIIb) 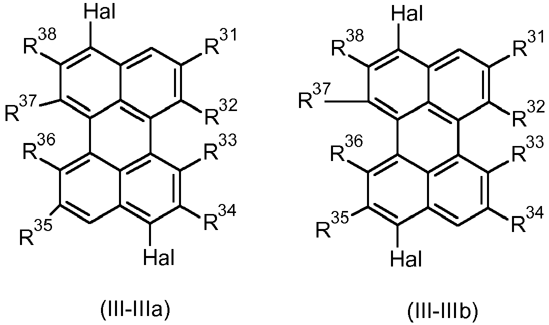 in which
   R³¹, R³², R³³, R³⁴, R³⁵, R³⁶, R³⁷ and R³⁸ are each hydrogen; and
   Hal are each all chlorine or bromine;
b) the mixture of compounds of the formulae IIIa and IIIb obtained in step a) is reacted with an organometallic compound of the formula (III-IV)

   Z³-Met (III-IV)

   and optionally with an organometallic compound of the formula (III-V)

   Z^{3*}-Met (III-V)

   in which
   - Z³: is selected from C₁-C₁₈-alkyl, C₂-C₁₈-alkenyl, C₂-C₁₈-alkynyl, C₃-C₁₂-cycloalkyl and C₆-C₁₄-aryl, where C₁-C₁₈-alkyl, C₂-C₁₈-alkenyl, C₂-C₁₈-alkynyl are unsubstituted or bear one or more identical or different Z^{3a} substituents,
   C₃-C₁₂-cycloalkyl is unsubstituted or bears one or more identical or different Z^{3b} substituents, and
   C₆-C₁₄-aryl is unsubstituted or bears one or more identical or different Z^{3Ar} substituents;
   - Z^{3*}: is selected from C₁-C₁₈-alkyl, C₂-C₁₈-alkenyl, C₂-C₁₈-alkynyl, C₃-C₁₂-cycloalkyl and C₆-C₁₄-aryl, where C₁-C₁₈-alkyl, C₂-C₁₈-alkenyl, C₂-C₁₈-alkynyl are unsubstituted or bear one or more identical or different Z^{3a} substituents,
   C₃-C₁₂-cycloalkyl is unsubstituted or bears one or more identical or different Z^{3b} substituents, and
   C₆-C₁₄-aryl is unsubstituted or bears one or more identical or different Z^{3Ar} substituents;
   where Z^{3*} may also be as defined for Z³;
   - Met: is B(OH)₂, B(OR^{3'})(OR^{3"}), Zn-Hal or Sn(R^{3*})₃, in which
   R^{3'} and R^{3"} are each independently hydrogen, C₁-C₃₀-alkyl, C₅-C₈-cycloalkyl, C₆-C₁₄-aryl or heteroaryl, or R^{3'} and R^{3"} together are C₂-C₄-alkylene which optionally bears 1, 2, 3, 4, 5, 6, 7 or 8 substituents selected from C₁-C₄-alkyl, C₅-C₈-cycloalkyl, C₆-C₁₄-aryl and heteroaryl,
   - Hal: is chlorine or bromine, and
   - R^{3*}: is C₁-C₈-alkyl or phenyl,
   to obtain a mixture of compounds of the formulae (III-VIa) and (III-VIb), 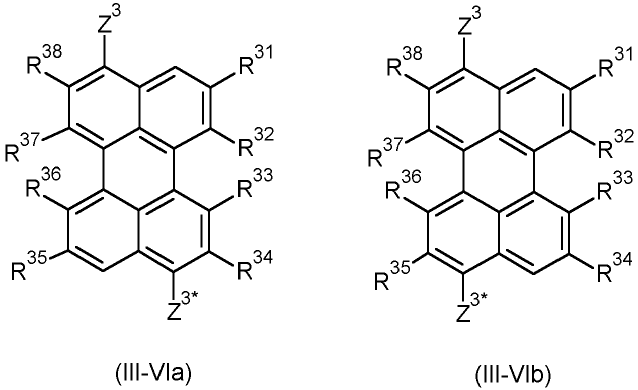 in which
   R³¹, R³², R³³, R³⁴, R³⁵, R³⁶, R³⁷ and R³⁸ are each hydrogen; and
   Z³ and Z^{3*} are each as defined above;
c) the mixture of compounds of the formulae (III-VIa) and (III-VIb) obtained in step b) is halogenated to obtain a reaction mixture which comprises compounds of the formulae (III-VIIa) and (III-VIIb) 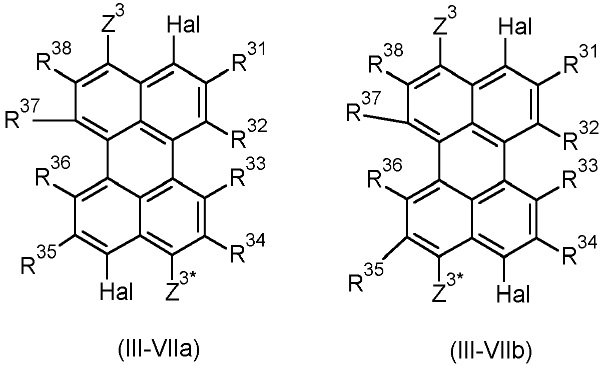 in which
   Z³ and Z^{3*} are each as defined above;
   Hal is halogen selected from chlorine and bromine, where the Hal substituents are either all chlorine or all bromine,
   R³¹, R³², R³³, R³⁴, R³⁵, R³⁶, R³⁷ and R³⁸ are each hydrogen or halogen selected from chlorine and bromine, where the R³¹, R²³, R³³, R³⁴, R³⁵, R³⁶, R³⁷ and R³⁸ substituents that are not hydrogen are either all chlorine or all bromine;
d) the compounds of the formulae (III-VIIa) and (III-VIIb) present in the reaction mixture obtained in step c) are subjected to a substitution of halogen for cyano, and optionally partly for hydrogen, to obtain at least one compound of the formula (III-A) or mixtures thereof; and
e) the at least one compound of the formula (III-A) or mixtures thereof present in the reaction mixture obtained in step d) is optionally subjected to at least one separation and/or purification step.

### Step a)

The halogenation of perylene of the formula (III-II) is effected typically with a brominating agent or a chlorinating agent, meaning that either all the Hal substituents in the compounds of the formulae (III-IIIa) or (III-IIIb) are bromine or all the Hal substituents are chlorine.

Typically, elemental bromine in a solvent is used as the brominating agent. Further suitable brominating agents are N-bromosuccinimide and dibromoisocyanuric acid. Suitable solvents are water or aliphatic monocarboxylic acids, and chlorinated hydrocarbons such as chlorobenzene and chloroform. Suitable aliphatic monocarboxylic acids are those having 2 to 6 carbon atoms, such as acetic acid, propionic acid, butyric acid, pentanecarboxylic acid and hexanecarboxylic acid, and mixtures thereof. When an aliphatic monocarboxylic acid is used as a solvent, it may be advantageous to use iodine as a catalyst.

Suitable chlorinating agents are chlorine in a solvent, e.g. tetrachloromethane. Likewise suitable are N-chlorosuccinimide and dichloroisocyanuric acid. Chlorination with dichloroisocyanuric acid is effected preferably in concentrated sulfuric acid.

The molar ratio of brominating agent to perylene of the formula (III-II) is typically about 10:1 to 2.5:1, more preferably 9:1 to 3.0:1. The molar ratio is especially 8.5:1 to 3.5:1. The molar ratio of chlorinating agent to perylene of the formula (III-II) is typically about 10:1 to 2.5:1, more preferably 9:1 to 3.0:1. The molar ratio is especially 8.5:1 to 3.5:1.

The dihalogenated compounds of the formulae (III-IIIa) and (III-IIIb) obtained in reaction step a) are generally used in step b) without further purification.

### Step b)

In the reaction in step b), the compounds of the formulae (III-IIIa) and (III-IIIb) obtained in step a) are subjected to a cross-coupling with an organometallic compound of the formula (III-IV) and optionally with an organometallic compound of the formula (III-V).

Preference is given to effecting the reaction in the presence of catalytically active amounts of a transition metal of transition group VIII of the Periodic Table (group 10 according to IUPAC), for example nickel, palladium or platinum, especially in the presence of a palladium catalyst. Suitable catalysts are, for example, palladium-phosphine complexes such as tetrakis(triphenylphosphine)palladium(0), PdCl₂(o-tolyl₃P)₂, bis(triphenylphosphine)palladium(II) chloride, the [1,1'-bis(diphenyl-phosphino)ferrocene]palladium(II) chloride-dichloromethane complex, bis[1,2-bis(diphenylphosphino)ethane]palladium(0) and [1,4-bis(diphenylphosphino)butane]-palladium(II) chloride, palladium on activated carbon in the presence of phosphine compounds, and palladium(II) compounds such as palladium(II) chloride or bis(acetonitrile)palladium(II) chloride in the presence of phosphine compounds such as triphenylphosphine, 1,1'-bis(diphenylphosphino)ferrocene, 1,2-bis(diphenylphosphino)-ethane, 1,3-bis(diphenylphosphino)propane and 1,4-bis(diphenylphosphino)butane. The amount of catalyst is typically 10 to 150 mol%, based on the compounds of the formulae (III-IIIa) and (III-IIIb).

Especially suitable organometallic compounds (III-IV) are an appropriately substituted arylboronic acid and arylboronic esters (compounds III-IV where Met = B(OH)₂ or B(OR^{3'})(OR^{3"}) where R³¹, R^{3"} = C₁-C₄-alkyl, or R^{3'} and R^{3"} together are C₂-C₄-alkylene optionally bearing 1, 2, 3 or 4 substituents selected from C₁-C₄-alkyl).

The reaction is effected under the conditions of a Suzuki coupling, as known, for example, from Suzuki et al., Chem. Rev., 1995, 95, 2457-2483 and the literature cited therein. The arylboronic acids and esters thereof are known from the literature, commercially available, or can be prepared from the corresponding arylmagnesium compounds by reaction with appropriate boric esters. Suitable organometallic compounds (III-IV) are additionally alkylboronic acid or alkylboronic esters.

Suitable organometallic compounds (III-IV) are especially also arylstannanes, cycloalkylstannanes, alkynylstannanes, alkenylstannanes or alkylstannanes (compounds (III-IV) where Met = Sn(R^{3*})₃ where R^{3*} = C₁-C₄-alkyl). In that case, the reaction is effected under the conditions of a Stille coupling, as known, for example, from D. Milstein, J. K. Stille, J. Am. Chem. Soc. 1978, 100, p. 3636-3638 or V. Farina, V. Krishnamurthy, W. J. Scott, Org. React. 1997, 50, 1-652. Stannanes of the formula (III-IV) are either known or can be prepared by commonly known processes.

Suitable organometallic compounds (III-IV) are additionally organozinc compounds (compounds (III-IV) where Met = Zn-Hal where Hal = Cl, Br, especially Br). In that case, the reaction is effected under the conditions of a Negishi coupling, as known, for example, from A. Lützen, M. Hapke, Eur. J. Org. Chem., 2002, 2292-2297. Arylzinc compounds of the formula (III-IV) or alkylzinc compounds of the formula (III-IV) are either known or can be prepared by commonly known processes.

The reaction of (III-IIIa) and (III-IIIb) with the organometallic compound (III-IV), especially in the case of the Suzuki coupling, is effected under basic conditions. Suitable bases are alkali metal carbonates and alkali metal hydrogencarbonates such as sodium carbonate, potassium carbonate, cesium carbonate, sodium hydrogencarbonate, alkaline earth metal carbonates and alkaline earth metal hydrogencarbonates such as magnesium carbonate or magnesium hydrogencarbonate, or tertiary amines such as triethylamine, trimethylamine, triisopropylamine or N-ethyl-N-diisopropylamine.

Typically, the coupling of the compounds (III-IIIa) and (III-IIIb) with the compound (III-IV) is effected in a solvent. Suitable solvents are organic solvents such as aromatics, e.g. toluene, mesitylene, acyclic ethers, e.g. 1,2-dimethoxyethane, cyclic ethers such as tetrahydrofuran or 1,4-dioxane, polyalkylene glycols such as diethylene glycol, carbonitriles such as acetonitrile, propionitrile, carboxamides such as dimethylformamide or dimethylacetamide. In the Suzuki coupling, the aforementioned solvents can also be used in a mixture with water; for example, the ratio of organic solvent to water may be in the range from 5:1 to 1:5.

At least one mole of the organometallic compound (III-IV) is used per mole of halogen atom to be exchanged. It may be advantageous to use a 5 to 30% molar excess of organometallic compound of the formula (III-IV) per mole of halogen atom to be exchanged.

If Z³ is different than Z^{3*}, a further coupling with an organometallic compound of the formula V is subsequently conducted. In terms of the process, the procedure is as in the reaction of the compound of the formulae (III-IIIa) and (III-IIIb) with the organometallic compound (III-IV).

### Step c)

The halogenation of compounds of the formulae (III-VIa) and (III-VIb) is typically effected with a brominating agent or a chlorinating agent. Suitable brominating agents or chlorinating agents are those mentioned in step a). In general, the molar ratio of brominating agent to compound of the formulation (III-VIa) and (III-VIb) to be halogenated is 10:1 to 30:1, preferably 15:1 to 25:1.

Step c) of the process according to the invention is typically undertaken in the presence of a solvent at elevated temperatures. Suitable solvents are aprotic solvents such as halogenated aromatics such as chlorobenzene or dichlorobenzenes or halogenated hydrocarbons. Also suitable are aqueous aprotic solvents.

It may be advantageous to conduct step c) in the presence of catalytic amounts of iodine.

The reaction temperature in step c) is typically 50°C up to the boiling temperature of the solvent, in particular 80 to 150°C.

### Step d)

Suitable process conditions for cyano-dehalogenation are described in J. March, Advanced Organic Chemistry, 4th edition, John Wiley & Sons Publishers (1992), p. 660-661, and in WO 2004/029028. One example is the reaction with copper cyanide. Additionally suitable are alkali metal cyanides such as potassium cyanides and sodium cyanide, and also zinc cyanide. Typically, the cyanide source is used in excess. The reaction is generally effected in polar aprotic solvents in the presence of transition metals such as Pd(II) salts or Pd complexes, copper complexes or nickel complexes. The palladium catalyst can be prepared in situ from Pd(0) complexes such as tris(dibenzylideneacetone)dipalladium(0) and 1,1'-bis(diphenylphosphino)ferrocene. Preferred polar aprotic solvents are dimethylformamide, N-methylpyrrolidone, (CH₃)₂SO, dimethyl sulfone and sulfolane. The reaction is performed typically at temperatures of 80 to 160°C, preferably 100 to 140°C, especially preferably 130 to 150°C. The molar ratio of halogen atom to be exchanged to zinc cyanide is typically 1:1 to 1:3, preferably 1.5:2.5. Alternatively, it is also possible to use copper cyanide in N-methylpyrrolidone or sulfolane in the absence of a catalyst.

### Step e)

Optionally, the reaction mixture obtained in step d), comprising at least one perylene compound of the formula III-A or mixtures thereof, is subjected to a partial or complete separation and/or a purifying step. The separation and/or purification in step e) can be effected by customary processes known to those skilled in the art, such as extraction, distillation, recrystallization, separation on suitable stationary phases, and a combination of these measures.

It may be advantageous to undertake a partial or full separation of the isomers obtained after reaction step a) and/or b) and/or c).

A cyanated perylene compound of the formula III corresponding to the formula III-B 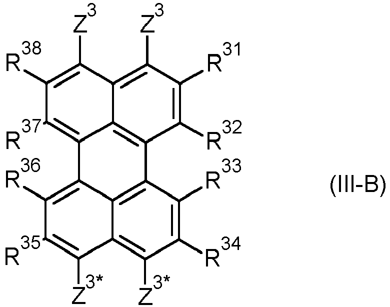 in which
one of the Z³ substituents is cyano and the other Z³ substituent is COOR³⁹;
one of the Z^{3*} substituents is cyano and the other Z^{3*} substituent is COOR³⁹;
R³¹, R³⁴, R³⁵ and R³⁸ are hydrogen;
one of the R³², R³³, R³⁶, or R³⁷ substituents is cyano and the other R³², R³³, R³⁶ and R³⁷ substituents are hydrogen;
R³⁹ is hydrogen, C₁-C₁₀-alkyl, C₂-C₁₀-alkenyl, C₂-C₁₀-alkynyl, C₃-C₁₂-cycloalkyl or C₆-C₁₄-aryl, where
   C₁-C₁₀-alkyl, C₂-C₁₀-alkenyl, C₂-C₁₀-alkynyl are unsubstituted or bear one or more identical or different R^{3a} substituents,
   C₃-C₁₂-cycloalkyl is unsubstituted or bears one or more identical or different R^{3b} substituents and
   C₆-C₁₄-aryl is unsubstituted or bears one or more identical or different R^{3Ar} substituents,
where R^{3a}, R^{3b} and R^{3Ar} are each as defined above,
or mixtures thereof,
is obtainable by a process in which
f) a mixture of perylene compounds of the formulae (III-VIIIa) and (III-VIIIb) 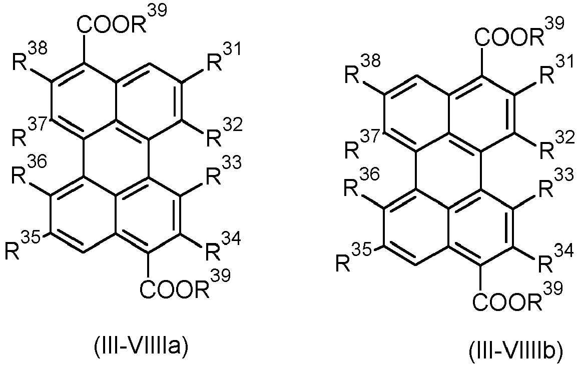 in which
   R³¹, R³², R³³ R³⁴, R³⁵, R³⁶, R³⁷ and R³⁸ are hydrogen; and
   R³⁹ is as defined above
   is halogenated to obtain a reaction mixture which comprises compounds of the formulae (III-IXa) and (III-IXb) 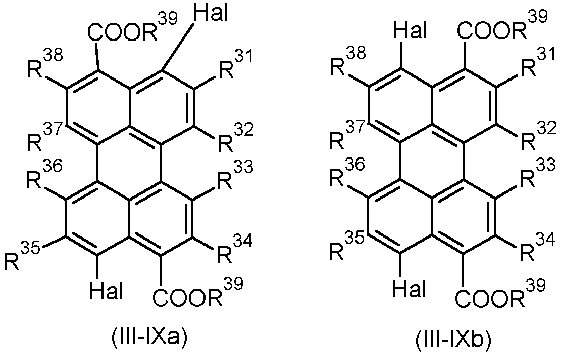 in which
   R³¹, R³², R³³, R³⁴, R³⁵, R³⁶, R³⁷ and R³⁸ are each hydrogen or halogen selected from chlorine and bromine, where the R³¹, R³², R³³, R³⁴, R³⁵, R³⁶, R³⁷ and R³⁸ substituents that are not hydrogen are either all chlorine or all bromine;
   - Hal: is halogen selected from chlorine and bromine, where the Hal substituents are either all chlorine or all bromine; and
   - R³⁹: is as defined above,
   or mixtures thereof;
g) the compounds of the formulae (III-IXa) and(III-IXb) present in the reaction mixture obtained in step f) are subjected to a substitution of halogen for cyano groups, and optionally partly for hydrogen, to obtain at least one compound of the formula III-B or mixtures thereof; and
h) the at least one compound of the formula III-B or mixtures thereof present in the reaction mixture obtained in step g) is optionally subjected to at least one separation and/or purification step.

### Step f)

In terms of the process, step f) is conducted like step c).

### Step g)

In terms of the process, step g) is conducted like step d).

Compounds of the formula (III) are usually yellow or yellow-green fluorescent dyes.

Compounds of the formula (IV) are subject-matter of unpublished EP 15161081.3. Preferred are compounds of the formula (IV), wherein X⁴⁰ is O. Also preferred are compounds of the formula (IV), wherein X⁴⁰ is S.

Preferred are compounds of the formula (IV), wherein two of the radicals R⁴², R⁴³, R⁴⁴ and R⁴⁵ are cyano and the remaining two radicals R⁴², R⁴³, R⁴⁴ and R⁴⁵ are selected from hydrogen and bromine. Even more preferred are compounds of formula (IV), wherein two of the radicals R⁴², R⁴³, R⁴⁴ and R⁴⁵ are cyano and the remaining two radicals R⁴², R⁴³, R⁴⁴ and R⁴⁵ are each hydrogen. In particular, R⁴² and R⁴⁴ are each cyano and R⁴³ and R⁴⁵ are each hydrogen.

Preferred are compounds of the formula (IV), wherein m4 is 0, i.e R⁴¹ is absent.

Preferred are also compounds of the formula (IV), wherein m4 is 1 or 2. In this context, each R⁴¹ is preferably selected from cyano, bromine, chlorine, C₁-C₄-alkyl such as methyl, ethyl, n-propyl, isopropyl, n-butyl or isobutyl, C₁-C₄-alkoxy such as methoxy, ethoxy, n-propoxy, isopropoxy, n-butoxy or isobutoxy, C₁-C₄-haloalkyl, in particular C₁-C₂-haloalkyl, phenyl and phenyloxy, where the phenyl ring in the two last mentioned radicals is unsubstituted or carries 1, 2 or 3 substituents selected from C₁-C₁₀-alkyl. In particular, R⁴¹ is selected from cyano, bromine, and phenyl which is unsubstituted or carries 1 or 2 radicals selected from C₁-C₄-alkyl; especially R⁴¹ is cyano. Especially, m is 1. More especially, m4 is 1 and R⁴¹ is cyano.

Compounds of the formula (IV) where A is a radical of the formula (A.1) are also referred to as compounds of formula (IV-A.1), 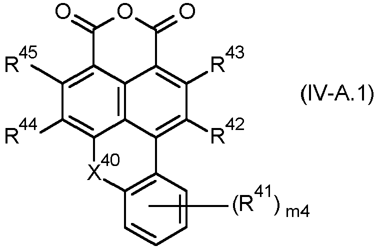 wherein
m4, X⁴⁰, R⁴¹, R⁴², R⁴³, R⁴⁴ and R⁴⁵ are as defined above and especially have one of the preferred meanings.

Examples for compounds of formula (IV-A.1) are shown below:

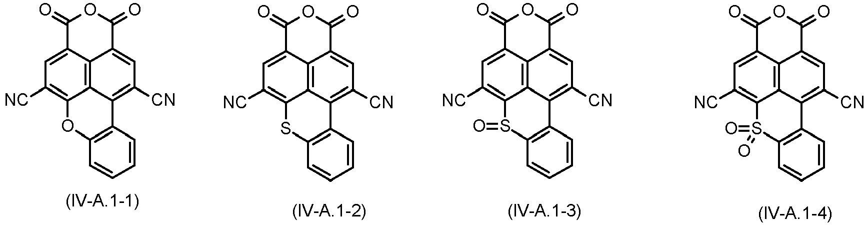

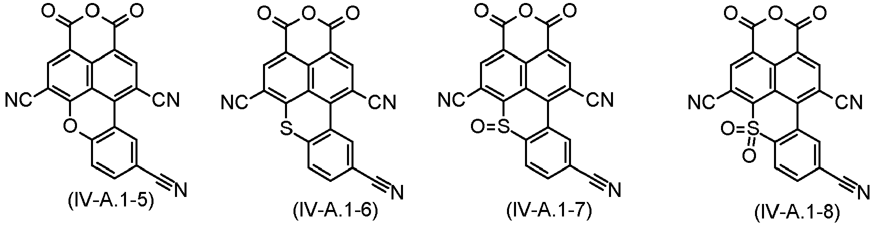

Likewise, compounds of the formula (IV) are preferred, wherein A is a radical of the formula (A.2). Compounds of the formula (IV), where A is a radical of the formula (A.2) are also referred to as compounds of formula (IV-A.2), 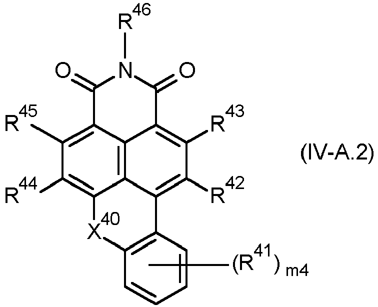 wherein
m4, X⁴⁰, R⁴¹, R⁴², R⁴³, R⁴⁴, R⁴⁵ and R⁴⁶ are as defined above. In particular, m4, X⁴⁰, R⁴¹, R⁴², R⁴³, R⁴⁴ and R⁴⁵ have one of the preferred meanings mentioned above.

In the compounds of the formula (I-A.2), R⁴⁶ is preferably selected from hydrogen, linear C₁-C₂₄-alkyl, branched C₃-C₂₄-alkyl, C₆-C₁₀-aryl and C₆-C₁₀-aryl-C₁-C₁₀-alkylene, where the aryl ring in the two last mentioned moieties is unsubstituted or substituted with 1, 2, 3, 4 or 5 identical or different radicals R^{46a}. In a special embodiment, R⁴⁶ is selected from linear C₁-C₂₄-alkyl, a radical of the formula (B.1) and a radical of the formula (B.2) 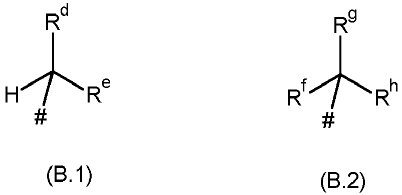 in which
- #: is the bonding site to the nitrogen atom;
- R^{d} and Re,: in the formula (B.1), independently from each other are selected from C₁-C₂₃-alkyl, where the sum of the carbon atoms of the R^{d} and Re radicals is an integer from 2 to 23;
- R^{f}, R^{g} and R^{h},: in the formula (B.2) are independently selected from C₁- to C₂₀-alkyl, where the sum of the carbon atoms of the R^{f}, R^{g} and R^{h} radicals is an integer from 3 to 23.

Preferred radicals of the formula (B.1) are: 1-ethylpropyl, 1-methylpropyl, 1-propylbutyl, 1-ethylbutyl, 1-methylbutyl, 1-butylpentyl, 1-propylpentyl, 1-ethylpentyl, 1-methylpentyl, 1-pentylhexyl, 1-butylhexyl, 1-propylhexyl, 1-ethylhexyl, 1-methylhexyl, 1-hexylheptyl, 1-pentylheptyl, 1-butylheptyl, 1-propylheptyl, 1-ethylheptyl, 1-methylheptyl, 1-heptyloctyl, 1-hexyloctyl, 1-pentyloctyl, 1-butyloctyl, 1-propyloctyl, 1-ethyloctyl, 1-methyloctyl, 1-octylnonyl, 1-heptylnonyl, 1-hexylnonyl, 1-pentylnonyl, 1-butylnonyl, 1-propylnonyl, 1-ethylnonyl, 1-methylnonyl, 1-nonyldecyl, 1-octyldecyl, 1-heptyldecyl, 1-hexyldecyl, 1-pentyldecyl, 1-butyldecyl, 1-propyldecyl, 1-ethyldecyl, 1-methyldecyl, 1-decylundecyl, 1-nonylundecyl, 1-octylundecyl, 1-heptylundecyl, 1-hexylundecyl, 1-pentylundecyl, 1-butylundecyl, 1-propylundecyl, 1-ethylundecyl, 1-methylundecyl, 1-undecyldodecyl, 1-decyldodecyl, 1-nonyldodecyl, 1-octyldodecyl, 1-heptyldodecyl, 1-hexyldodecyl, 1-pentyldodecyl, 1-butyldodecyl, 1-propyldodecyl, 1-ethyldodecyl, 1-methyldodecyl, 1-undecyltridecyl, 1-decyltridecyl, 1-nonyltridecyl, 1-octyltridecyl, 1-heptyltridecyl, 1-hexyltridecyl, 1-pentyltridecyl, 1-butyltridecyl, 1-propyltridecyl, 1-ethyltridecyl, 1-methyltridecyl, 1-tridecyltetradecyl, 1-decyltetradecyl, 1-nonyltetradecyl, 1-octyltetradecyl, 1-heptyltetradecyl, 1-hexyltetradecyl, 1-pentyltetradecyl, 1-butyltetradecyl, 1-propyltetradecyl, 1-ethyltetradecyl, 1-methyltetradecyl, 1-octylhexadecyl, 1-heptylhexadecyl, 1-hexylhexadecyl, 1-pentylhexadecyl, 1-butylhexadecyl, 1-propylhexadecyl, 1-ethylhexadecyl, 1-methylhexadecyl, 1-hexyloctadecyl, 1-pentyloctadecyl, 1-butyloctadecyl, 1-propyloctadecyl, 1-ethyloctadecyl, 1-methyloctadecyl, 1-pentadecyleicosanyl, 1-tetradecyleicosanyl, 1-tridecyleicosanyl, 1-dodecyleicosanyl, 1-undecyleicosanyl, 1-butyleicosanyl, 1-propyleicosanyl, 1-ethyleicosanyl, 1-methyleicosanyl.

Particularly preferred radicals of the formula (B.1) are:
1-methylethyl, 1-methylpropyl, 1-methylbutyl, 1-methylpentyl, 1-methylhexyl, 1-methylheptyl, 1-methyloctyl, 1-ethylpropyl, 1-ethylbutyl, 1-ethylpentyl, 1-ethylhexyl, 1-ethylheptyl, 1-ethyloctyl, 1-propylbutyl, 1-propylpentyl, 1-propylhexyl, 1-propylheptyl, 1-propyloctyl, 1-butylpentyl, 1-butylhexyl, 1-butylheptyl, 1-butyloctyl, 1-pentylhexyl, 1-pentylheptyl, 1-pentyloctyl, 1-hexylheptyl, 1-hexyloctyl, 1-heptyloctyl.

A particularly preferred radical of the formula (B.2) is tert.-butyl.

In a further special embodiment, R⁴⁶ is a radical of the formula (C.1), a radical of the formula (C.2) or a radical of the formula (C.3) 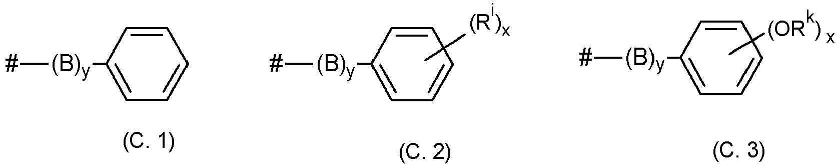 where
- #: represents the bonding side to the nitrogen atom,
- B: where present, is a C₁-C₁₀-alkylene group which may be interrupted by one or more nonadjacent groups selected from -O- and -S-,
- y: is 0 or 1,
- Rⁱ: is independently of one another selected from C₁-C₂₄-alkyl, C₁-C₂₄-fluoroalkyl, fluorine, chlorine or bromine,
- R^{k}: is independently of one another selected from C₁-C₂₄-alkyl,
- x: in formulae C.2 and C.3 is 1, 2, 3, 4 or 5.

Preferably, y is 0, i.e. the variable B is absent.

Irrespectively of its occurrence, Rⁱ is preferably selected from C₁-C₂₄-alkyl, more preferably linear C₁-C₁₀-alkyl or branched C₃-C₁₀-alkyl, especially isopropyl. Irrespectively of its occurrence, R^{k} is preferably selected from C₁-C₃₀-alkyl, more preferably linear C₁-C₁₀-alkyl or branched C₃-C₁₀-alkyl. The variable x in formulae C.2 and C.3 is preferably 1, 2 or 3.

Examples for preferred inventive compounds of formula (IV-A.2) are shown below:

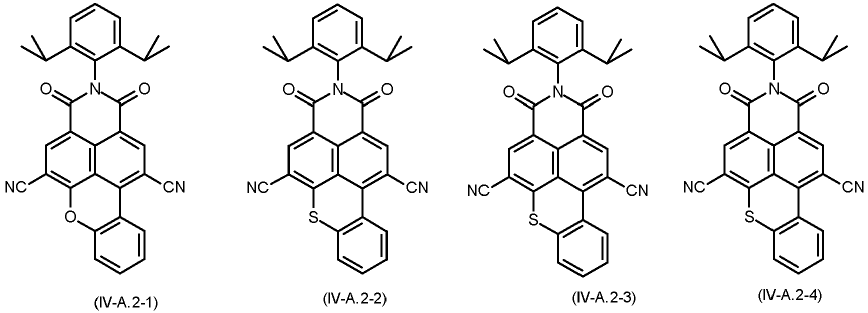

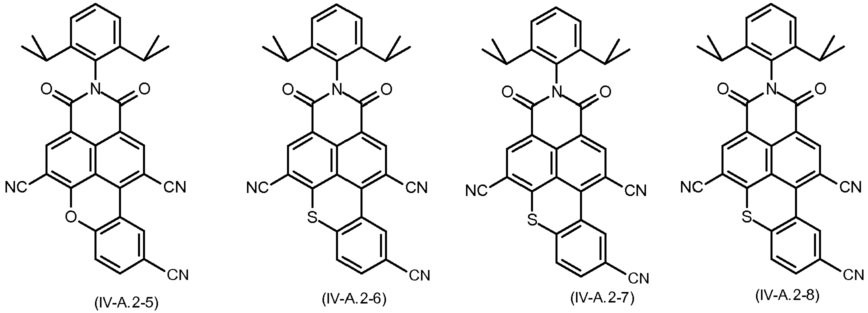

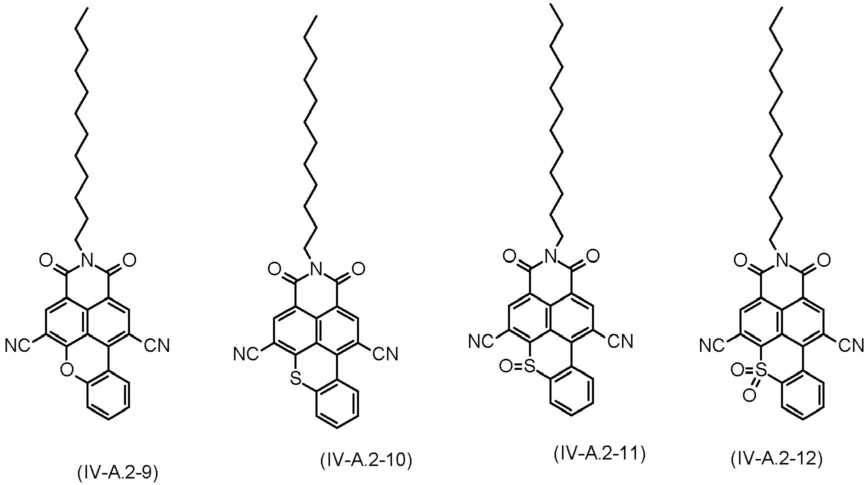

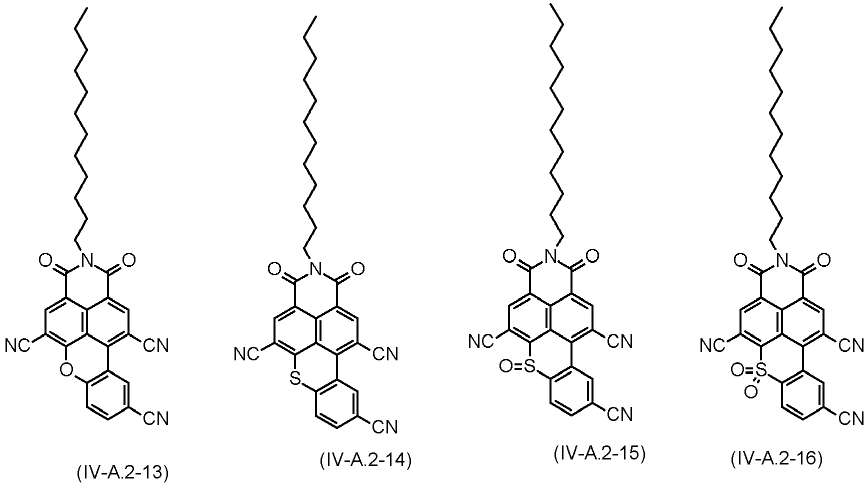

A special group of embodiments relates to compounds of formula (IV-A.2), wherein the variables m4, X⁴⁰, R⁴¹, R⁴², R⁴³ R⁴⁴, and R⁴⁵ independently of each other or in particular in combination, have the following meanings:
- X⁴⁰: is O or S;
- R42 and R⁴⁴: are each cyano;
- R⁴³ and R⁴⁵: are each hydrogen or one of R⁴³ and R⁴⁵ is bromine and the other of R⁴³ and R⁴⁵ is hydrogen;
- R⁴¹: is selected from cyano, bromine, and phenyl which is unsubstituted or carries 1 or 2 radicals selected from C₁-C₄-alkyl;
- R⁴⁶: is selected from hydrogen, C₁-C₂₄-linear alkyl, branched C₃-C₂₄-alkyl, a radical of the formula (C.1), a radical of the formula (C.2) and a radical of the formula (C.3);
- m4: is 0 or 1.

Even more preferably,
- X⁴⁰: is O or S;
- R⁴² and R⁴⁴: are each cyano;
- R⁴³ and R⁴⁵: are each hydrogen;
- R⁴¹: is selected from cyano, bromine, and phenyl which is unsubstituted or carries 1 or 2 radicals selected from C₁-C₄-alkyl; especially cyano;
- R⁴⁶: is selected from linear C₁-C₂₄-alkyl, branched C₃-C₂₄-alkyl, a radical of the formula (C.1), a radical of the formula (C.2) and a radical of the formula (C.3); especially linear C₁-C₂₄-alkyl, branched C₃-C₂₄-alkyl, or phenyl which carries 1 or 2 radicals selected from C₁-C₄-alkyl such as 2,6-diisopropylphenyl ;
- m4: is 0 or 1.

Likewise, compounds of the formula (IV) are preferred, wherein A is a radical of the formula (A.3). This group of embodiments includes the pure regioisomer of the formula (IV-A.3a), the pure regioisomer of the formula (IV-A.3b) and mixtures thereof, 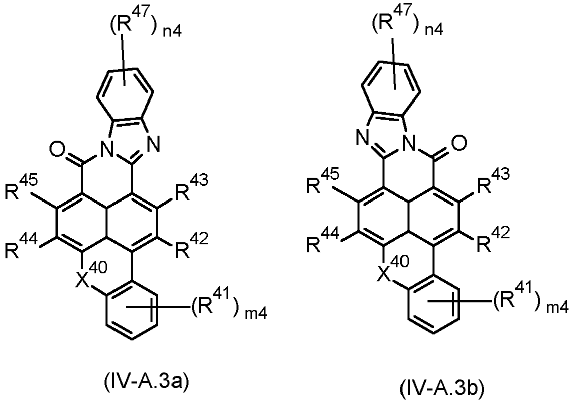 wherein X⁴⁰, R⁴¹ R⁴², Rv³, R⁴⁴, R⁴⁵, R⁴⁷, n4 and m4 are as defined above. In particular, X⁴⁰, R⁴¹ R⁴², R⁴³, R⁴⁴, R⁴⁵, and m4 have one of the preferred meanings mentioned above.

Preferred are compounds of the formulae (IV-A.3a) and (IV-A.3b), wherein n4 is 0, i.e R⁴⁷ is absent. Preferred are also compounds of the formulae (IV-A.3a) and (IV-A.3b), wherein n4 is 1 or 2. In this context, each R⁴⁷ is preferably selected from cyano, bromine, chlorine, C₁-C₄-alkyl such as methyl, ethyl, n-propyl, isopropyl, n-butyl or isobutyl, C₁-C₄-alkoxy such as methoxy, ethoxy, n-propoxy, isopropoxy, n-butoxy or isobutoxy, C₁-C₄-haloalkyl, in particular C₁-C₂-haloalkyl, phenyl and phenyloxy, wherein phenyl in the two last mentioned radicals is unsubstituted or carries 1, 2 or 3 substituents selected from C₁-C₁₀-alkyl. In particular, R⁴⁷ is selected from cyano, bromine and phenyl which is unsubstituted or carries 1 or 2 radicals selected from C₁-C₄-alkyl.

Examples for such preferred compounds are given in tables 1 to 4.

### Table 1:

Compounds of the formulae (IV-A.3a) or (IV-A.3b) and their mixtures, wherein X⁴⁰ is O, and wherein R⁴², R⁴³, R⁴⁴, R⁴⁵, (R⁴¹)ₘ₄ and (R⁴⁷)ₙ₄ have the meanings given in any of lines 1 to 10 of table A.

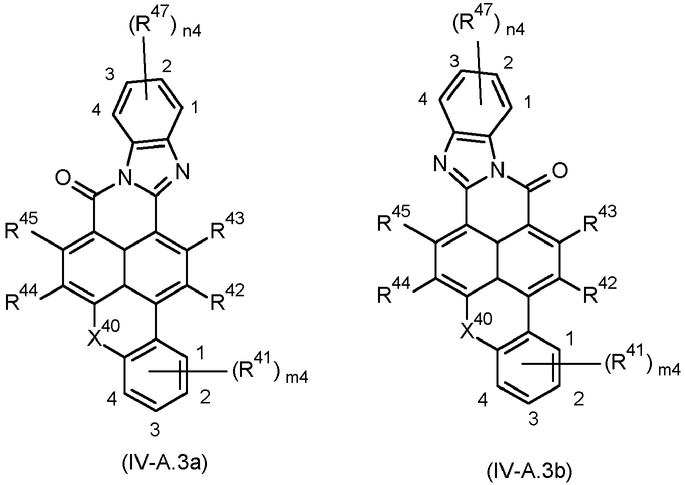

**Table A:**

| | R⁴² | R⁴⁴ | R⁴³ | R⁴⁵ | (R⁴¹)ₘ₄ | (R⁴⁷)ₙ₄ |
|---|---|---|---|---|---|---|
| 1. | CN | CN | H | H | -- | -- |
| 2. | CN | CN | H | H | -- | 1-Ph, 4-Ph |
| 3. | CN | CN | H | H | -- | 1-Ph, 3-Ph |
| 4. | CN | CN | H | H | -- | 2-Ph, 3-Ph |
| 5. | CN | CN | H | H | -- | 2-Ph, 4-Ph |
| 6. | CN | CN | H | H | 2-CN | -- |
| 7. | CN | CN | H | H | 2-CN | 1-Ph, 4-Ph |
| 8. | CN | CN | H | H | 2-CN | 1-Ph, 3-Ph |
| 9. | CN | CN | H | H | 2-CN | 2-Ph, 3-Ph |
| 10. | CN | CN | H | H | 2-CN | 2-Ph, 4-Ph |

In table A the sign " -- " in the definition of (R⁴¹)ₘ₄ has the meaning of m4 being 0, i.e. R⁴¹ is absent; the sign " -- " in the definition of (R⁴⁷)ₙ₄ has the meaning of n4 being 0, i.e. R⁴⁷ is absent; in case that m4 is different from 0, the number in the definition of (R⁴¹)ₘ₄ indicates the position the radical R⁴¹ is attached to the aromatic ring; in case that n4 is different from 0, the numbers in the definition of (R⁴⁷)ₙ₄ indicate the positions the radicals R⁴⁷ are attached to the benzimidazole ring; Ph is phenyl.

Amongst compounds of the formulae (IV-A.3a) or (IV-A.3b), preference is also given to the compounds defined in the following tables 2, 3 and 4:

**Table 2:**

| |
|---|
| Compounds of the formulae (IV-A.3a) or (IV-A.3b) and their mixtures, wherein X⁴⁰ is S, and wherein R⁴², R⁴³, R⁴⁴, R⁴⁵, (R⁴¹)ₘ₄ and (R⁴⁷)ₙ₄ have the meanings given in any of lines 1 to 10 of table A. |

**Table 3:**

| |
|---|
| Compounds of the formulae (IV-A.3a) or (IV-A.3b) and their mixtures, wherein X⁴⁰ is SO, and wherein R⁴², R⁴³, R⁴⁴, R⁴⁵, (R⁴¹)ₘ₄ and (R⁴⁷)ₙ₄ have the meanings given in any of lines 1 to 10 of table A. |

**Table 4:**

| |
|---|
| Compounds of the formulae (IV-A.3a) or (IV-A.3b) and their mixtures, wherein X⁴⁰ is SO₂, and wherein R⁴², R⁴³, R⁴⁴, R⁴⁵, (R⁴¹)ₘ₄ and (R⁴⁷)ₙ₄ have the meanings given in any of lines 1 to 10 of table A. |

According to a further group of embodiments, compounds of the formula (IV) are preferred, wherein A is a radical of the formula (A.4). This group of embodiments includes the pure regioisomer of the formula (IV-A.4a), the pure regioisomer of the formula (IV-A.4b) and mixtures thereof, 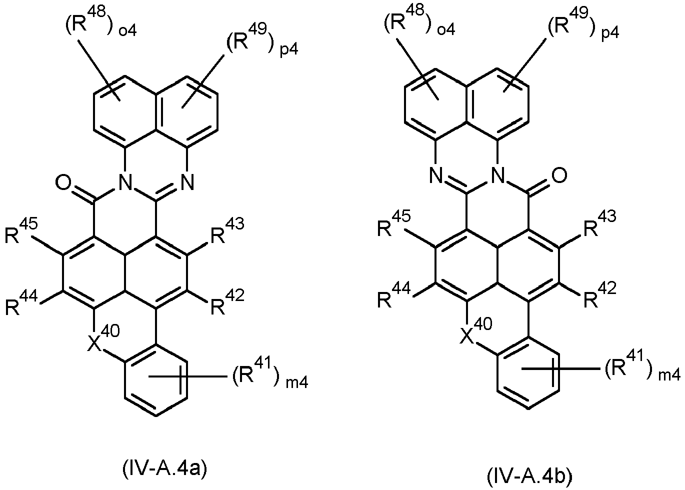 wherein X⁴⁰, R⁴¹, R⁴², R⁴³, R⁴⁴, R⁴⁵, R⁴⁴, R⁴⁹, o4, p4 and m4 are as defined above. In particular, X⁴⁰, R⁴¹, R⁴², R⁴³, R⁴⁴, R⁴⁵, and m4 have one of the preferred meanings mentioned above.

Preferred are compounds of the formulae (IV-A.4a) or (IV-A.4b) and their mixtures, wherein o4 and p4 are 0, i.e R⁴⁸ and R⁴⁹ are absent. Preferred are also compounds of the formulae (IV-A.4a) and (IV-A.4b), wherein the sum of o4 and p4 is 1, 2, 3 or 4. In this context, R⁴⁸ and R⁴⁹ are, independently of each other, preferably selected from cyano, bromine, chlorine, C₁-C₄-alkyl such as methyl, ethyl, n-propyl, isopropyl, n-butyl or isobutyl, C₁-C₄-alkoxy such as methoxy, ethoxy, n-propoxy, isopropoxy, n-butoxy or isobutoxy, C₁-C₄-haloalkyl, in particular C₁-C₂-haloalkyl, phenyl and phenyloxy, wherein phenyl in the two last mentioned radicals is unsubstituted or carries 1, 2 or 3 substituents selected from C₁-C₁₀-alkyl. In particular, R⁴⁸ and R⁴⁹ are, independently of each other, selected from cyano, bromine and phenyl which is unsubstituted or carries 1 or 2 radicals selected from C₁-C₄-alkyl.

Examples for preferred inventive compounds of formulae (IV-A.4a) and (IV-A.4b) are shown below:

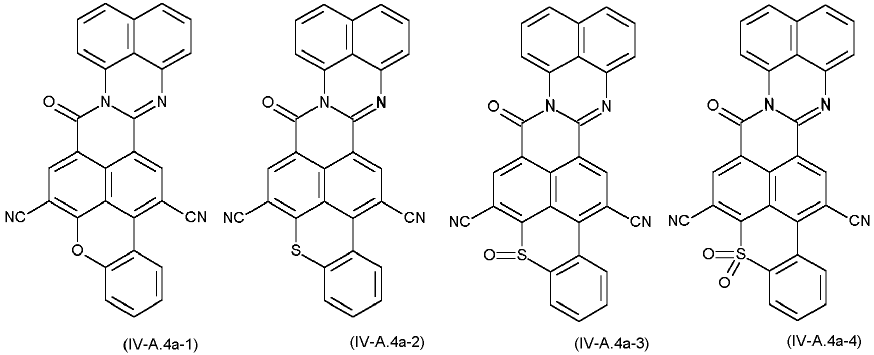

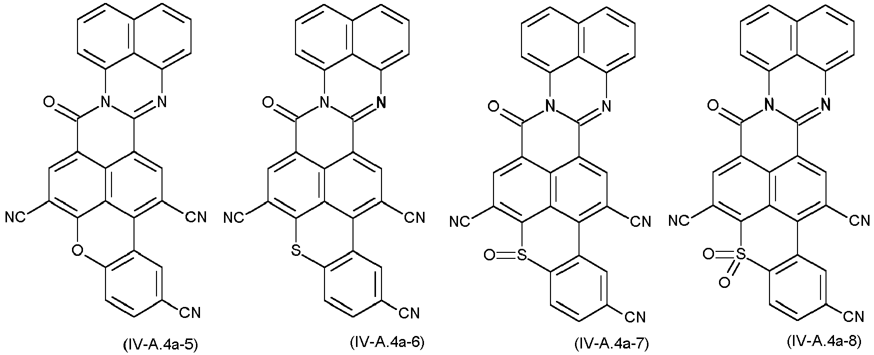

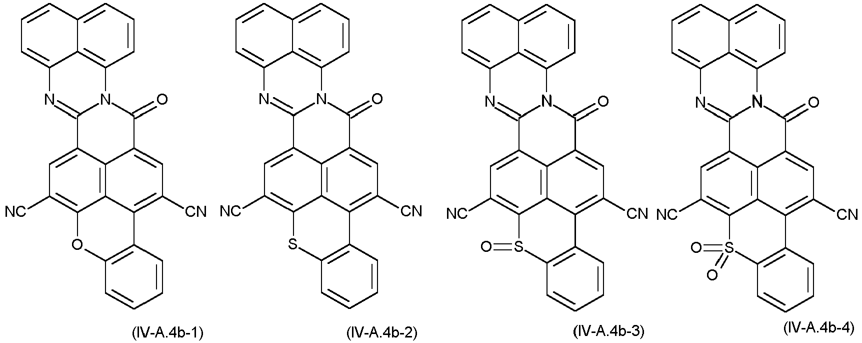

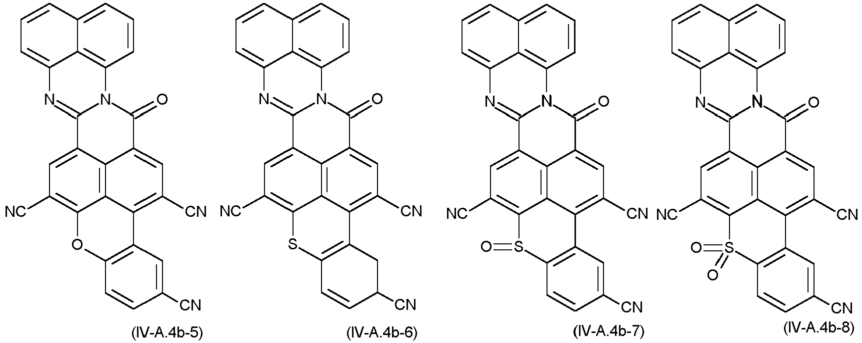

Compounds of the formula (IV) used according to the present invention can be prepared e.g. according to the preparation methods as described below by treating a compound of the formula (IV-II) 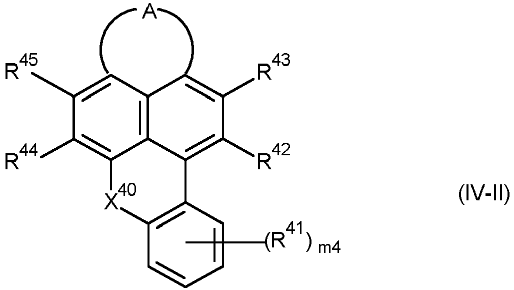 wherein
at least one of the radicals R⁴², R⁴³, R⁴⁴ and R⁴⁵ is selected from bromine and chlorine and the remaining radicals R⁴², R⁴³, R⁴⁴ and R⁴⁵ are hydrogen;
- X⁴⁰: is O, S, SO or SO₂;
- A: is a radical of the general formulae (A.1), (A.2), (A.3), or (A.4), wherein (A.1), (A.2), (A.3), and (A.4) are as defined above;
- R⁴¹: is bromine, chlorine, -NR^{4a}R^{4b}, C₁-C₂₄-alkyl, C₁-C₂₄-haloalkyl, C₁-C₂₄-alkoxy, C₁-C₂₄-haloalkoxy, C₃-C₂₄-cycloalkyl, heterocycloalkyl, heteroaryl, C₆-C₂₄-aryl, C₆-C₂₄-aryloxy, C₆-C₂₄-aryl-C₁-C₁₀-alkylene, where the rings of cycloalkyl, heterocycloalkyl, heteroaryl, aryl, aryloxy and-aryl-alkylene in the six last-mentioned radicals are unsubstituted or substituted with 1, 2, 3, 4 or 5 identical or different radicals R^{41a} and where C₁-C₂₄-alkyl, C₁-C₂₄-haloalkyl, C₁-C₂₄-alkoxy, and the alkylene moiety of C₆-C₂₄-aryl-C₁-C₁₀-alkylene may be interrupted by one or more groups selected from O, S and NR^{4c}, wherein R^{4a}, R^{4b} and R^{4c} are as defined above; and
- m: is 0, 1, 2, 3 or 4,
with a cyanating agent. A suitable cyanating agent is for example copper(I) cyanide.

Suitable process conditions for the exchange of bromine or chlorine with cyano are described e.g. in J. March, Advanced Organic Chemistry, 4th edition, John Wiley & Sons Publishers (1992), p. 660-661, in WO 2004/029028 and WO 2015/019270.

Compounds of the formula (IV-II) can be prepared by treating a compound of the formula (IV-III) 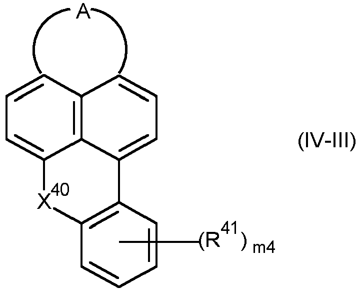 wherein X⁴⁰, A, R⁴¹ and m4 are as defined above
with a halogenating agent selected from brominating agents and chlorinating agents.

Bromination is typically carried out with elemental bromine in a solvent as described e.g. in WO 2014/131628. Further suitable brominating agents are N-bromosuccinimide and dibromoisocyanuric acid. Suitable solvents are water or aliphatic monocarboxylic acids, and chlorinated hydrocarbons such as chlorobenzene and chloroform. Suitable aliphatic monocarboxylic acids are those having 2 to 6 carbon atoms, such as acetic acid, propionic acid, butyric acid, pentanecarboxylic acid and hexanecarboxylic acid, and mixtures thereof. When an aliphatic monocarboxylic acid is used as a solvent, it may be advantageous to use iodine as a catalyst.

Chlorination is typically carried out with elemental chlorine, N-chlorosuccinimide, chlorosulfonic acid, sulfuryl chloride in an inert solvent as described e.g. in US 2011/0068328. A further suitable chlorinating agent is N-chlorosuccinimide.

Depending on the molar ratio of the halogenating agent to compound of the formula (IV-III), a mono-, di- or multihalogene-substituted compound of formula (IV-II), i.e. a mono-, di-, or multibromo-substituted compound of formula (IV-II) and mono-, di- or multichlorine-substituted compound of formula (IV-II), respectively, is obtained which can be separated by column chromatography (SiO₂).

Benzoxanthene compounds or benzothioxanthene compounds of formula (IV), where A is a radical of formulae (A.1) or (A.2) are known in the art and for example described in US 3,748,330, US 3,812,051, GB 1 440 450 or WO 2014/131628. Benzoxanthene compounds or benzothioxanthene compounds of formula (IV), where A is a radical of formulae (A.3) or (A.4) can be prepared in analogy to the method described in WO 2015/019270.

Compounds of the formula (IV), wherein X is SO or SO₂, can be obtained by oxidizing compounds of the formula (IV), wherein X is S. Suitable oxidizing agents are metachloroperbenzoic acid, hypochlorite or hydrogen peroxide.

Benzoxanthene compounds of the formula (V) are known from WO 2014/131628. They are usually yellow or yellow-green fluorescent dyes.

Compounds of the formula (VI) are known from WO 2012/168395. Particular preference is given to the compounds specified in WO 2012/168395, at page 28, line14 to page 32, line 5.

Especially preferably, organic fluorescent dyes of formula VI are selected from compounds of formulae (VI-1), (VI-2), (VI-3), (VI-4), (VI-5), (VI-6), (VI-7), (VI-8), (VI-9), (VI-10), (VI-11), (VI-12), (VI-13), (VI-14), (VI-15), (VI-16), (VI-17), (VI-18), (VI-19), (VI-20), (VI-21), (VI-22), (VI-23), (VI-24), (VI-25), (VI-26), (VI-27), (VI-28), (VI-29), (VI-30), (VI-31), (VI-32), (VI-33), (VI-34), (VI-35), (VI-36), (VI-37), (VI-38), (VI-39), (VI-40), (VI-41), (VI-42), (VI-43), (VI-44), (VI-45), (VI-46), (VI-47), (VI-48), (VI-49), (VI-50), (VI-51), (VI-52), (VI-53), (VI-54), (VI-55), and mixtures thereof 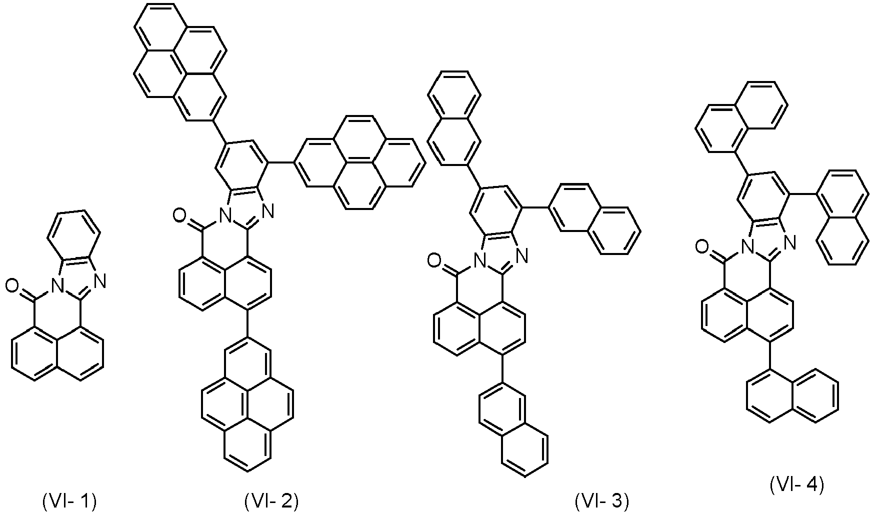 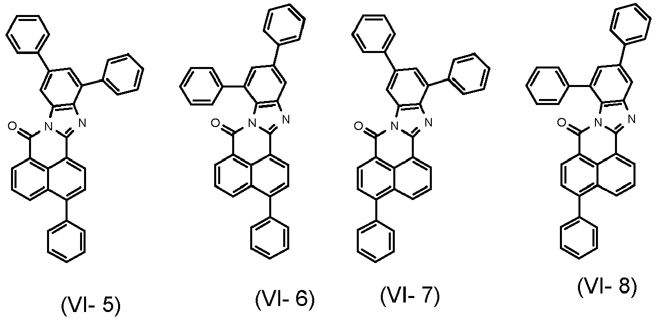 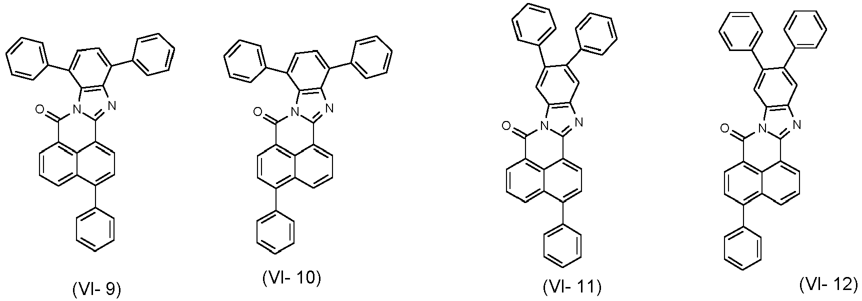 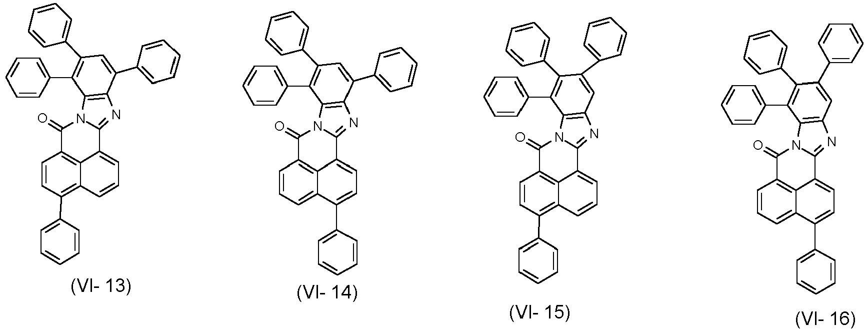 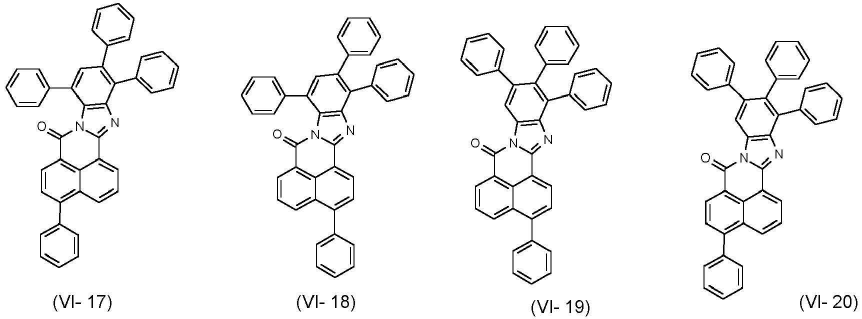 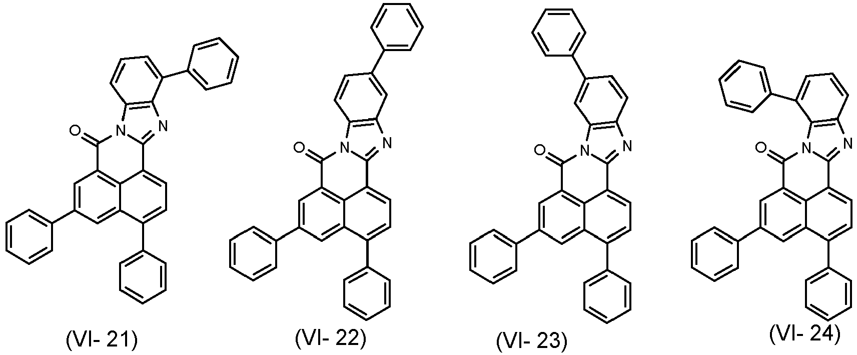 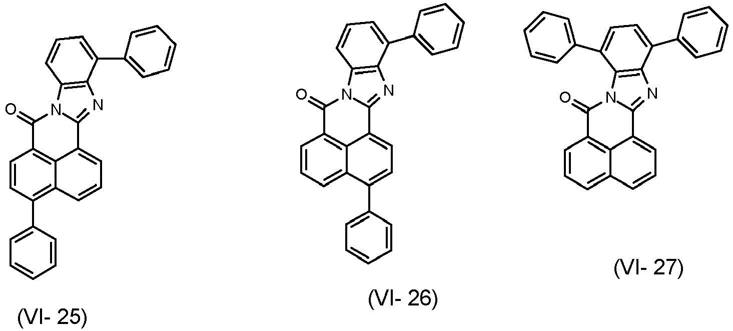 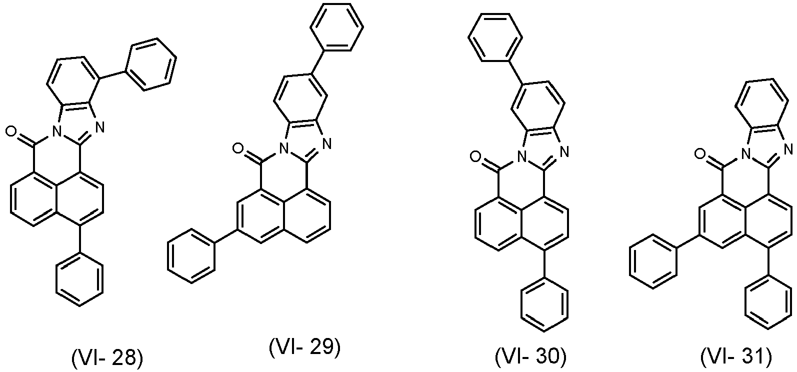 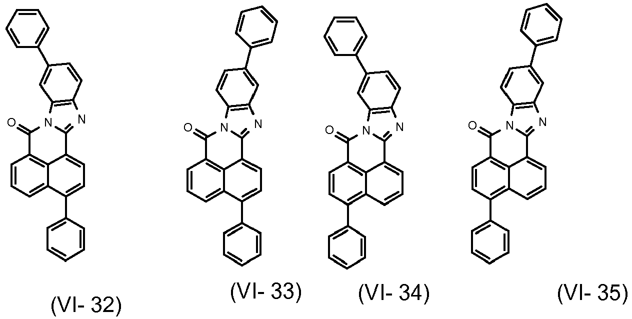 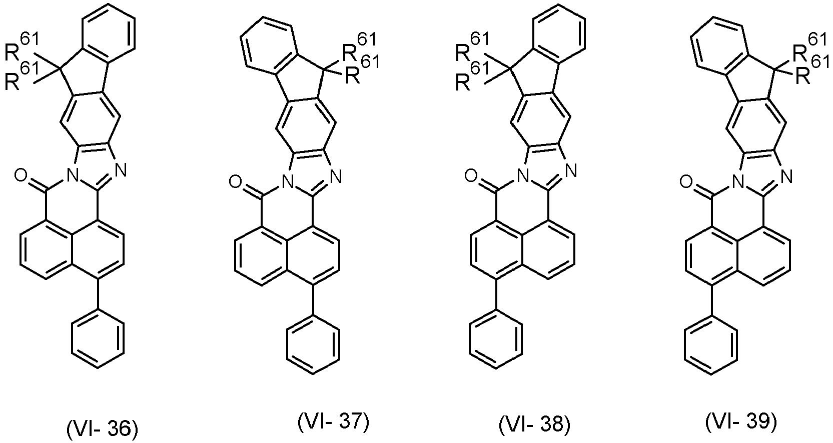 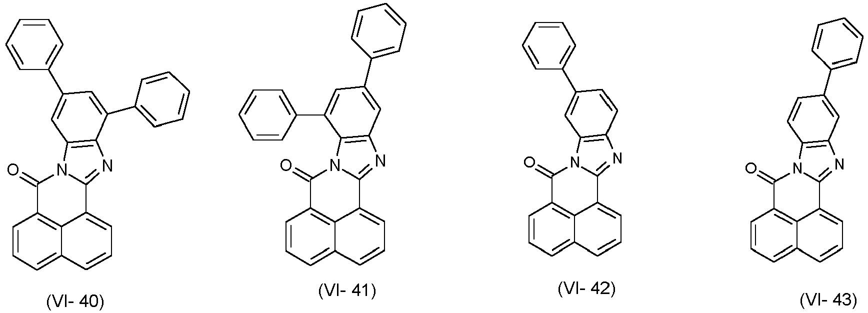 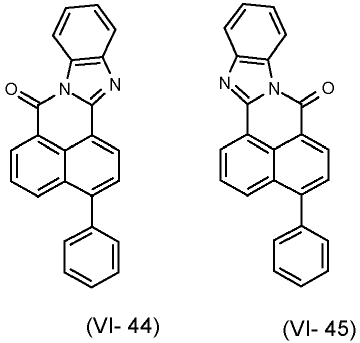 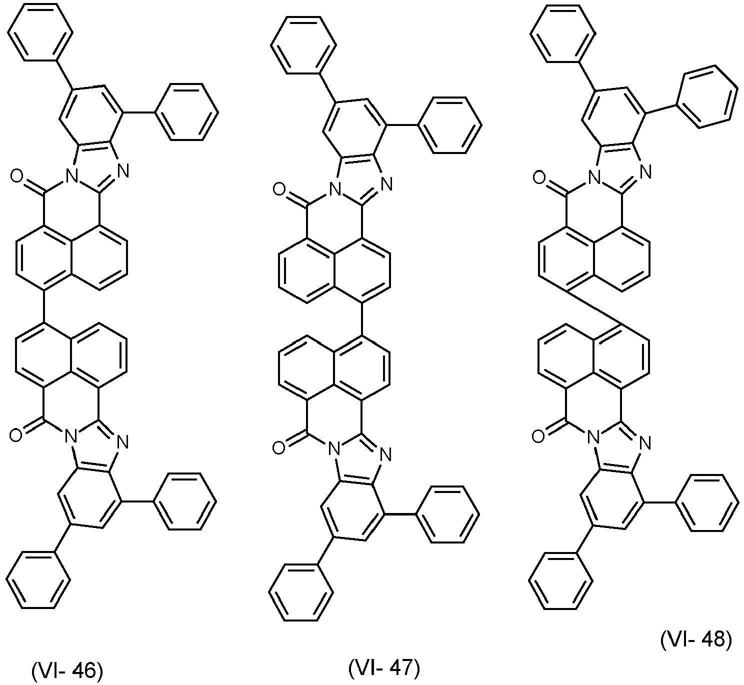 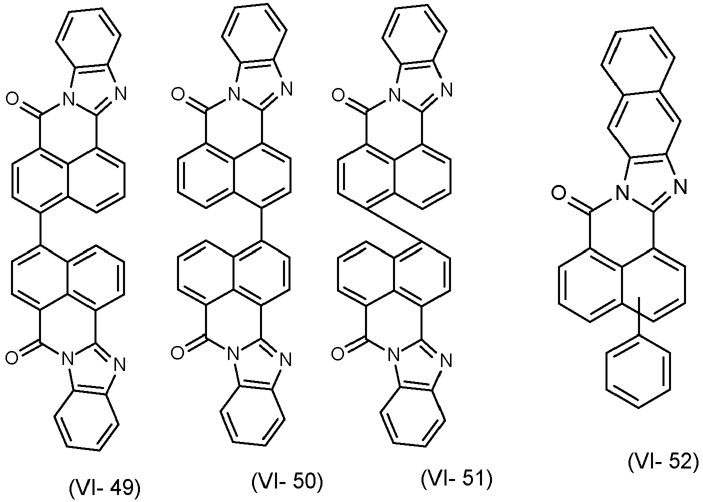 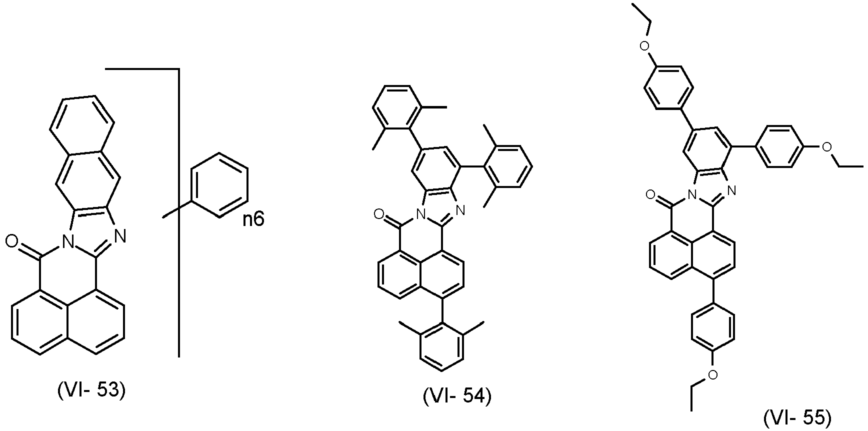 or mixtures thereof,
where n6 is a number from 0, 1, 2, 3, 4, 5, 6, 7, 8, 9 or 10;
- R⁶¹: is independently hydrogen, C₁-C₁₈-alkyl or cycloalkyl, the carbon chain of which may comprise one or more -O-, -S-, -CO-, -SO- and/or -SO₂- moieties and which may be mono- or polysubstituted;
aryl or heteroaryl which may be mono- or polysubstituted.

Compounds of formula (VI) are especially preferred for use in combination with the inventive red-fluorescent dye. Especially preferred are the compounds of formulae (VI-5), (VI-6), (VI-7) and (VI-8) and mixtures thereof.

Compounds of the formula (VI) are usually yellow or yellow-green fluorescent dyes.

The number of organic fluorescent dyes to be used in combination with the inventive red-fluorescent dye can be any number, preferably 1, 2, 3 or 4, more preferably 1 or 2.

In a preferred embodiment, inventive color converters comprise, as well as the at least one organic fluorescent dye present in accordance with the invention, at least one further organic fluorescent dye selected from compounds of formulae (II), (IV), and (VI). In particular, the at least one further organic fluorescent dye is a compound of formula (VI), especially one of the compounds of formulae (VI-1), (VI-2), (VI-3), (VI-4), (VI-5), (VI-6), (VI-7), (VI-8), (VI-9), (VI-10), (VI-11), (VI-12), (VI-13), (VI-14), (VI-15), (VI-16), (VI-17), (VI-18), (VI-19), (VI-20), (VI-21), (VI-22), (VI-23), (VI-24), (VI-25), (VI-26), (VI-27), (VI-28), (VI-29), (VI-30), (VI-31), (VI-32), (VI-33), (VI-34), (VI-35), (VI-36), (VI-37), (VI-38), (VI-39), (VI-40), (VI-41), (VI-42), (VI-43), (VI-44), (VI-45), (VI-46), (VI-47), (VI-48), (VI-49), (VI-50), (VI-51), (VI-52), (VI-53), (VI-54), (VI-55), and mixtures thereof. Among these, especially preferred are the compounds of formulae (VI-5), (VI-6), (VI-7), (VI-8) and mixtures thereof.

The red-fluorescent dye according to the invention used in combination with at least one further organic fluorescent dye, especially a yellow or green-yellow fluorescent dye, leads to excellent properties in terms of energetic and photometric conversion efficacy.

The concentration of the organic fluorescent dyes in the polymer is set as a function of the thickness of the color converter and the type of polymer. If a thin polymer layer is used, the concentration of the organic fluorescent dye(s) is generally higher than in the case of a thick polymer layer.

Typically, the concentration of inventive organic fluorescent dye of the formula I or mixtures thereof is 0.001 to 0.5% by weight, preferably 0.002 to 0.3% by weight, most preferably 0.003 to 0.1 % by weight, based in each case on the amount of polymer used. Typically, the concentration of the at least one further organic fluorescent dye is 0.001 to 0.5% by weight, preferably 0.002 to 0.3% by weight, based on the amount of the polymer used.

The ratio of at least one further organic fluorescent dye to the inventive red organic fluorescent dye is typically in the range from 4:1 to 25:1, preferably 5:1 to 20:1.

In a further preferred embodiment, the color converter according to the present invention may comprise inorganic fluorescent materials. According to this embodiment, the color converter is preferably used for conversion of light which has been produced by a blue diode, using at least one compound of formula I or a mixture thereof as a fluorescent dye in combination with at least one inorganic fluorescent colorant. In this embodiment, the blue LED and the color converter are in a remote phosphor arrangement.

The at least one inorganic fluorescent material is preferably selected from garnets, silicates, sulfides, nitrides and oxynitrides.

Particularly preferred among these are those selected from garnets, silicates, sulfides, nitrides and oxynitrides. Suitable examples of garnets, silicates, sulfides, nitrides and oxynitrides are compiled in table I below:

**Table I:**

| Class | Compounds | Excitation Peak nm | Emission Peak nm | Reference |
|---|---|---|---|---|
| Garnets | • YAG:Ce | 460-470 | 550 | US 5,998,925 |
| | • (Y₃Al₅O₁₂:Ce) | | | |
| | • (Y, Gd, Tb,Lu)₃Al₅O₁₂:Ce | | | |
| | • TAG:Ce (Tb₃Al₅O₁₂:Ce) | 460-470 | 575 | US 6,669,866, US 6,812,500, US 6,576,930, US6,0060,861, US 6,245,259, US 6,765,237 |
| Silicates | • Eu-doped Silicates | <460 | 510 to | US 7,311,858, US 7,267,787 |
| | - A₂Si(OD)₄:Eu wit A = Sr, Ba, Ca, Mg, Zn and D = F, Cl, S, N, Br | | 610 | |
| | - (SrBaCa)₂SiO₄:Eu | | | US 6,809,347, US 6,943,380 |
| | - Sr₃SiO₅ | | | |
| | - Ba₂MgSi₂O₇:Eu²⁺; | | | US 6,429,583 |
| | - Ba₂SiO₄:Eu²⁺ | | | WO 02/11214 |
| | - (Ca,Ce)₃(Sc,Mg)₂Si₃O₁₂ | | | |
| Sulfides | • (Ca, Sr)S:Eu | <460 | 615-660 | |
| Nitrides | • (CaAlSiN₃:Eu²) | 455 | red | |
| | • (Sr,Ca)AlSiN₃:Eu²⁺ | | orange | WO2005052087 |
| Oxynitrides | • SiAlON:Ce | 300-580 | 490 | |
| | • β-SiAlON:Eu | | 540 | |
| | • Ca-alpha-SiAlON:Eu (Ba₃Si₆O₁₂N₂:Eu) | | 585-595 | |
| | General formula Ca_{X}Eu_{y}(Si,Al)₁₂(O,N)₁₆ | | | |

According to a further embodiment, inventive color converters comprise, as well as the at least one organic fluorescent dye present in accordance with the invention, at least one further organic fluorescent dye selected from compounds of formulae (II), (III), (IV), (V), (VI), and mixtures thereof and at least one inorganic fluorescent material as defined above.

According to a further preferred embodiment, the color converter according to the invention comprises at least one quantum dot. Quantum dots are a nanocrystal of a semiconductor material having a diameter of about 20 nm or less. The quantum dot may include one of a Si-based nanocrystal, a group II-VI compound semiconductor nanocrystal, a group III-V compound semiconductor nanocrystal, a group IV-VI compound nanocrystal and a mixture thereof. The group II-VI compound semiconductor nanocrystal may include one selected from a group consisting of CdS, CdSe, CdTe, ZnS, ZnSe, ZnTe, HgS, HgSe, HgTe, CdSeS, CdSeTe, CdSTe, ZnSeS, ZnSeTe, ZnSTe, HgSeS, HgSeTe, HgSTe, CdZnS, CdZnSe, CdZnTe, CdHgS, CdHgSe, CdHgTe, HgZnS, HgZnSe, HggZnTe, CdZnSeS, CdZnSeTe, CdZnSTe, CdHgSeS, CdHgSeTe, CdHgSTe, HgZnSeS, HgZnSeTe and HgZnSTe. The group III-V compound semiconductor nanocrystal may include one selected from a group consisting of GaN, GaP, GaAs, AlN, AlP, AlAs, InN, InP, InAs, GaNP, GaNAs, GaPAs, AlNP, AlNAs, AlPAs, InNP, InNAs, InPAs, GaAlNP, GaAINAs, GaAlPAs, GalnNP, GalnNAs, GalnPAs, InAlNP, InAINAs, and InAIPAs. The IV-VI compound semiconductor nano crystal may be SbTe.

To synthesize a nanocrystal in form of a quantum dot, quantum dots may be prepared by vapor deposition such as metal organic chemical vapor deposition or molecular beam epitaxy, or by a wet chemical process in which a crystal is grown by adding one or more precursors into an organic solvent.

According to a further embodiment, inventive color converters comprise, as well as the at least one organic fluorescent dye present in accordance with the invention, at least one further organic fluorescent dye selected from compounds of formulae (II), (III), (IV), (V), (VI), and mixtures thereof and at least one quantum dot as defined above.

In a more preferred embodiment of the invention, the inventive color converter does not comprise quantum dots. Likewise, in a more preferred embodiment of the invention, the inventive color converter does not comprise inorganic fluorescent materials.

In one embodiment of the invention, inventive color converters have a laminate structure. They may either have a monolayer structure or a multilayer structure, generally composed of a plurality of polymer layers comprising one or more fluorescent dyes and/or scattering bodies. If the color converter has a multilayer structure, one layer comprises the red fluorescent dye according to the invention and another layer comprises at least one fluorescent dye encompassed by the present invention.

In one embodiment, the at least one red organic fluorescent dye is present in the layer of the color converter facing the LED. In another embodiment, the at least one further fluorescent dye is present in the layer of the color converter facing the LED.

According to a preferred embodiment, the color converter additionally comprises at least one inorganic white pigment as a scattering body.

In a preferred embodiment, at least one of the layers or matrices comprising organic fluorescent dye comprises scattering bodies for light.

Suitable scattering bodies are inorganic white pigments, for example titanium dioxide, barium sulphate, lithopone, zinc oxide, zinc sulphide, calcium carbonate with a mean particle size to DIN 13320 of 0.01 to 10 µm, preferably 0.1 to 1 µm, more preferably 0.15 to 0.4 µm, especially scattering bodies based on TiO₂.

Scattering bodies are included typically in an amount of 0.01 to 2.0% by weight, preferably 0.05 to 1 % by weight, more preferably 0.1 to 0.5% by weight, based in each case on the polymer of the layer comprising scattering bodies.

In a preferred embodiment, the color converter has a two-layer structure with a red-fluorescing layer and a green-yellow-fluorescing layer comprising at least one fluorescent dye present in accordance with the invention, with the red layer facing the blue light source. In this embodiment, both layers comprise TiO₂ as a scattering body.

In one embodiment, the color converters consist of a plurality of polymer layers which have been laminated together to form a composite and wherein the various fluorescent dyes/colorants and/or scattering bodies may be present in different polymer layers.

If inventive color converters comprise more than one fluorescent dyes/colorant, it is possible in one embodiment of the invention for a plurality of fluorescent dyes/colorants to be present alongside one another in one layer.

In another embodiment, the various fluorescent dyes/colorants are present in various layers.

In a preferred embodiment, inventive color converters comprise, as well as the at least one organic fluorescent dye present in accordance with the invention, at least one further organic fluorescent dye selected from compounds of formulae (II), (IV), and (VI) scattering bodies based on TiO₂ and at least one polymer consisting essentially of polystyrene, polyethylene terephthalate or polycarbonate.

In an even more preferred embodiment, inventive color converters comprise, as well as the at least one organic fluorescent dye present in accordance with the invention, at least one further organic fluorescent dye selected from compounds of formulae (II), (IV), and (VI), scattering bodies based on TiO₂ and at least one polymer consisting essentially of polyethylene terephthalate.

Likewise, in an even more preferred embodiment, inventive color converters comprise, as well as the at least one organic fluorescent dye present in accordance with the invention, at least one further organic fluorescent dye selected from compounds of formulae (II), (IV), and (VI), scattering bodies based on TiO₂ and at least one polymer consisting essentially of polycarbonate.

In a particularly preferred embodiment, inventive color converters comprise, as well as the at least one organic fluorescent dye present in accordance with the invention, at least one further organic fluorescent dye of formula (VI), scattering bodies based on TiO₂ and at least one polymer consisting essentially of polystyrene, polyethylene terephthalate or polycarbonate.

In an even more particularly preferred embodiment, inventive color converters comprise, as well as the at least one organic fluorescent dye present in accordance with the invention, at least one further organic fluorescent dye of formula (VI), scattering bodies based on TiO₂ and at least one polymer consisting essentially of polyethylene terephthalate.

Likewise, in a particularly preferred embodiment, inventive color converters comprise, as well as the at least one organic fluorescent dye present in accordance with the invention, at least one further organic fluorescent dye of formula (VI), scattering bodies based on TiO₂ and at least one polymer consisting essentially polycarbonate.

In one embodiment, at least one polymer layer of the color converter has been mechanically reinforced with glass fibers.

Inventive color converters may be in any desired geometric arrangement. The color converters may, for example, be in the form of films, sheets or plaques. Equally, the matrix containing organic fluorescent dyes may be in droplet form or hemispherical form or in the form of lenses with convex and/or concave, flat or spherical surfaces.

"Casting" refers to the embodiment where LEDs or components comprising LEDs are cast or enveloped fully with a polymer comprising organic fluorescent dye.

In one embodiment of the invention, the polymer layers (matrices) comprising organic fluorescent dye are 25 to 250 micrometers thick, preferably 35 to 200 µm and particularly 50 to 180 µm.

In another embodiment, the polymer layers comprising organic fluorescent dyes are 0.2 to 5 millimeters thick, preferably 0.3 to 3 mm and more preferably 0.4 to 1 mm.

If the color converters consist of one layer or they have a laminate structure, the individual layers, in a preferred embodiment, are continuous and do not have any holes or interruptions.

Inventive color converters may optionally comprise further constituents such as a backing layer.

Backing layers serve to impart mechanical stability to the color converter. The type of material for the backing layers is not crucial, provided that it is transparent and has the desired mechanical strength. Suitable materials for backing layers are, for example, glass or transparent rigid organic polymers such as polycarbonate, polystyrene or polymethacrylates or polymethylmethacrylates.

Backing layers generally have a thickness of 0.1 mm to 10 mm, preferably 0.2 mm to 5 mm, more preferably 0.3 mm to 2 mm.

In one embodiment of the invention, inventive color converters have at least one barrier layer against oxygen and/or water, as disclosed in WO 2012/152812. Examples of suitable barrier materials for barrier layers are, for example, glass, quartz, metal oxides, SiO₂, a multilayer system composed of alternating layers of Al₂O₃ and SiO₂ layers, titanium nitride, SiO₂/metal oxide multilayer materials, polyvinyl alcohol, polyacrylonitrile, polyvinylidene chloride (PVDC), liquid crystal polymers (LCP), polystyrene-acrylonitrile (SAN), polybutylene terephthalate (PBT), polybutylene naphthalate (PBN), polyethylene terephthalate (PET), polyethylene naphthalate (PEN), polyvinyl butyrate (PBT), polyvinyl chloride (PVC), polyamides, polyoxymethylenes, polyimides, polyetherimides, epoxy resins, polymers which derive from ethylene-vinyl acetate (EVA) and polymers which derive from ethylene-vinyl alcohol (EVOH).

A preferred material for barrier layers is glass or a multilayer system composed of alternating layers of Al₂O₃ and SiO₂ layers.

Preferably, suitable barrier layers have low permeability for oxygen.

More preferably, suitable barrier layers have low permeability for oxygen and water.

Inventive color converters are especially suitable for the conversion of blue light to white light.

More particularly, they are suitable for conversion of light emitted by blue LEDs. Suitable LEDs are, for example, those based on gallium nitride (GaN) or indium gallium nitride (InGaN). Likewise possible is use for conversion of light produced by mercury lamps, by organic light-emitting diodes (OLEDs) or by UV LEDs.

According to a further embodiment, they are suitable for conversion of light emitted by green LEDs. Suitable LEDs are, for example, those based on GalnNAs, such as Te-doped GalnNAs and Mg-doped GalnNAs. Particularly, they are suitable for conversion of light emitted by white LEDs, especially cold-white light of LEDs, into pleasant light with natural-white color temperature or warm-white color temperature with high conversion efficacy.

They are additionally suitable for applications as a light-collecting system (fluorescence collector) in photovoltaics and in fluorescence conversion solar cells.

In a further embodiment, the inventive color converters are used for the conversion of blue light.

Inventive color converters on irradiation with light, especially with blue LED light, exhibit a high quantum yield and overall energy efficiency. In addition, they have a high photostability on illumination with blue light. Moreover, they are stable toward oxygen and water.

Inventive color converters can be produced by different processes.

In one embodiment, the process for producing inventive color converters comprises the dissolution of the at least one polymer and the at least one organic fluorescent dye and, if present, further organic fluorescent dyes, in a solvent and subsequent removal of the solvent.

In another embodiment, the process for producing inventive color converters comprises the extrusion of the at least one organic fluorescent dye and, if present, further organic fluorescent dyes with the at least one polymer.

The invention further provides lighting devices comprising at least one LED and at least one color converter according to the invention. The at least one LED is preferably blue and emits light preferably within a wavelength range from 400 to 500 nm, preferably 420 to 480 nm, most preferably 430 to 470 nm.

In one embodiment, inventive lighting devices comprise exactly one LED. In another embodiment, inventive lighting devices comprise two or more LEDs.

In one embodiment, inventive lighting devices comprise a plurality of LEDs, all of which are blue.

Furthermore, the type of LED used is not crucial for the inventive lighting devices. In a preferred embodiment, the power density of the blue LED light impinging the surface of the converter plate is usually less than 200 mW/cm², preferably less than 120 mW/cm², more preferably less than 80 mW/cm². The use of LEDs of higher power densities, such as 150 or 200 mW/cm², is likewise possible. However, a higher power density of the LED at the converter surface can reduce the lifetime of the fluorescent dyes and the color converters.

Inventive color converters can be used in combination with LEDs in virtually any geometric form and irrespective of the construction of the lighting device.

In one embodiment, color converter and LED are in a phosphor on a chip arrangement.

Preferably, inventive color converters are used in a remote phosphor setup. In this case, the color converter is spatially separated from the LED. In general, the distance between LED and color converter is from 0.1 cm to 50 cm, preferably 0.2 to 10 cm and most preferably 0.5 to 3 cm. Between color converter and LED may be different media such as air, noble gases, nitrogen or other gases or mixtures thereof.

The color converter may, for example, be arranged concentrically around the LED or have a planar geometry. It may take the form, for example, of a plaque, sheet or film, be in droplet form or take the form of a casting.

Inventive lighting devices are suitable for lighting in interiors, outdoors, of offices, of vehicles, in torches, games consoles, streetlights, traffic signs.

Inventive lighting devices exhibit a high quantum yield and high energetic and photometric efficacy. In addition, they have a long lifetime, especially a high photostability on illumination with blue light.

The present invention further provides a device producing electric power upon illumination comprising a photovoltaic cell (solar cell) and the color converter as defined above, where at least a part of the light not absorbed by the photovoltaic cell (solar cell) is absorbed by the color converter. The color converter is usually on top of the photovoltaic cell. The color converter is used to modify the spectrum such that UV and visible light are converted to a more bathochromic spectrum that is converted at higher efficiency by the solar cell.

### Examples

### Spectroscopic measurements

The fluorescence spectra and fluorescence quantum yields were recorded using an absolute quantum yield measurement instrument (C9920-02) manufactured by Hamamatsu, and, if necessary, equipped with cuvettes for liquid samples.

Example 1: Compound C1 (compound of the formula (I), where x = y = 0; Ar1 = Ar2 = 2,6-diisopropylphenyl; R³ = R⁴ = R⁵ = R⁶ = (A)_{z}-Ar3-X, where z = 1, X is O, Ar3 is phenyl which carries a group A, where R¹ = R² = n-hexyl)

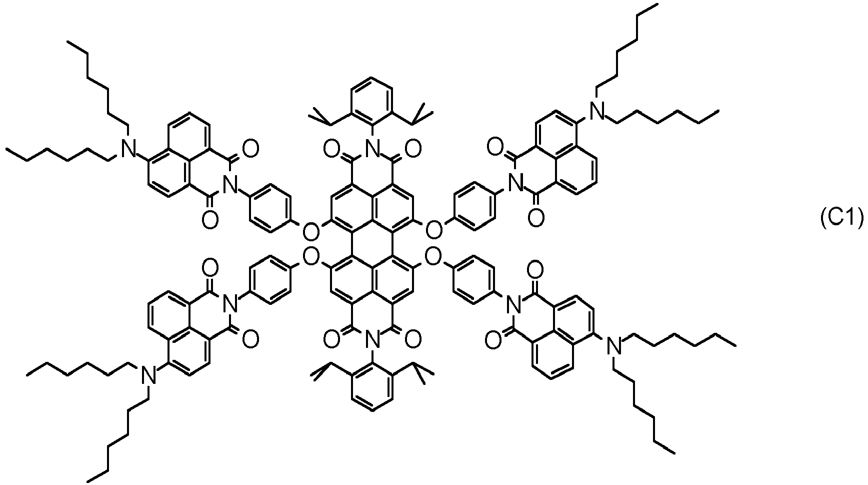

### 1.1 N-(4'-hydroxyphenyl)-4-chloronaphtalene-1,8-biscarboximide

4-Chloro-1,8-naphtalic anhydride (15 g, 1 eq, 65 mmol), 4-amino-phenol (20 g, 2.8 eq, 184 mmol) and acetic acid (500 mL) were introduced in a 1L-3-necks flask and stirred under argon at 95°C for 4. 5 hours. The mixture was cooled down to room temperature and was acidified with 20 mL of HCl (10% aqueous), filtered. The residue was washed with 100 mL of HCl (10% aqueous) and dried under vacuum at 75°C to get the title compound as a yellow solid (17.74 g, 85%). Purity (HPLC-MS 254 nm and NOR ¹³C and ¹H) > 95%. R_{f} (cyclohexane/ethyl acetate (7:3)) = 0.20.
¹H-NMR (500Mz, CDCl₃): 5.57 (1H, OH, s), 6.96 (2H, ar phenol, d, J=8.70Hz), 7.13 (2H, ar phenol, d, J=8,8Hz), 7.87-7.90 (2H, m), 8.49 (1H, d, J=8,00Hz), 8.64 (1H, d, J=2,10Hz), 8.66 (1H, d, J=3.75Hz).

### 1.2 N-(4'-hydroxyphenyl)-4-dihexylaminonaphtalene-1,8-biscarboximide

N-(4'-Hydroxyphenyl)-4-chloronaphtalene-1,8-biscarboximide (1.0 g, 1 eq, 3.1 mmol), dihexylamine (16 g, 28 eq, 86 mmol) and N-methyl-2-pyrrolidone (20 mL) were introduced in a 100 mL three necks flask, stirred at 200°C under argon for 4 hours. The mixture was cooled down to room temperature and then poured into 50 mL of H₂SO₄ (10% aqueous) stirred, filtered and washed with distilled water. A gelly mixture was obtained. Purification with silica was carried out (with cyclohexane/ethyl acetate (8:2)). Fractions were combined, evaporated under reduced pressure and then dried under vacuum at 75°C to afford the title compound as a yellow solid (0.91 g, 62%). Purity (HPLC-MS 254 nm and NMR ¹H) > 95%. Mp = 168.1°C. R_{f} (cyclohexane/ethyl acetate (7:3)) = 0.38.
¹H-NMR (400Mz, CDCl₃, δ(ppm)): 0.85 (6H, 2*CH₃ hexyl, t, J=7.10Hz), 1.24-1.28 (12H, 2*C₃H₆ hexyl, m), 1.60-1.67 (4H, 2*CH₂ hexyl, m), 3.41 (4H, 2*N-CH₂, t, J = 7.87 Hz), 5.80 (1H, OH, s), 6.89 (2H, ortho phenol, d, J= 9.08Hz), 7.10 (2H, meta phenol, d, J = 9.04Hz), 7.24 (1H, d, J = 8.56Hz), 7.69 (1H, dd, J = 7.68Hz and J=7.24Hz), 8.47 (1H, d, J= 8.20Hz, 8.51 (1H, d, J= 8.64Hz), 8.57 (1H, d, J=7.6Hz).

### 1.3 Compound C1

N,N'-Bis(2,6-diisopropylbenzene)-1,6,7,12-tetra-chloro-3,4;9,10-tetracarboxybisimide (prepared as described in WO 2007/006717; 77 mg, 1 eq, 0.09 mmol), the compound from example 1.2 (200 mg, 4.2 eq, 0.36 mmol), K₂CO₃ (120 mg mg, 9.6 eq, 0.87 mmol) and N-methyl-2-pyrrolidone (10 mL) were introduced in a three-necks 25 mL flask and stirred under argon at room temperature for 21 hours, then the mixture was heated up to 80°C and stirred at 80°C for 2 hours, followed by stirring at 110°C for 20 hours and at 200°C for 4 hours. The reaction mixture was poured into 15 mL of H₂SO₄ (2% aqueous), stirred for 5 min and filtered. The residue was washed with 10 mL of distilled water and dried under vacuum oven at 75°C, followed by purification with silica (dichloromethane). Fractions were combined, evaporated under reduced pressure and dried under vacuum at 75°C to afford the title compound as a red solid (58 mg, 25%). Purity (HPLC-MS 254 nm and NMR ¹³C and ¹H) > 95%. R_{f} (cyclohexane/ethyl acetate (8:2)) = 0.33.
¹H-NMR (500Mz, CDCl₃): 0.81 (24H, t, J=7.3Hz), 1.13-1.15 (64H, m), 1.21-1.25 (16H, m), 2.76-2.80 (4H, m, J=6.95Hz), 3.36 (16H, t, J=7.3Hz), 7.11 (8H, d, J=9.50Hz), 7.18 (4H, d, J=8.25Hz), 7.32 (8H, d, J=8.2Hz), 7.35 (4H, d, J=7.6Hz), 7.50 (2H, t, J=7.10Hz), 7.61 (4H, t, J=8.25Hz), 8.41 (4H, d, J=8.2Hz), 8.45 (4H, d, J=8.85Hz), 8.49 (4H, d, J=7.6Hz), 8.53 (4H, s).

### Toluene solution:

Absorption: λₘₐₓ = 440 nm, εₘₐₓ 26 L/g·cm
Emission: λₘₐₓ = 605 nm, 653 nm,

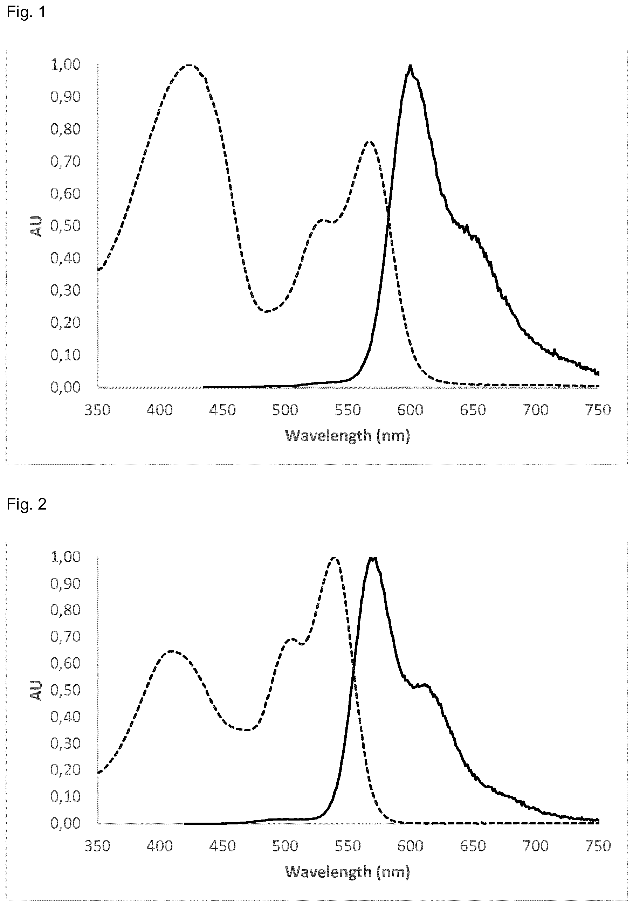

Figure 1 shows the absorption spectrum (broken line) and emission spectrum (solid line; excitation at 440 nm) of the compound C1 in toluene (c = 10⁻⁴ mol/L).

Example 2: Compound C2 (compound of the formula (I), where x = y = 0; Ar1 = Ar2 = 2,6-diisopropylphenyl; R³ = R⁵ = H; R⁴ = R⁶ = (A)_{z}-Ar3-X, where z = 1, X is O, Ar3 is phenyl which carries a group A, where R¹ = R² = n-hexyl)

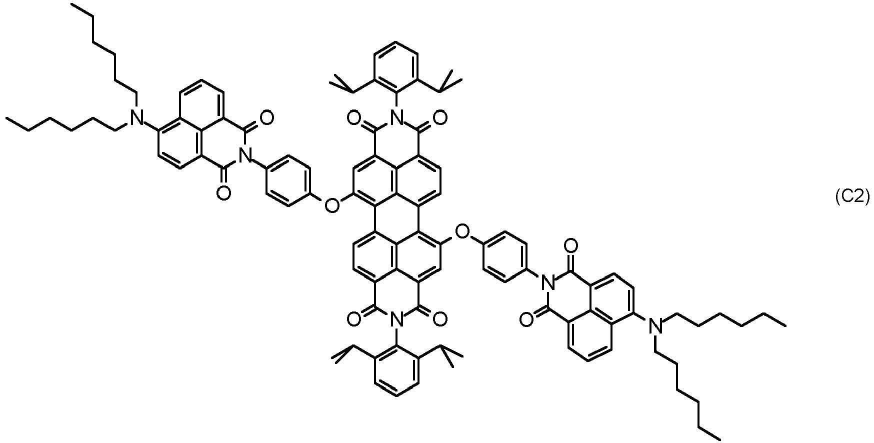

N,N'-Bis(2,6-diisopropylbenzene)-1,7-di-bromo-3,4;9,10-tetracarboxybisimide (prepared according to DE 19547210; 165 mg, 1 eq, 0.19 mmol), compound from example 1.2 (200 mg, 2 eq, 0.36 mmol), N-methyl-2-pyrrolidone (5 mL) and K₂CO₃ (117 mg, 4.4 eq, 0.85 mmol) were introduced in a three-necks 50 mL flask and stirred under argon at room temperature for 4.75 hours. The reaction mixture was poured into 10 mL of H₂SO₄ (2% aqueous), stirred for 10 min and filtered. The residue was washed with 5 mL of distilled water and dried under vacuum oven at 75°C. Purification with silica (0.040-0.063 nm, 20 g, dichloromethane) and further drying under vacuum at 75°C afforded the title compound as a red solid (220 mg, 70%). Purity (HPLC-MS 254 nm and NMR ¹³C and ¹H) > 95%. Mp = 406.2°C. R_{f} (cyclohexane/ethyl acetate (8:2)) = 0.25.
¹H-NMR (400Mz, CDCl₃, δ(ppm)): 0.83 (12H, 4*CH₃ hexyl, t, J=7.60Hz), 1.13-1.16 (32H, 16*CH₂, m), 1.21-1.26 (8H, 4*N-CH₂, m), 3.37-3.41 (6H, aromatic 6*CH diisopropylbenzene, m), 7.22-7.37 (16H, phenoxy, m), 7.51 (2H, 2*CH naphthalene, d, J=7.32Hz), 7.67 (2H, 2*CH naphthalene, d, J=7.96Hz), 8.45 (2H, 2*CH naphthalene, d, J=8.60Hz), 8.49 (2H, 2*CH naphthalene, d, J=7.86Hz), 8.54 (2H, 2*CH naphthalene, d, J=7.36Hz), 8.58 (2H, 2*CH perylene, s), 8.75 (2H, 2*CH perylene, d, J= 8.64Hz), 9.75 (2H, 2*CH perylene, d, J=8.92Hz).

### Toluene solution:

Absorption: λₘₐₓ = 430 nm, εₘₐₓ 41 L /g·cm
Emission: λₘₐₓ = 572 nm and 618 nm.

Figure 2 shows the absorption spectrum (broken line) and emission spectrum (solid line; excitation at 408 nm) of the compound C2 in toluene (c = 10⁻⁴ mol/L).

### Materials used

Polymer 1: transparent polycarbonate based on a polycondensate of bisphenol A and phosgene (Makrolon® 2805 from Bayer MaterialScience AG)

Polymer 2: transparent polystyrene based on a homopolymer of styrene with a density of 1048 kg/m3 and a Vicat softening temperature of 98°C to DIN EN ISO 306 (PS 168 N from BASF SE)

Polymer 3: transparent polyethylene terephthalate PET Terez® 3200, from TER Plastics

Titanium dioxide: TiO₂ rutile pigment; in the case of polystyrene films: Kronos® 2220; and in the case of polycarbonate films: Kronos® 2233 - both from Kronos Titan.

Production of polymer films of the dyes as color converters:
Approximately 2.5 g of polymer (PC or PS) and 0.02% or 0.04% by weight of dye were dissolved in approximately 6 mL of dichloromethane and, in relevant case, 0.5% by weight of TiO₂ were dispersed therein, based in each case on the amount of polymer used. The resulting solution/dispersion was coated onto glass surface with a box-type coating bar (wet film thickness 400 µm). After the solvent had dried off, the film was detached from the glass and dried at 50°C in a vacuum drying cabinet overnight. A circular film piece with a diameter of 15 mm was punched out of the film of thickness 80-85 µm, and then served as test sample.

Production of solutions of the dyes:
Approximately 2 mmol of dye were dissolved in 25 mL of solvent (dichloromethane or toluene). The resulting solution was diluted 80 times in order to obtain a concentration of *ca.* 10⁻⁴ mol/L. The resulting solution was used for testing fluorescence quantum yield.

The results for the fluorescence quantum yield measurement of C1 and C2 in PC (polycarbonate), PS (polystyrene) and toluene (excitation 510 nm) are shown in table II.

**Table II:**

| Compound | PC | | | PS | | | Toluene |
|---|---|---|---|---|---|---|---|
| | 0.02% | 0.04% | 0.02% + 0.5% TiO₂ | 0.02% | 0.04% | 0.02% + 0.5% TiO₂ | 10⁻⁴ mol/L |
| C1 | 85% | 84% | 91% | 87% | 86% | 93% | 95% |
| C2 | 79% | 79% | 83% | 90% | 91% | 95% | 91% |

In Table 2, in case of polymer films of the dyes, the amounts of red fluorescent dye C1, red-fluorescent dye C2 and TiO₂ are based on the amount of polymer (either polycarbonate (PC) or polystyrene (PS) used).

## Claims

1. 3,4;9,10-Perylenetetracarboxylic diimide of the formula I 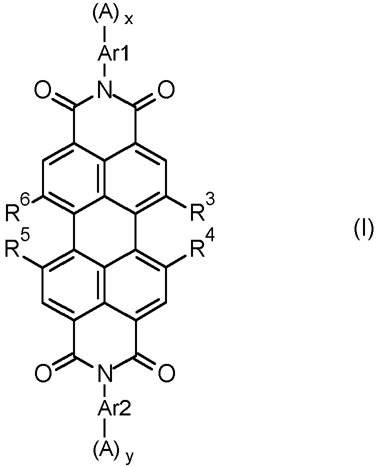 wherein the compound of the formula (I) comprises at least one group A
wherein
each group A is independently selected from a group of the following formula 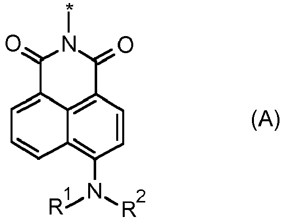 wherein
* denotes the bonding site to the remainder of the molecule;
R¹ and R², independently of each other, are selected from hydrogen, C₁-C₃₀-alkyl, C₂-C₃₀-alkenyl, C₂-C₃₀-alkynyl, C₃-C₁₂-cycloalkyl and C₆-C₂₄-aryl wherein the two last-mentioned radicals are unsubstituted or carry one, two or three radicals selected from C₁-C₁₀-alkyl;
x is 0 or 1;
y is 0 or 1;
Ar1 is C₆-C₂₄-aryl which does not bear a substituent R^{Ar1} or bears 1, 2 or 3 identical or different substituent(s) R^{Ar1}, where each R^{Ar1}, independently of each other, is selected from C₁-C₃₀-alkyl, C₁-C₃₀-haloalkyl, C₁-C₃₀-alkoxy, C₁-C₃₀-alkylsulfanyl, C₂-C₃₀-alkenyl and C₂-C₃₀-alkynyl;
Ar2 is C₆-C₂₄-aryl which does not bear a substituent R^{Ar2} or bears 1, 2 or 3 identical or different substituent(s) R^{Ar2}, where each R^{Ar2}, independently of each other, is selected from C₁-C₃₀-alkyl, C₁-C₃₀-haloalkyl, C₁-C₃₀-alkoxy, C₁-C₃₀-alkylsulfanyl, C₂-C₃₀-alkenyl and C₂-C₃₀-alkynyl;
R³, R⁴, R⁵ and R⁶, independently of each other, are selected from hydrogen and a group of formula
(A)_{z}-Ar3-X-#,
wherein
# denotes the bonding site to the remainder of the molecule;
z is 0 or 1;
X is O or S;
Ar3 C₆-C₂₄-aryl which does not bear a substituent R^{Ar3} or bears 1, 2 or 3 identical or different substituent(s) R^{Ar3}, wherein each R^{Ar3}, independently of each other, is selected from C₁-C₃₀-alkyl, C₁-C₃₀-haloalkyl, C₁-C₃₀-alkoxy, C₁-C₃₀-alkyl-sulfanyl, C₂-C₃₀-alkenyl, C₃-C₁₂-cycloalkynyl, C3-C12-cycloalku=yL and C₆-c₂₄-aryl.

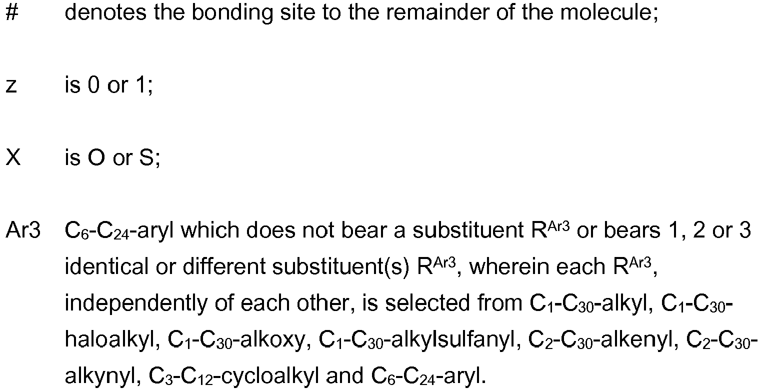

2. 3,4;9,10-Perylenetetracarboxylic diimide of the formula I according to claim 1, where R¹ and R², independently of each other, are selected from C₁-C₂₀-alkyl.

3. 3,4;9,10-Perylenetetracarboxylic diimide of the formula I according to claim 1 or 2, where
Ar1 is selected from phenyl which carries 1 or 2 C₁-C₆-alkyl substituents R^{Ar 1} and phenyl which carries one group A; and
Ar2 is selected from phenyl which carries 1 or 2 C₁-C₆-alkyl substituents R^{Ar 2} and phenyl which carries one group A.

4. 3,4;9,10-Perylenetetracarboxylic diimide of the formula I according to any of the preceding claims, where R³, R⁴, R⁵ and R⁶, independently of each other, are selected from hydrogen, phenoxy, which is not substituted and phenoxy which carries one group A.

5. 3,4;9,10-Perylenetetracarboxylic diimide of the formula I according to any of the preceding claims, wherein two of the radicals R³, R⁴, R⁵ and R⁶ are hydrogen and the other radicals R³, R⁴, R⁵ and R⁶ are selected from phenoxy, which is not substituted and phenoxy which carries one group A.

6. 3,4;9,10-Perylenetetracarboxylic diimide of the formula I according to any of claims 1 to 4, wherein the radicals R³, R⁴, R⁵ and R⁶ have the same meaning.

7. 3,4;9,10-Perylenetetracarboxylic diimide of the formula I according to any of the preceding claims selected from

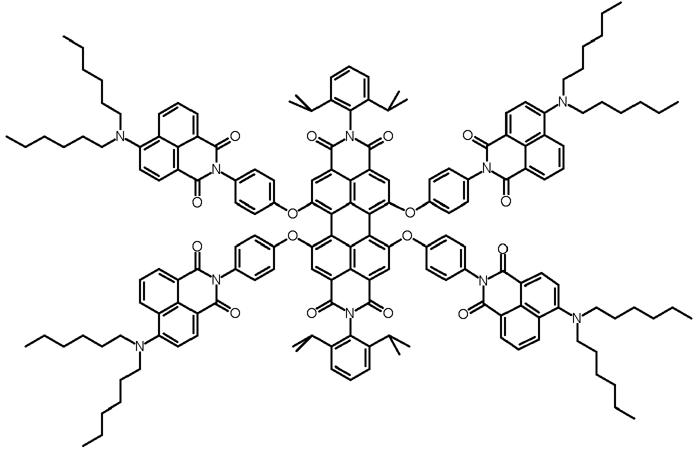

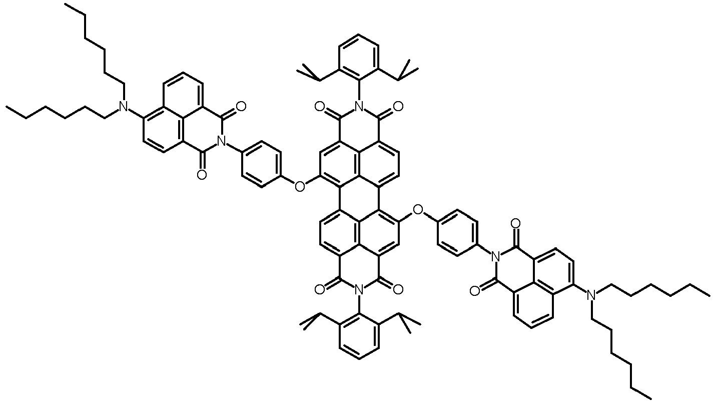

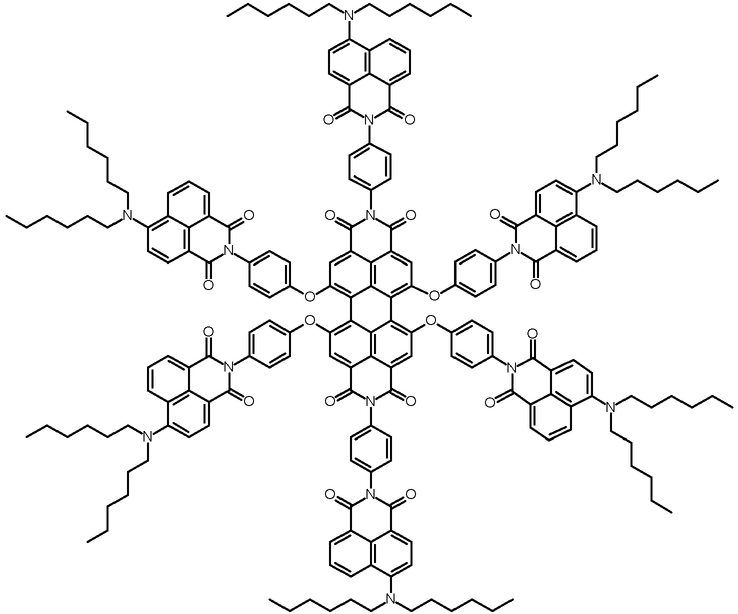

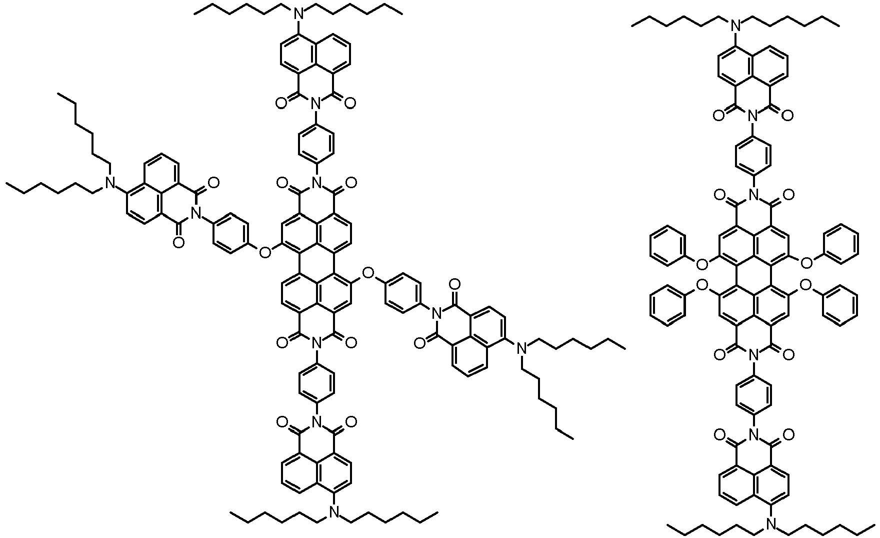

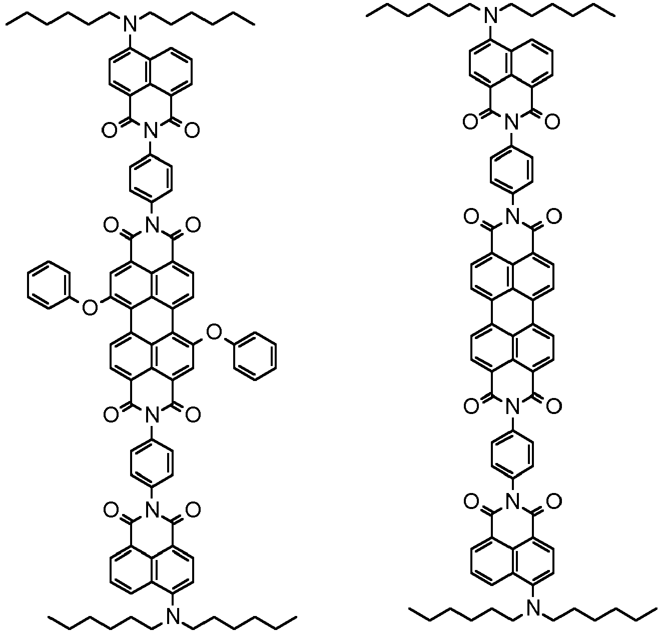

8. A color converter comprising at least one polymer as a matrix and at least one compound of the formula I or a mixture thereof as defined in any of claims 1 to 7 as a fluorescent dye, wherein the at least one polymer is selected from polystyrene, polycarbonate, polymethylmethacrylate, polyvinylpyrrolidone, polymethacrylate, polyvinyl acetate, polyvinyl chloride, polybutene, silicone, polyacrylate, epoxy resin, polyvinyl alcohol, poly(ethylene vinylalcohol)-coplymer (EVA, EVOH), polyacrylonitrile, polyvinylidene chloride (PVDC), polystyreneacrylonitrile (SAN), polybutylene terephthalate (PBT), polyethylene terephthalate (PET), polyvinyl butyrate (PVB), polyvinyl chloride (PVC), polyamides, polyoxymethylenes, polyimides, polyetherimides and mixtures thereof, in particular the at least one polymer consists essentially of polystyrene, polycarbonate, or polyethylene terephthalate.

9. The color converter according to claim 8, wherein the color converter additionally comprises at least one inorganic white pigment as a scattering body.

10. The color converter according to any of claims 8 or 9, comprising at least a further fluorescent material selected from inorganic fluorescent material and further organic fluorescent dyes.

11. The use of a color converter as defined in any of claims 8 to 10, for conversion of light generated by LEDs or OLEDs, in particular selected from a blue LED or a white LED.

12. The use of a color converter as defined in any of claims 8 to 11 in displays.

13. A lighting device comprising at least one LED and at least one color converter according to any of claims 7 to 9, the LED and color converter being preferably in a remote phosphor arrangement.

14. A device producing electric power upon illumination comprising a photovoltaic cell and the color converter as defined in any of claims 8 to 11, where at least a part of the light not absorbed by the photovoltaic cell is absorbed by the color converter.

15. The use of a compound of the formula I or a mixture thereof as defined in any of claims 1 to 7, in color converters for converting light emitted from a light source, in particular a light source selected from LEDs and OLEDs, into light of a second, longer wavelength, for coloring coatings, printing inks and plastics, producing aqueous polymer dispersions which absorb and/or emit electromagnetic radiation, for data storage, for optical labels, for security labels in documents and for brand protection or as a fluorescent label for biomolecules.

## Patentansprüche

1. 3,4;9,10-Perylentetracarbonsäurediimid der Formel I 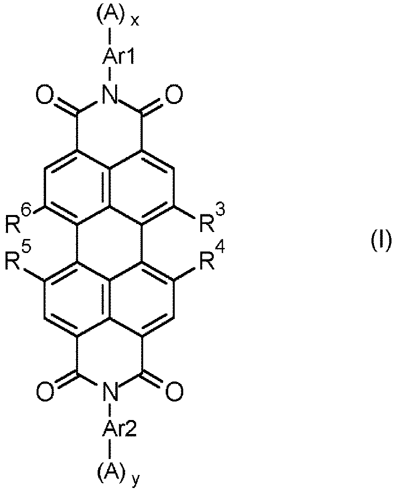 wobei die Verbindung der Formel (I) mindestens eine Gruppe A umfasst,
wobei
jede Gruppe A unabhängig aus einer Gruppe der folgenden Formel 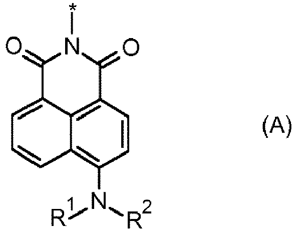 ausgewählt ist, in der
* die Bindungsstelle zum Rest des Moleküls anzeigt;
R¹ und R² unabhängig voneinander aus Wasserstoff, C₁-C₃₀-Alkyl, C₂-C₃₀-Alkenyl, C₂-C₃₀-Alkinyl, C₃-C₁₂-Cycloalkyl und C₆-C₂₄-Aryl ausgewählt sind, wobei die zwei letztgenannten Reste unsubstituiert sind oder einen, zwei oder drei aus C₁-C₁₀-Alkyl ausgewählte Reste tragen;
x für 0 oder 1 steht;
y für 0 oder 1 steht;
Ar1 für C₆-C₂₄-Aryl, das keinen Substituenten R^{Ar1} trägt oder 1, 2 oder 3 gleiche oder verschiedene Substituenten R^{Ar1} trägt, wobei R^{Ar1} jeweils unabhängig voneinander aus C₁-C₃₀-Alkyl, C₁-C₃₀-Halogenalkyl, C₁-C₃₀-Alkoxy, C₁-C₃₀-Alkyl-sulfanyl, C₂-C₃₀-Alkenyl und C₂-C₃₀-Alkinyl ausgewählt ist;
Ar2 für C₆-C₂₄-Aryl, das keinen Substituenten R^{Ar2} trägt oder 1, 2 oder 3 gleiche oder verschiedene Substituenten R^{Ar2} trägt, wobei R^{Ar2} jeweils unabhängig voneinander aus C₁-C₃₀-Alkyl, C₁-C₃₀-Halogenalkyl, C₁-C₃₀-Alkoxy, C₁-C₃₀-Alkyl-sulfanyl, C₂-C₃₀-Alkenyl und C₂-C₃₀-Alkinyl ausgewählt ist;
R³, R⁴, R⁵ und R⁶ unabhängig voneinander aus Wasserstoff und einer Gruppe der Formel (A)_{z}-Ar3-X-# ausgewählt sind,
wobei
# die Bindungsstelle zum Rest des Moleküls anzeigt;
z für 0 oder 1 steht;
X für 0 oder S steht;
Ar3 für C₆-C₂₄-Aryl, das keinen Substituenten R^{Ar3} trägt oder 1, 2 oder 3 gleiche oder verschiedene Substituenten R^{Ar3} trägt, wobei R^{Ar3} jeweils unabhängig voneinander aus C₁-C₃₀-Alkyl, C₁-C₃₀-Halogenalkyl, C₁-C₃₀-Alkoxy, C₁-C₃₀-Alkylsulfanyl, C₂-C₃₀-Alkenyl, C₂-C₃₀-Alkinyl, C₃-C₁₂-Cycloalkyl und C₆-C₂₄-Aryl ausgewählt ist.

2. 3,4;9,10-Perylentetracarbonsäurediimid der Formel I nach Anspruch 1, wobei R¹ und R² unabhängig voneinander aus C₁-C₂₀-Alkyl ausgewählt sind.

3. 3,4;9,10-Perylentetracarbonsäurediimid der Formel I nach Anspruch 1 oder 2, wobei
Ar1 aus Phenyl, das 1 oder 2 C₁-C₆-Alkylsubstituenten R^{Ar1} trägt, und Phenyl, das eine Gruppe A trägt, ausgewählt ist; und
Ar2 aus Phenyl, das 1 oder 2 C₁-C₆-Alkylsubstituenten R^{Ar2} trägt, und Phenyl, das eine Gruppe A trägt, ausgewählt ist.

4. 3,4;9,10-Perylentetracarbonsäurediimid der Formel I nach einem der vorhergehenden Ansprüche, wobei R³, R⁴, R⁵ und R⁶ unabhängig voneinander aus Wasserstoff, Phenoxy, das unsubstituiert ist, und Phenoxy, das eine Gruppe A trägt, ausgewählt sind.

5. 3,4;9,10-Perylentetracarbonsäurediimid der Formel I nach einem der vorhergehenden Ansprüche, wobei zwei der Reste R³, R⁴, R⁵ und R⁶ für Wasserstoff stehen und die anderen Reste R³, R⁴, R⁵ und R⁶ aus Phenoxy, das unsubstituiert ist, und Phenoxy, das eine Gruppe A trägt, ausgewählt sind.

6. 3,4;9,10-Perylentetracarbonsäurediimid der Formel I nach einem der Ansprüche 1 bis 4, wobei die Reste R³, R⁴, R⁵ und R⁶ die gleiche Bedeutung haben.

7. 3,4;9,10-Perylentetracarbonsäurediimid der Formel I nach einem der vorhergehenden Ansprüche, ausgewählt aus

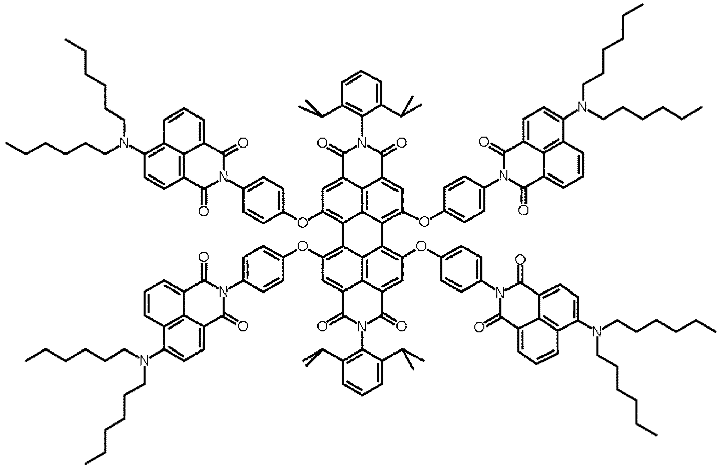

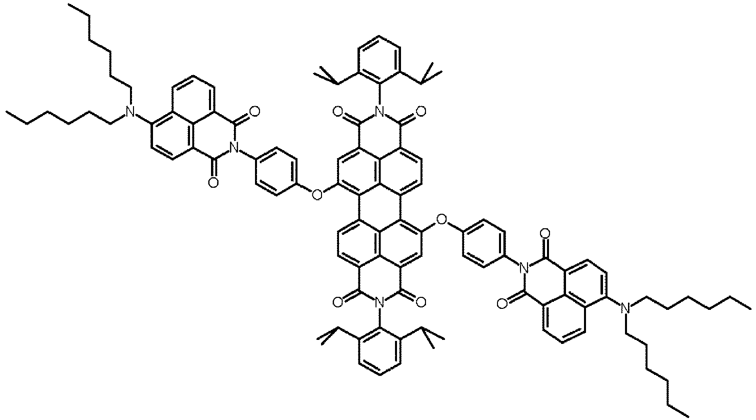

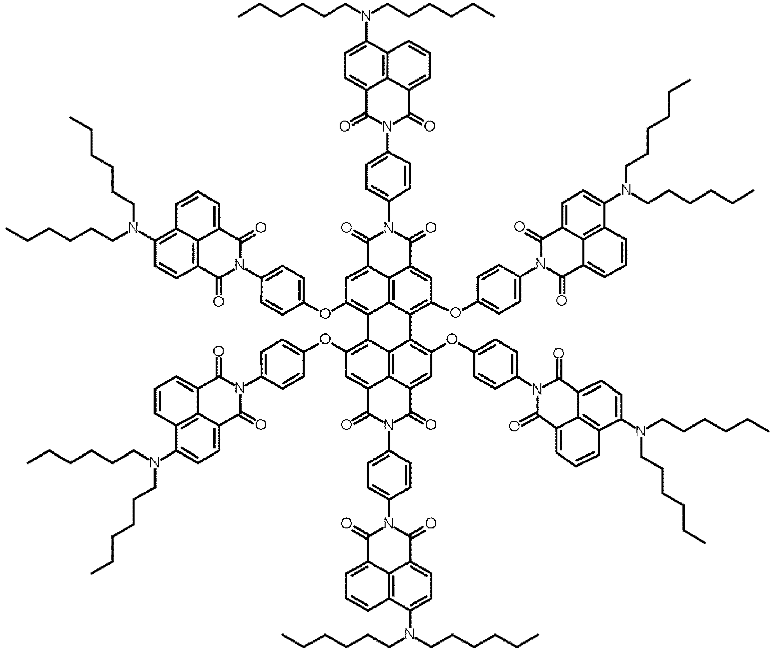

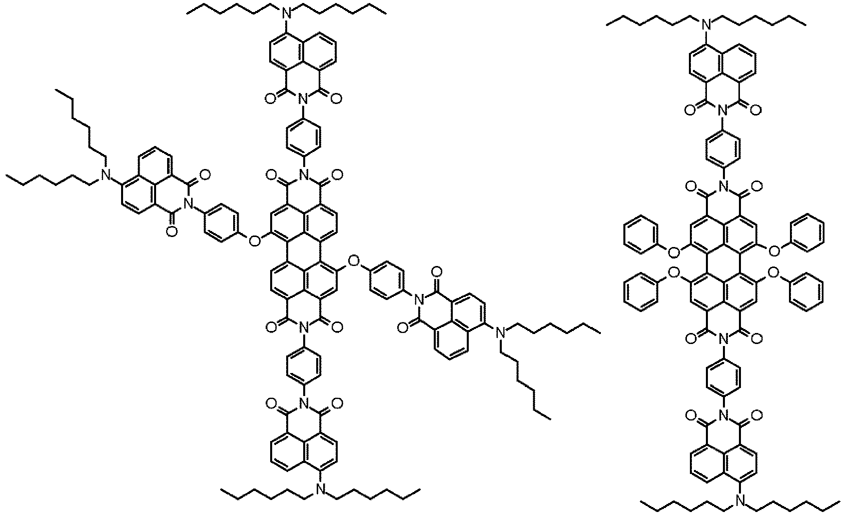

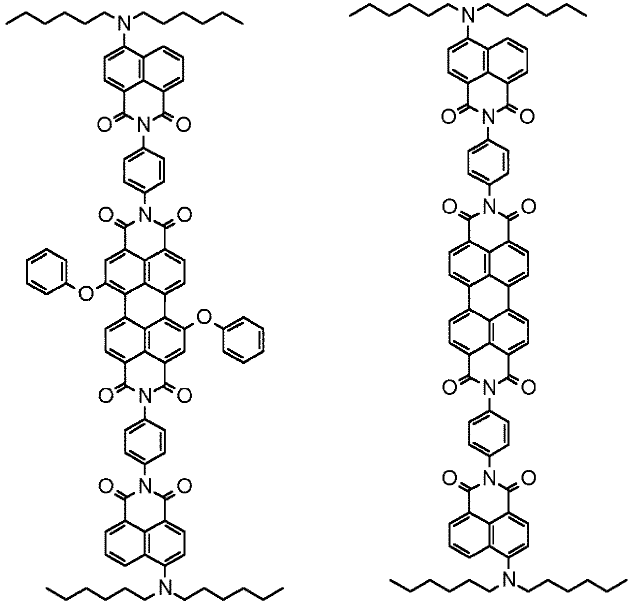

8. Farbkonverter, umfassend mindestens ein Polymer als Matrix und mindestens eine Verbindung der Formel I oder eine Mischung davon gemäß einem der Ansprüche 1 bis 7 als Fluoreszenzfarbstoff, wobei das mindestens eine Polymer aus Polystyrol, Polycarbonat, Polymethylmethacrylat, Polyvinylpyrrolidon, Polymethacrylat, Polyvinylacetat, Polyvinylchlorid, Polybuten, Silikon, Polyacrylat, Epoxidharz, Polyvinylalkohol, Poly(ethylenvinylalkohol)-Copolymer (EVA, EVOH), Polyacrylnitril, Polyvinylidenchlorid (PVDC), Polystyrolacrylnitril (SAN), Polybutylenterephthalat (PBT), Polyethylenterephthalat (PET), Polyvinylbutyrat (PVB), Polyvinylchlorid (PVC), Polyamiden, Polyoxymethylenen, Polyimiden, Polyetherimiden und Mischungen davon ausgewählt ist, insbesondere das mindestens eine Polymer im Wesentlichen aus Polystyrol, Polycarbonat oder Polyethylenterephthalat besteht.

9. Farbkonverter nach Anspruch 8, wobei der Farbkonverter zusätzlich mindestens ein anorganisches Weißpigment als Streukörper umfasst.

10. Farbkonverter nach Anspruch 8 oder 9, umfassend mindestens ein weiteres fluoreszierendes Material, das aus einem anorganischen fluoreszierenden Material und weiteren organischen Fluoreszenzfarbstoffen ausgewählt ist.

11. Verwendung eines Farbkonverters gemäß einem der Ansprüche 8 bis 10 zur Umwandlung von durch LEDs oder OLEDs, insbesondere ausgewählt aus einer blauen LED oder einer weißen LED, erzeugtem Licht.

12. Verwendung eines Farbkonverters gemäß einem der Ansprüche 8 bis 11 in Displays.

13. Beleuchtungsvorrichtung mit mindestens einer LED und mindestens einem Farbkonverter nach einem der Ansprüche 7 bis 9, wobei die LED und der Farbkonverter vorzugsweise in einer Remote-Phosphor-Anordnung vorliegen.

14. Vorrichtung, die bei Beleuchtung Strom erzeugt, mit einer Photovoltaikzelle und dem Farbkonverter gemäß einem der Ansprüche 8 bis 11, wobei mindestens ein Teil des nicht von der Photovoltaikzelle absorbierten Lichts von dem Farbkonverter absorbiert wird.

15. Verwendung einer Verbindung der Formel I oder einer Mischung davon gemäß einem der Ansprüche 1 bis 7 in Farbkonvertern zur Konvertierung von von einer Lichtquelle, insbesondere einer aus LEDs und OLEDs ausgewählten Lichtquelle, emittiertem Licht in Licht einer zweiten, längeren Wellenlänge, zur Einfärbung von Lacken, Druckfarben und Kunststoffen, zur Herstellung von elektromagnetische Strahlung absorbierenden und/oder emittierenden wässrigen Polymerdispersionen, zur Datenspeicherung, für optische Label, für Sicherheitslabel in Dokumenten und für den Markenschutz oder als Fluoreszenzlabel für Biomoleküle.

## Revendications

1. Diimide 3,4;9,10-pérylènetétracarboxylique de formule I 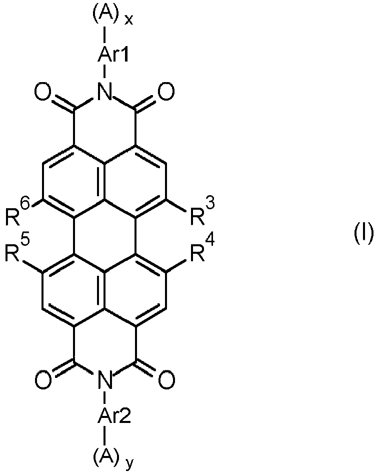 le composé de formule (I) comprenant au moins un groupe A
dans lequel
chaque groupe A est indépendamment choisi parmi les groupes de formule suivante 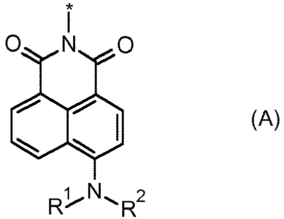 dans lesquels
* désigne le site de liaison au reste de la molécule ;
R¹ et R², indépendamment l'un de l'autre, sont choisis parmi l'atome d'hydrogène et les groupes alkyle en C₁-C₃₀, alcényle en C₂-C₃₀, alcynyle en C₂-C₃₀, cycloalkyle en C₃-C₁₂ et aryle en C₆-C₂₄, les deux derniers radicaux mentionnés étant non substitués ou portant un, deux ou trois radicaux choisis parmi les radicaux alkyle en C₁-C₁₀ ;
x vaut 0 ou 1 ;
y vaut 0 ou 1 ;
Ar1 est un groupe aryle en C₆-C₂₄ qui ne porte par de substituant R^{Ar1} ou qui porte 1, 2 ou 3 substituants R^{Ar1} identiques ou différents, chaque R^{Ar1}, indépendamment des autres, étant choisi parmi les groupes alkyle en C₁-C₃₀, halogénoalkyle en C₁-C₃₀, alcoxy en C₁-C₃₀, alkylsulfanyle en C₁-C₃₀, alcényle en C₂-C₃₀ et alcynyle en C₂-C₃₀ ;
Ar2 est un groupe aryle en C₆-C₂₄ qui ne porte pas de substituant R^{Ar2} ou qui porte 1, 2 ou 3 substituants R^{Ar2} identiques ou différents, chaque R^{Ar2}, indépendamment des autres, étant choisi parmi les groupes alkyle en C₁-C₃₀, halogénoalkyle en C₁-C₃₀, alcoxy en C₁-C₃₀, alkylsulfanyle en C₁-C₃₀, alcényle en C₂-C₃₀ et alcynyle en C₂-C₃₀ ;
R³, R⁴, R⁵ et R⁶, indépendamment les uns des autres, sont choisis entre l'atome d'hydrogène et un groupe de formule
(A)_{z}-Ar3-X-#,
dans lequel
# désigne le site de liaison au reste de la molécule ;
z vaut 0 ou 1 ;
X est 0 ou S ;
Ar3 est un groupe aryle en C₆-C₂₄ qui ne porte pas de substituant R^{Ar3} ou qui porte 1, 2 ou 3 substituants R^{Ar3} identiques ou différents, chaque R^{Ar3}, indépendamment des autres, étant choisi parmi les groupes alkyle en C₁-C₃₀, halogénoalkyle en C₁-C₃₀, alcoxy en C₁-C₃₀, alkylsulfanyle en C₁-C₃₀, alcényle en C₂-C₃₀, alcynyle en C₂-C₃₀, cycloalkyle en C₃-C₁₂ et aryle en C₆-C₂₄.

2. Diimide 3,4;9,10-pérylènetétracarboxylique de formule I selon la revendication 1, dans lequel R¹ et R², indépendamment l'un de l'autre, sont choisis parmi les groupes alkyle en C₁-C₂₀.

3. Diimide 3,4;9,10-pérylènetétracarboxylique de formule I selon la revendication 1 ou 2, dans lequel
Ar1 est choisi entre un groupe phényle qui porte 1 ou 2 substituants R^{Ar1} alkyle en C₁-C₆ et un groupe phényle qui porte un groupe A ; et
Ar2 est choisi entre un groupe phényle qui porte 1 ou 2 substituants R^{Ar2} alkyle en C₁-C₆ et un groupe phényle qui porte un groupe A.

4. Diimide 3,4;9,10-pérylènetétracarboxylique de formule I selon l'une quelconque des revendications précédentes, dans lequel R³, R⁴, R⁵ et R⁶, indépendamment les uns des autres, sont choisis entre l'atome d'hydrogène, un groupe phénoxy qui n'est pas substitué et un groupe phénoxy qui porte un groupe A.

5. Diimide 3,4;9,10-pérylènetétracarboxylique de formule I selon l'une quelconque des revendications précédentes, dans lequel deux des radicaux R³, R⁴, R⁵ et R⁶ sont des atomes d'hydrogène et les autres radicaux R³, R⁴, R⁵ et R⁶ sont choisis entre un groupe phénoxy qui n'est pas substitué et un groupe phénoxy qui porte un groupe A.

6. Diimide 3,4;9,10-pérylènetétracarboxylique de formule I selon l'une quelconque des revendications 1 à 4, dans lequel les radicaux R³, R⁴, R⁵ et R⁶ ont la même signification.

7. Diimide 3,4;9,10-pérylènetétracarboxylique de formule I selon l'une quelconque des revendications précédentes choisi entre

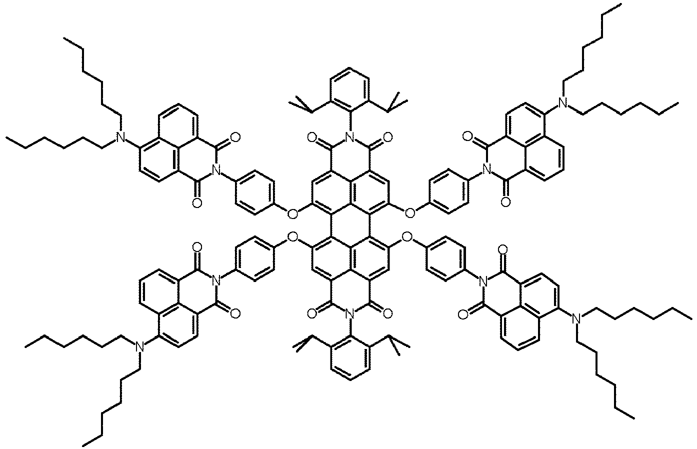

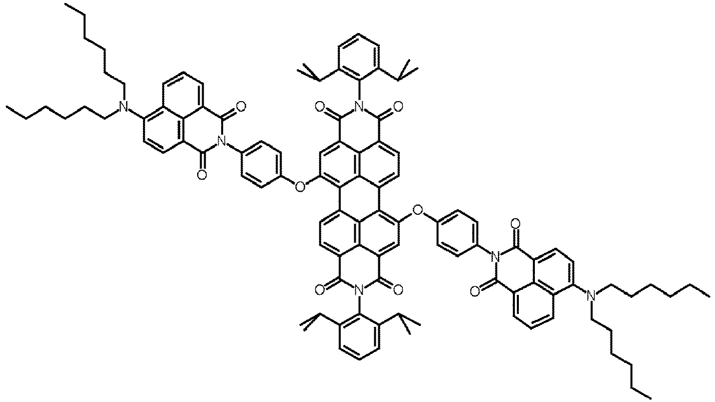

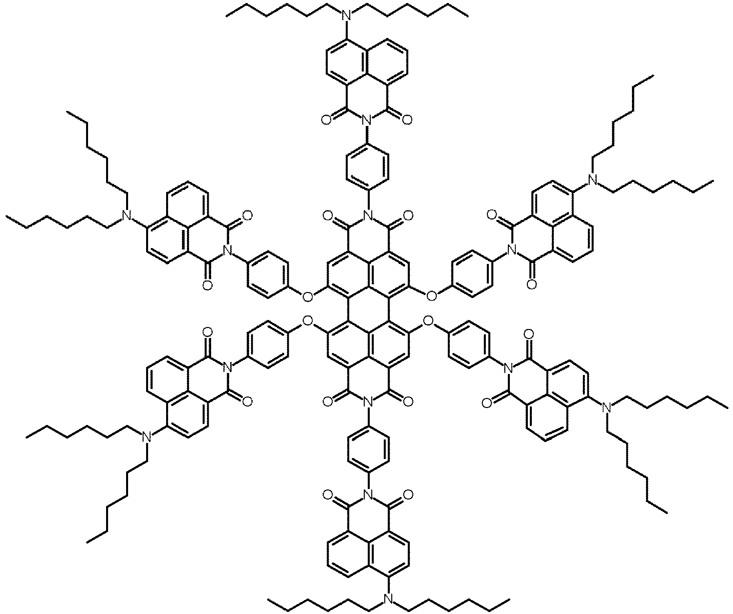

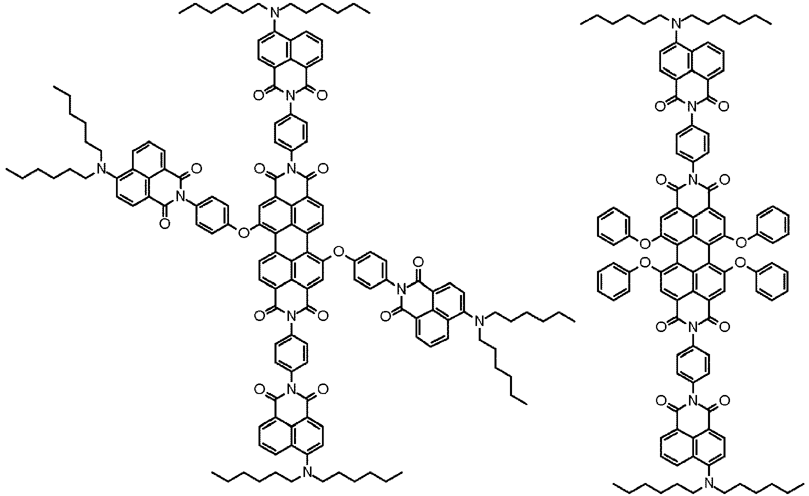

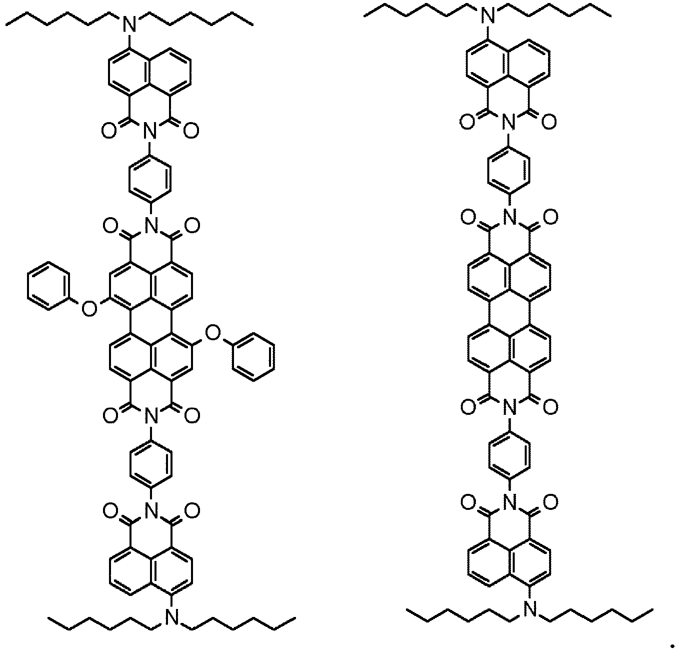

8. Convertisseur de couleur comprenant au moins un polymère en tant que matrice et au moins un composé de formule I ou un mélange de tels composés tels que définis dans l'une quelconque des revendications 1 à 7 en tant que colorant fluorescent, dans lequel l'au moins un polymère est choisi parmi le polystyrène, un polycarbonate, le poly(méthacrylate de méthyle), la polyvinylpyrrolidone, un polyméthacrylate, le poly(acétate de vinyle), le poly(chlorure de vinyle), le polybutène, la silicone, un polyacrylate, une résine époxyde, le poly(alcool vinylique), un copolymère poly(éthylène-alcool vinylique) (EVA, EVOH), le polyacrylonitrile, le poly(chlorure de vinylidène) (PVDC), le poly(styrène-acrylonitrile) (SAN), le poly(téréphtalate de butylène) (PBT), le poly(téréphtalate d'éthylène) (PET), le poly(butyrate de vinyle) (PVB), le poly(chlorure de vinyle) (PVC), les polyamides, les polyoxyméthylènes, les polyimides, les polyétherimides et les mélanges de ceux-ci, en particulier l'au moins un polymère est constitué essentiellement de polystyrène, de polycarbonate ou de poly(téréphtalate d'éthylène).

9. Convertisseur de couleur selon la revendication 8, le convertisseur de couleur comprenant de plus au moins un pigment blanc inorganique en tant que corps de diffusion.

10. Convertisseur de couleur selon l'une quelconque des revendications 8 ou 9, comprenant au moins une autre substance fluorescente choisie parmi les substances fluorescentes inorganiques et d'autres colorants fluorescents organiques.

11. Utilisation d'un convertisseur de couleur tel que défini dans l'une quelconque des revendications 8 à 10, pour la conversion de lumière produite par des DEL ou des DELO, en particulier choisies entre une DEL bleue et une DEL blanche.

12. Utilisation d'un convertisseur de couleur tel que défini dans l'une quelconque des revendications 8 à 11 dans des écrans.

13. Dispositif d'éclairage comprenant au moins une DEL et au moins un convertisseur de couleur selon l'une quelconque des revendications 7 à 9, la DEL et le convertisseur de couleur étant de préférence en un agencement de luminophore distant.

14. Dispositif produisant de l'énergie électrique lorsqu'il est éclairé comprenant une cellule photovoltaïque et le convertisseur de couleur tel que défini dans l'une quelconque des revendications 8 à 11, dans lequel au moins une partie de la lumière non absorbée par la cellule photovoltaïque est absorbée par le convertisseur de couleur.

15. Utilisation d'un composé de formule I ou d'un mélange de tels composés tels que définis dans l'une quelconque des revendications 1 à 7, dans des convertisseurs de couleur pour la conversion de lumière émise à partir d'une source de lumière, en particulier une source de lumière choisie parmi les DEL et les DELO, en lumière ayant une seconde longueur d'onde plus grande, pour la coloration de revêtements, d'encres d'impression et de plastiques, la production de dispersions aqueuses de polymère qui absorbent et/ou émettent un rayonnement électromagnétique, pour le stockage de données, pour des étiquettes optiques, pour des étiquettes de sécurité dans des documents et pour la protection de marques ou en tant que marqueurs fluorescents pour des biomolécules.
